# EUROPÄISCHE PATENTSCHRIFT

(11) **EP 0 737 680 B1**
(45) Veröffentlichungstag und Bekanntmachung des Hinweises auf die Patenterteilung: **03.03.2004**
(21) Anmeldenummer: 96105335.2
(22) Anmeldetag: 03.04.1996
(51) Int. Cl.: C07D 261/12, C07D 271/06, C07D 285/08, C07D 413/04, C07D 417/04, A61K 31/41, A61K 31/42

(54) **(Phenylalkylaminoalkyloxy)-hetero-aryl-Verbindungen sowie Verfahren und Zwischenprodukte zu ihrer Herstellung und diese Verbindungen enthaltende Arzneimittel**
(Phenylalkylaminoalkyloxyl)-hetero-aryl compounds, processes and intermediates for their preparation and medicaments containing these compounds
Composés de (phénylalkylaminoalkyloxy)-hétéro-aryle, procédés et produits intermédiaires pour leur préparation et médicaments contenant ces composés

(30) Priorität: 10.04.1995 DE 19513503
(43) Veröffentlichungstag der Anmeldung: 16.10.1996
(73) Patentinhaber: Solvay Pharmaceuticals GmbH, 30173 Hannover (DE)
(72) Erfinder: Mlinaric, Michael, Dr., 30163 Hannover (DE); Kehrbach, Wolfgang, Dr., 30173 Hannover (DE); Ziegler, Dieter, Dr., 30966 Hemmingen (DE); Brückner, Reinhard, Dr., 30559 Hannover (DE); Bielenberg, Willi, Dr., 31061 Alfeld/Leine (DE); Rose, Horst, Dr., 30455 Hannover (DE)
(74) Vertreter: Gosmann, Martin, Dr.

(56) Entgegenhaltungen:
- EP-A- 0 170 861
- EP-A- 0 335 723
- EP-A- 0 405 905

## Beschreibung

Die vorliegende Erfindung betrifft neue (Phenylalkylaminoalkyloxy)-heteroaryl-Verbindungen, in welchen der Heteroarylrest einen durch Thienyl oder Phenyl substituierten 5-gliedrigen Ring mit 2 oder 3 Heteroatomen, von denen eines Stickstoff ist und eines Sauerstoff ist oder bei insgesamt 3 Heteroatomen auch Schwefel sein kann, darstellt, und deren Salze sowie diese Verbindungen enthaltende pharmazeutische Zubereitungen und Verfahren und Zwischenprodukte zur Herstellung dieser Verbindungen.

Aus der europäischen Patentanmeldung Nr. 170 861 sind 3-(Aminoalkylaminocarbonylmethoxy)-5-phenylpyrazol-Verbindungen mit antiarrhythmischen Wirkungen, insbesondere die Reizschwelle zur Auslösung von Rhythmusstörungen am Herzen erhöhenden Wirkungen, bekannt.

In der europäischen Patentanmeldung Nr. 335 723 werden Isoxazol-Derivate beschrieben, welche als cerebro-aktive Arzneimittel und als zentral wirksame Muskelrelaxantien einsetzbar sind.

Aus der europäischen Patentanmeldung Nr. 405 905 ist die Verwendung gewisser Isoxalin-3-on-Derivate als Antidepressiva bekannt.

Der vorliegenden Erfindung liegt die Aufgabe zugrunde, neue Aminoalkyloxyheteroaryl-Verbindungen mit wertvollen pharmakologischen Eigenschaften zu entwickeln. Ferner liegt der Erfindung die Aufgabe zugrunde, neue zur Behandlung ischämischer Herzzustände einsetzbare pharmazeutische Wirkstoffe zu entwickeln.

Es wurde nun gefunden, daß die erfindungsgemäßen neuen an der Aminogruppe durch einen Phenylalkylrest substituierten Aminoalkyloxyheteroaryl-Verbindungen wertvolle herzwirksame pharmakologische Eigenschaften besitzen und ein zur Behandlung von ischämischen Herzzuständen geeignetes Wirkprofil aufweisen.

Die Erfindung betrifft daher neue Verbindungen der allgemeinen Formel I 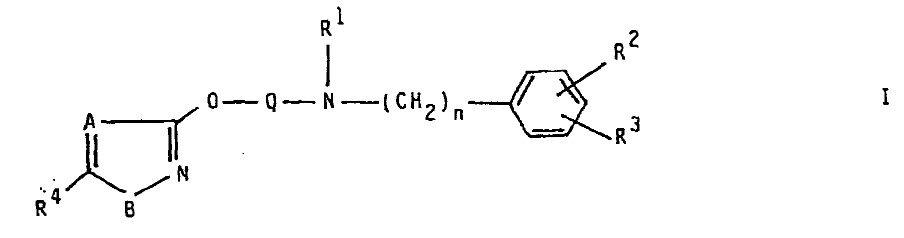 worin
- R¹: Wasserstoff oder niederes Alkyl bedeutet
- R²: Wasserstoff, Halogen, niederes Alkyl, niederes Alkoxy, Hydroxy oder, falls Q und R⁴ keine OH-Gruppe enthalten und R³ nicht Hydroxy ist, auch niederes Alkanoyloxy bedeutet und
- R³: Wasserstoff, Halogen, niederes Alkyl, niederes Alkoxy, Hydroxy oder, falls Q und R⁴ keine OH-Gruppe enthalten und R² nicht Hydroxy ist, auch niederes Alkanoyloxy oder, falls R² Wasserstoff ist, auch Trifluormethyl, Nitro, Amino, niederes Alkylamino, niederes Alkylsulfonylamino oder niederes Alkanoylamino bedeutet, wobei jedoch R³ nicht Nitro bedeutet, falls R⁶ Amino, niederes Alkylamino oder niederes Alkanoylamino ist, und nicht niederes Alkanoylamino bedeutet, falls R⁶ Amino oder niederes Alkylamino ist, oder
- R² und R³: an zwei benachbarte Kohlenstoffatome gebunden sind und gemeinsam eine Alkylendioxygruppe mit 1 - 2 Kohlenstoffatomen darstellen,
- R⁴: für Thienyl oder für eine gegebenenfalls substituierte Phenylgruppe der allgmeinen Formel a 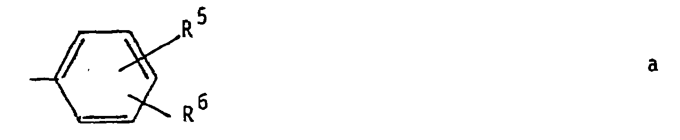
worin
- R⁵: Wasserstoff, Halogen, niederes Alkyl, niederes Alkoxy, Hydroxy oder, falls Q keine OH-Gruppe enthält und R², R³ und R⁶ nicht Hydroxy sind, auch niederes Alkanoyloxy bedeutet und
- R⁶: Wasserstoff, Halogen, niederes Alkyl, niederes Alkoxy, Hydroxy oder, falls Q keine OH-Gruppe enthält und R², R³ und R⁵ nicht Hydroxy sind, auch niederes Alkanoyloxy oder, falls R⁵ Wasserstoff ist, auch Trifluormethyl, Nitro, Amino, niederes Alkylamino oder niederes Alkänoylamino bedeutet,
- A: für Stickstoff oder eine R⁷-C-Gruppe, worin R⁷ Wasserstoff oder niederes Alkyl bedeutet, steht,
- B: für Sauerstoff oder, falls A Stickstoff ist, auch für Schwefel steht,
- n: eine ganze Zahl von 2 bis 4 bedeutet, und
- Q: für eine (CH₂)ₘ-Gruppe, worin m eine ganze Zahl von 2 bis 8 bedeutet und welche gegebenenfalls durch niederes Alkyl substituiert sein kann, oder für die 2-Hydroxypropylenkette steht,
sowie deren physiologisch verträgliche Säureadditionssalze.

Sofern in den Verbindungen der Formel I die Substituenten R², R³, R⁵ und R⁶ der Phenylringe niedere Alkylgruppen darstellen oder enthalten, können diese gerade oder verzweigt sein und insbesondere 1 bis 4, vorzugsweise 1 Kohlenstoffatom enthalten und stellen insbesondere Methyl oder Methoxy dar. Sofern die Substituenten Halogen darstellen, kommen insbesondere Fluor, Chlor oder Brom, vorzugsweise Fluor oder Chlor in Frage.

Die Substituenten R² und R³ sind vorzugsweise in 3- und/oder 4-Stellung angeordnet und stellen bevorzugt niederes Alkoxy, insbesondere Methoxy dar.

Der Substituent R⁴ steht vorzugsweise für eine gegebenenfalls substituierte Phenylgruppe. Allfällige Substituenten R⁵ und R⁶ dieser Phenylgruppe sind vorzugsweise in 2- und/oder 3-Position angeordnet. Eine Phenylgruppe R⁴ ist vorzugsweise unsubstituiert oder auch monosubstituiert, d.h. R⁶ ist vorzugsweise Wasserstoff, während R⁵ vorzugsweise Wasserstoff niederes Alkoxy oder auch Halogen, niederes Alkyl oder Hydroxy darstellt.

Die Gruppe Q steht vorzugsweise für eine vorzugsweise unsubstituierte Alkylenkette (CH₂)ₘ, worin m 2-8 bedeutet. Die Gruppe (CH₂)ₘ kann vorzugsweise 2-6, insbesondere 3 oder 5, bevorzugt 3 Kohlenstoffatome enthalten.

Der Substituent R¹ steht vorzugsweise für niederes Alkyl, insbesondere Methyl.

Erfindungsgemäß werden die neuen Verbindungen der Formel I und deren Säureadditionssalze erhalten, indem man in an sich bekannter Weise
a) zur Herstellung von Verbindungen der allgemeinen Formel Ia 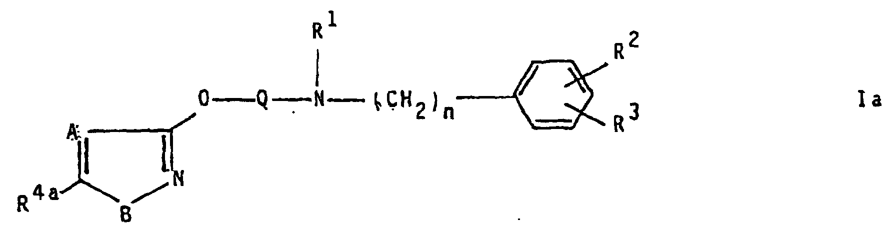 worin R¹, R², R³, A, B, Q und n obige Bedeutung besitzen und R^{4a} die für R⁴ angegebene Bedeutung mit Ausnahme von NH-haltigen Resten besitzt,
   Verbindungen der allgemeinen Formel II 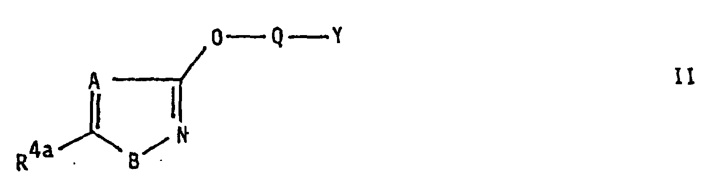 worin R^{4a}, A, B und Q obige Bedeutung besitzen und Y eine aminolytisch abspaltbare Fluchtgruppe bedeutet, mit Verbindungen der allgemeinen Formel IIIa 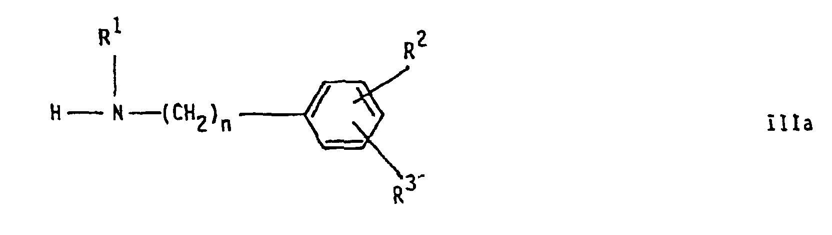 worin R¹, R², R³ und n obige Bedeutung besitzen, wobei jedoch allfällige freie Hydroxy- und/oder Aminogruppen R² und/oder R³ mit einer Schutzgruppe versehen sind, umsetzt und anschließend allfällige Hydroxy- und/oder Aminoschutzgruppen wieder abspaltet, oder
b) zur Herstellung von Verbindungen der allgemeinen Formel Ib 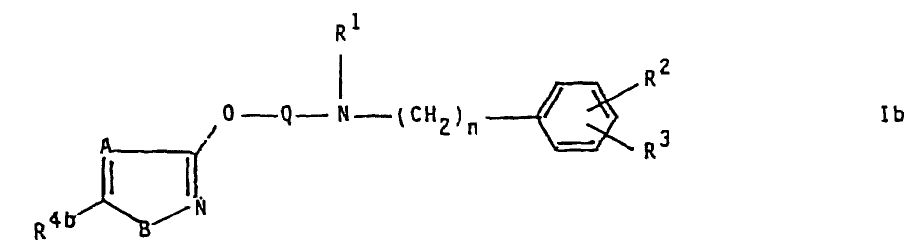 worin R¹, R², R³, A, B, Q und n obige Bedeutung besitzen und R^{4b} die für R⁴ angegebene Bedeutung mit Ausnahme von Hydroxy- und/oder NH-haltigen Resten besitzt, Verbindungen der allgemeinen Formel IVa 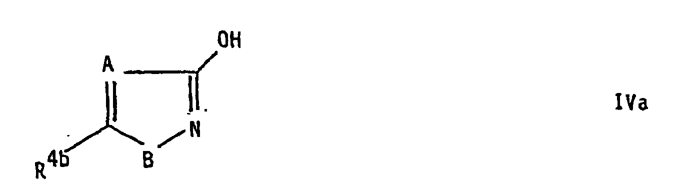 worin R^{4b}, A und B obige Bedeutung besitzen, mit Verbindungen der allgemeinen Formel V 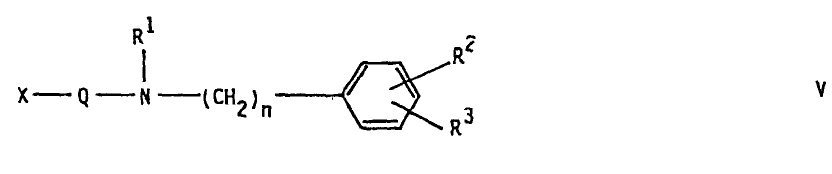 worin R¹, R², R³, n und Q obige Bedeutung besitzen, wobei jedoch allfällige freie Hydroxy- und/oder Aminogruppen mit einer Schutzgruppe versehen sind, und X für eine abspaltbare Fluchtgruppe steht, umsetzt und anschließend allfällige Hydroxy- und/oder Aminoschutzgruppen wieder abspaltet, oder
c) zur Herstellung von Verbindungen der allgemeinen Formel Ic 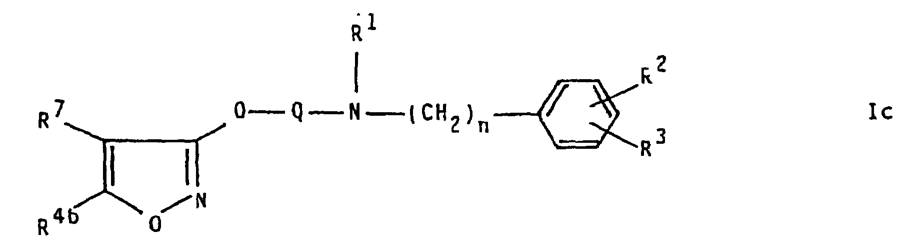 worin R¹, R², R³, R^{4b}, R⁷, Q und n obige Bedeutung besitzen,
   Verbindungen der allgemeinen Formel VI 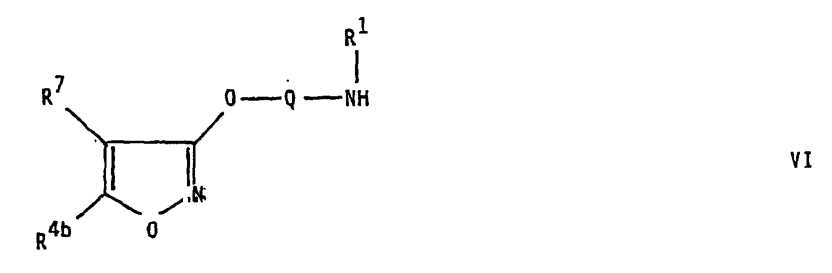 worin R¹, R⁷ und R^{4b} obige Bedeutung besitzen, mit Verbindungen der allgemeinen Formel VII 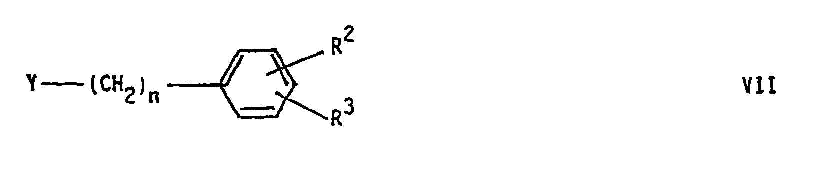 worin Y, n, R² und R³ obige Bedeutung besitzen, wobei jedoch freie Amino- und/oder Hydroxygruppen R² und/oder R³ mit einer Schutzgruppe versehen sind, umsetzt und anschließend allfällige Hydroxy- und/oder Aminoschutzgruppen wieder abspaltet, oder
d) zur Herstellung von Verbindungen der allgemeinen Formel Id 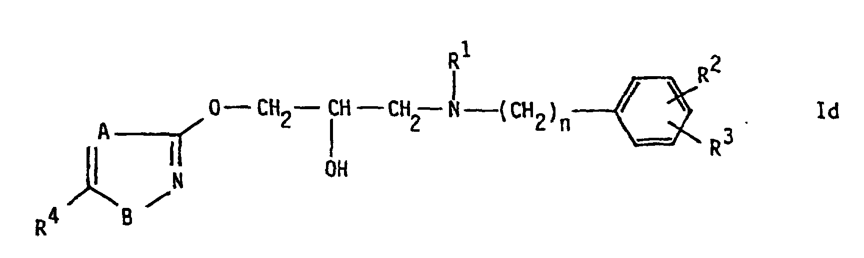 worin R¹, R², R³, R^{4b}, A, B, und n obige Bedeutung besitzen, Verbindungen der allgmeinen Formel IV 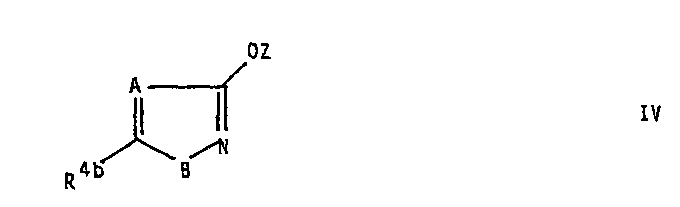 worin R^{4b}, A und B obige Bedeutung besitzen und Z für die 2,3-Epoxypropyl-Gruppe oder, falls A eine R⁷-C-Gruppe ist, auch für Wasserstoff steht, mit Verbindungen der allgemeinen Formel III 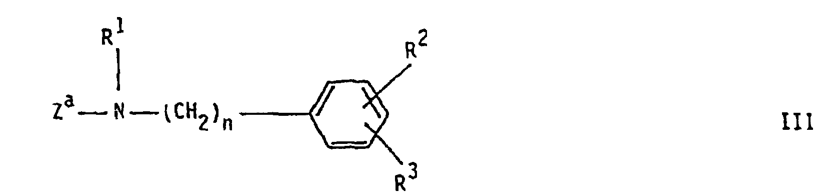 worin R¹, R², R³ und n obige Bedeutung besitzen, wobei jedoch allfällige freie Hydroxy- und/oder Aminogruppen mit einer Schutzgruppe versehen sind, und Z^{a} für Wasserstoff oder, falls Z in den Verbindungen der Formel IV Wasserstoff ist, auch für die 2,3-Epoxypropyl-Gruppe steht, umsetzt und anschließend allfällige Hydroxyund/oder Aminoschutzgruppen wieder abspaltet, oder
e) zur Herstellung von Verbindungen der allgemeinen Formel Ie 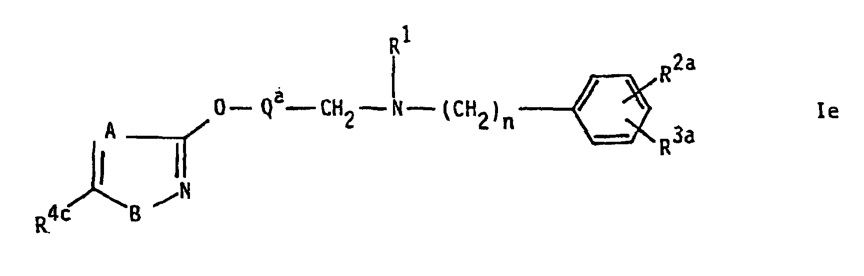 worin R¹, n, A und B obige Bedeutung besitzen, R^{4c} die für R^{4b} angegebene Bedeutung mit Ausnahme von Nitro und/oder niederes Alkanoyloxy enthaltenden Resten besitzt, R^{2a} und R^{3a} die für R² und R³ angegebenen Bedeutungen mit Ausnahme von Nitro, niederem Alkanoyloxy und niederem Alkanoylamino besitzen und Q^{a} für eine (CH₂)ₘ^{a}-Gruppe steht, worin m^{a} 3 oder 4 bedeutet und welche gegebenenfalls durch niederes Alkyl substituiert sein kann, steht, Verbindungen der allgemeinen Formel VIII 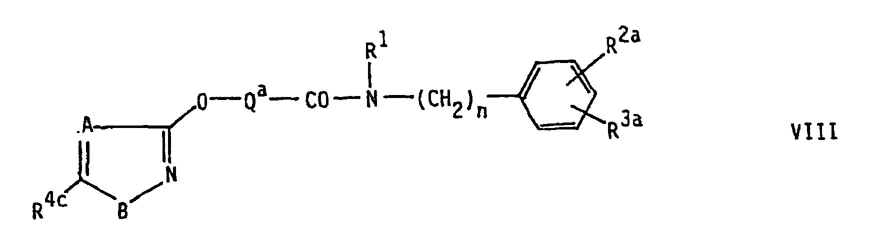 worin R¹, R^{2a}, R^{3a}, R^{4c}, A, B, Q^{a} und n obige Bedeutung besitzen, wobei jedoch allfällige freie Hydroxy- und/oder Aminogruppen mit einer Schutzgruppe versehen sind, reduziert und anschließend allfällige Hydroxy- und/oder Aminoschutzgruppen wieder abspaltet, oder
f) zur Herstellung von Verbindungen der allgemeinen Formel If 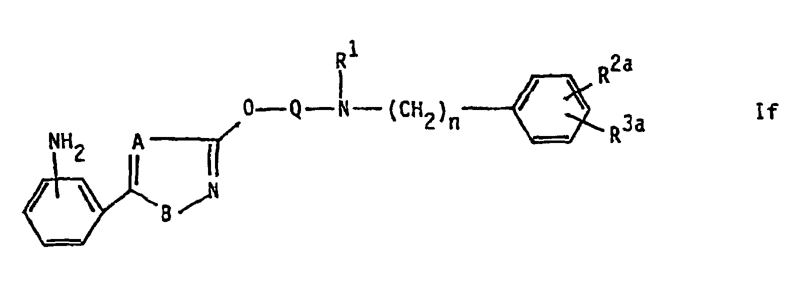 worin R¹, R^{2a}, R^{3a}, A, B, Q und n obige Bedeutung besitzen, Verbindungen der allgemeinen Formel Ig 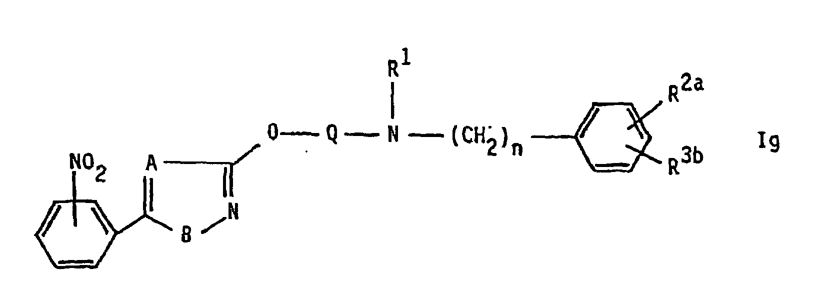 worin R¹, R^{2a}, A, B, Q und n obige Bedeutung besitzen und R^{3b} die für R³ angegebene Bedeutung mit Ausnahme von alkanoylhaltigen Resten besitzt, reduziert, oder
g) zur Herstellung von Verbindungen der allgemeinen Formel Ih 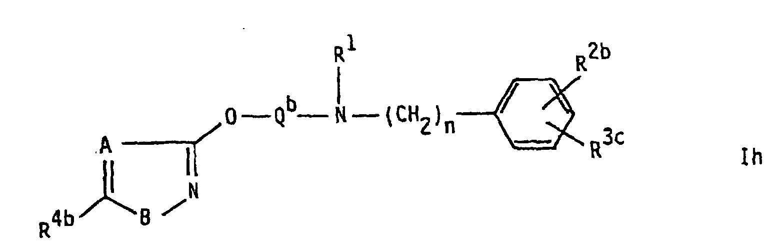 worin R¹, R^{4b}, A, B und n obige Bedeutung besitzen, Q^{b} die für Q angegebene Bedeutung mit Ausnahme der 2-Hydroxypropylenkette besitzt und R^{2b} und R^{3c} die für R² und R³ angegebenen Bedeutungen mit Ausnahme von OH-Gruppen besitzen, wobei jedoch von den Substituenten R^{2b} und R^{3c} und/oder von in R^{4b} enthaltenen Substituenten R⁵ und R⁶ mindestens einer niederes Alkanoyloxy oder niederes Alkanoylamino bedeutet, Verbindungen der allgemeinen Formel IX 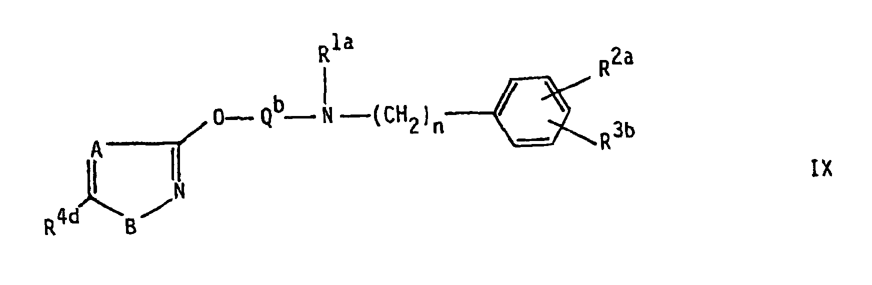 worin R^{2a}, R^{3b}, A, B, Q^{b} und n obige Bedeutung besitzen, R^{1a} niederes Alkyl oder eine Aminoschutzgruppe bedeutet und R^{4d} die für R⁴ angegebene Bedeutung mit Ausnahme von eine niedere Alkanoylgruppe enthaltenden Resten besitzt und worin gegebenenfalls Hydroxy- und/oder Aminoschutzgruppen enthalten sein können, wobei jedoch von den Substituenten R^{2a} und R^{3b} und/oder von in R^{4d} enthaltenen Substituenten R⁵ und R⁶ mindestens einer eine freie OH- oder NH₂-Gruppe bedeutet, acyliert und anschließend allfällige Schutzgruppen wieder abspaltet, oder
h) zur Herstellung von Verbindungen der allgemeinen Formel Ii 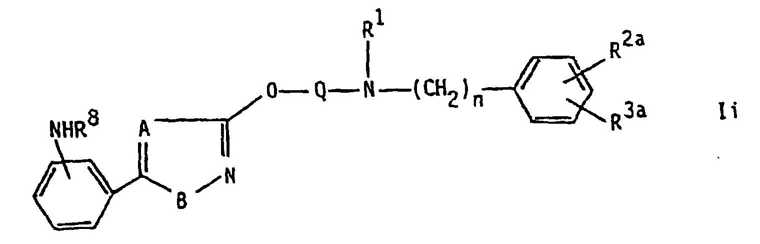 worin A, B, Q, R¹, R^{2a}, R^{3a} und n obige Bedeutung besitzen, und R⁸ niederes Alkyl bedeutet,
   Verbindungen der allgemeinen Formel Ij 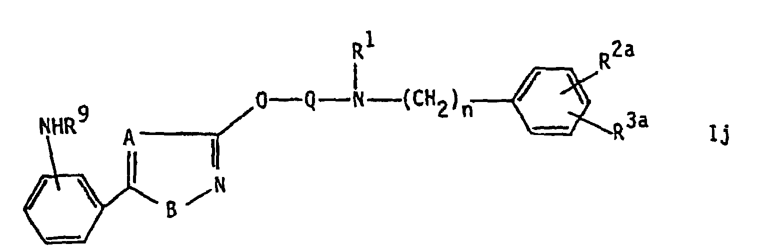 worin R¹, R^{2a}, R^{3a}, A, B und n obige Bedeutung besitzen und R⁹ niederes Alkanoyl bedeutet, reduziert, und
gewünschtenfalls in erhaltenen Verbindungen der Formel I, worin R², R³, R⁵ und/oder R⁶ Methoxy bedeuten, aus diesen Gruppen eine Hydroxygruppe freisetzt und/oder gewünschtenfalls erhaltene Verbindungen der Formel I, worin R¹ Wasserstoff bedeutet, zu Verbindungen der Formel I, worin R¹ niederes Alkyl bedeutet, alkyliert und gewünschtenfalls freie Verbindungen der Formel I in ihre Säureadditionssalze überführt oder die Säureadditionssalze in die freien Verbindungen der Formel I überführt.

Die Umsetzungen von Verbindungen der Formel II mit Verbindungen der Formel IIIa gemäß Verfahrensvariante a) und die Umsetzung von Verbindungen der Formel VI mit Verbindungen der Formel VII gemäß Verfahrensvariante c) können nach an sich zur Alkylierung von Aminen üblichen Methoden durchgeführt werden. Als aminolytisch abspaltbare Reste Y in den Verbindungen der Formeln II und VII eignen sich Halogene wie Chlor, Brom oder Jod, vorzugsweise Brom oder Chlor, oder auch organische Sulfonsäurereste, beispielsweise Reste von Niederalkansulfonsäuren wie z. B. Methansulfonsäure oder von aromatischen Sulfonsäuren wie Benzolsulfonsäure oder durch niederes Alkyl oder durch Halogen substituierten Benzolsulfonsäuren, z. B. Toluolsulfonsäuren oder Brombenzolsulfonsäuren. Die Umsetzungen können in einem unter den Reaktionsbedingungen inerten organischen Lösungsmittel unter basischen Bedingungen durchgeführt werden. Zweckmäßigerweise wird dem Reaktionsgemisch eine mindestens äquivalente Menge einer säurebindenden Base zugesetzt. Als Basen eignen sich z. B. anorganische oder organische Alkalimetallverbindungen. Beipiele von geeigneten Basen sind Alkalimetallcarbonate wie z. B. Kaliumcarbonat oder auch Alkalimetallhydroxyde, Alkalimetallhydride wie Natriumhydrid oder niedere Alkalimetallalkoholate wie z. B. Kalium-tert.-butylat oder Alkalimetallamide wie z. B. Lithiumamid. Als inerte organische Lösungsmittel eignen sich Dimethylformamid, Tetramethylharnstoff, oder je nach Art der als säurebindendes Mittel zugesetzten Base auch cyclische Ether wie Tetrahydrofuran, niedere Alkohole oder aromatische Kohlenwasserstoffe wie Benzol oder Toluol oder Gemische aus den vorgenannten Lösungsmitteln. Bei der Umsetzung von Verbindungen, in welchen A für Stickstoff steht, kann es zweckmäßig sein, das Lösungsmittel dem Reaktionsgemisch erst nach erfolgter Deprotonierung durch Umsetzung der Reaktanten mit der Base zuzugeben. Die Reaktion kann bei erhöhten Temperaturen, beispielsweise Temperaturen zwischen 60 °C und Siedetemperatur des Reaktionsgemisches, durchgeführt werden. Zweckmäßig wird die Umsetzung z. B. unter Verwendung einer anorganischen Base wie Kaliumcarbonat oder Natriumhydrid in einem inerten Lösungsmittel wie Dimethylformamid, bevorzugt bei Temperaturen zwischen 80° und 120 °C durchgeführt. Allfällige freie Hydroxygruppen in der Gruppe Q und/oder freie Hydroxy- und/oder Aminosubstituenten der Phenylringe können während der Reaktion durch anschließend leicht wieder abspaltbare Schutzgruppen geschützt werden. Die Verfahrensvariante a) eignet sich insbesondere für die Herstellung solcher Verbindungen der Formel I, worin A für Stickstoff steht.

Die Umsetzung von Verbindungen der Formel IVa mit Verbindungen der Formel V gemäß Verfahrensvariante b) kann auf an sich bekannte Weise unter zur Bildung von Heteroarylether-Verbindungen durch Alkylierung von Hydroxyheteroaryl-Verbindungen erfolgen. Als Fluchtgruppe X in den Verbindungen der Formel V, kommen bevorzugt Halogene wie Chlor, Brom oder Jod oder auch organische Sulfonsäurereste in Frage, beispielsweise Reste von Niederalkansulfonsäuren wie z. B. Methansulfonsäure oder von aromatischen Sulfonsäuren wie Benzolsulfonsäure oder durch niederes Alkyl oder Halogen substituierten Benzolsulfonsäuren, z. B. Toluolsulfonsäuren oder Bromsulfonsäuren. Im allgemeinen werden etwa äquivalente Mengen von Verbindungen der Formel IVa und V eingesetzt. Falls R¹ für Wasserstoff steht oder die Verbindungen freie Aminogruppen als Substituenten tragen, ist es zweckmäßig diese durch eine anschließend wieder abspaltbare Aminoschutzgruppe zu schützen, um Nebenreaktionen zu vermeiden. Allfällige freie Hydroxygruppen in den Verbindungen können während der Reaktion durch eine anschließend leicht wieder abspaltbare Hydroxyschutzgruppe geschützt werden. Die Umsetzung wird zweckmäßig in einem unter den Reaktionbedingungen inerten organischen Lösungsmittel unter basischen Bedingungen durchgeführt. Es können die vorstehend für die Verfahrensvarianten a) und c) angegebenen Lösungsmittel, Basen und Reaktionsbedingungen eingesetzt werden. Gewünschtenfalls können zur Beschleunigung der Reaktion katalytische Mengen eines Jodidsalzes, z. B. eines Alkalimetall- oder Ammoniumjodids wie Kaliumjodid oder tert. Butylammoniumjodid, zugesetzt werden. Die Reaktion kann bei Temperaturen zwischen Raumtemperatur und Siedetemperatur des Reaktionsgemisches durchgeführt werden. Zweckmäßig wird die Umsetzung z. B. unter Verwendung einer anorganischen Base wie Kaliumcarbonat oder Natriumhydrid in einem inerten Lösungsmittel wie Dimethylformamid, bevorzugt bei Temperaturen zwischen 80° und 120 °C durchgeführt.

Die Umsetzung von Verbindungen der Formel IV mit Verbindungen der Formel III gemäß Verfahrensvariante d) kann auf an sich bekannte Weise nach zur Umsetzung von Alkoholen oder Aminen mit Epoxiden üblichen Methoden erfolgen. So können Verbindungen der Formel IV mit Verbindungen der Formel III in einem unter den Reaktionsbedingungen inerten organischen Lösungsmittel unter basischen Bedingungen umgesetzt werden. Es können die für die Verfahrensvarianten a), b) und c) angegebenen Lösungsmittel und Basen eingesetzt werden. Die Reaktionstemperatur kann im Bereich von 60° bis 120 °C liegen. Die Reaktionsdauer kann je nach Art der Reaktionsbedingungen zwischen 4 und 12 Stunden betragen. Zweckmäßig wird die Umsetzung z. B. in einem inerten Lösungsmittel wie Dimethylformamid in Gegenwart einer organischen Base wie Kaliumcarbonat bei Temperaturen um 100 °C durchgeführt.

Die Reduktion von Verbindungen der Formel VIII gemäß Verfahrensvariante e) kann nach an sich zur Reduktion von Amiden üblichen Methoden erfolgen. Als Reduktionsmittel eignen sich zur Amidreduktion befähigte komplexe Metallhydride, insbesondere Aluminiumhydride wie Lithiumaluminiumhydrid oder Niederalkylaluminiumhydride, z. B. Diisobutylaluminiumhydrid. Die Reaktion findet in einem unter den Reaktionsbedingungen inerten ausreichend wasserfreien Lösungsmittel statt. Als Lösungsmittel eignen sich beispielsweise cyclische Ether wie Tetrahydrofuran oder Dioxan oder offenkettige Ether wie Ethylenglykoldimethylether oder Diethylenglykoldimethylether, gegebenenfalls im Gemisch mit aromatischen Kohlenwasserstoffen wie Benzol oder Toluol. Die Reaktion kann je nach Art des verwendeten Reduktionsmittels bei erhöhter Temperatur, beispielsweise Siedetemperatur des Reaktionsgemisches durchgeführt werden. Als günstig erweist sich beispielsweise die Reaktion mit Lithiumaluminiumhydrid bei Siedetemperatur des Reaktionsgemisches. Die Reaktionszeit kann zwischen 1 und 10 Stunden betragen.

Die Herstellung von Verbindungen der Formel If aus Verbindungen der Formel Ig gemäß Verfahrensvariante f) kann auf an sich bekannte Weise erfolgen. So können die Nitroverbindungen der Formel Ig auf an sich bekannte Weise zu entsprechenden Aminoverbindungen der Formel If reduziert werden. Die Reduktion kann nach für die Herstellung von Anilinderivaten aus entsprechenden Nitrophenolderivaten üblichen Methoden in einem unter den Reaktionsbedingungen inerten organischen Lösungsmittel durchgeführt werden, wobei die Reaktionsbedingungen so gewählt werden, daß der heterocyclische Ring nicht gespalten wird. So kann die Reduktion beispielsweise durch katalytische Hydrierung in Gegenwart von katalytischen Mengen von Raney-Nickel oder unter Verwendung von Hydrazinverbindungen als Reduktionsmittel erfolgen.

Die Acylierung von Verbindungen der Formel IX gemäß Verfahrensvariante g) kann nach an sich zur Bildung von Amiden und/oder Phenolestern üblichen Methoden durch Umsetzen von Verbindungen der Formel IX mit einem reaktiven Derivat einer entsprechenden niederen Carbonsäure, beispielsweise einem niederen Carbonsäurehalogenid oder -anhydrid, erfolgen. Beispielsweise kann die Umsetzung in Gegenwart eines säurebindenden Mittels, z. B. einer organischen Base wie einem Triniederalkylamin oder Pyridin in einem unter den Reaktionsbedingungen inerten organischen Lösungsmittel, z. B. halogenierten Kohlenwasserstoffen wie Dichlormethan oder cyclischen Ethern wie Tetrahydrofuran oder Pyridin, durchgeführt werden.

Die Reduktion von Verbindungen der Formel Ii nach der Verfahrensvariante h) kann nach an sich zur Reduktion von Amiden üblichen Bedingungen erfolgen und beispielsweise unter den für die Reduktion von Verbindungen der Formel VIII gemäß Verfahrensvariante e) angegebenen Bedingungen durchgeführt werden.

Falls die Gruppe Q eine Hydroxygruppe enthält und/oder freie Hydroxysubstituenten an Phenylresten der Verbindungen stehen und/oder die Verbindungen nicht an der Reaktion zu beteiligende freie Aminogruppen enthalten, müssen diese während der vorstehend beschriebenen Umsetzungen sowie auch bei der Herstellung der Ausgangsstoffe auf an sich bekannte Weise durch leicht wieder abspaltbare Schutzgruppen geschützt werden. Geeignete nach der Reaktion leicht wieder abspaltbare Schutzgruppen für Hydroxylgruppen sind z. B. bekannt aus E. McOmie "Protective Groups in Organic Chemistry" Plenum Press 1971. Beispielsweise eignen sich zum Schutz einer Hydroxygruppe Ester, z. B. Acetate, leicht abspaltbare Carbonate wie Benzylcarbonate und leicht abspaltbare Ether wie Tetrahydropyranylether oder Trimethylsilylether. Es müssen jeweils Schutzgruppen gewählt werden, die während der durchgeführten Reaktionen nicht angegriffen werden und die anschließend unter Bedingungen abspaltbar sind, unter denen die gebildeten Produkte nicht angegriffen werden. Bevorzugt können Etherschutzgruppen wie Trimethylsilylether verwendet werden. Die freien NH-Gruppen können gewünschtenfalls auf an sich bekannte Weise ebenfalls durch leicht wieder abspaltbare Schutzgruppen geschützt werden. Geeignete nach der Reaktion leicht wieder abspaltbare Schutzgruppen für NH-Gruppen sind z. B. bekannt aus E.McOmie "Protective Groups in Organic Chemistry" Plenum Press 1971. Beispielsweise eignen sich zum Schutz einer NH-Funktion die aus der Peptidchemie bekannten Schutzgruppen, beispielsweise leicht abspaltbare Carbamate. Es müssen jeweils Aminoschutzgruppe gewählt werden, die während der durchgeführten Reaktionen nicht angegegriffen werden und die anschließend unter Bedingungen abspaltbar sind, unter denen die gebildeten Produkte nicht angegriffen werden.

In Verbindungen der Formel I, worin Methoxysubstituenten enthalten sind, kann daraus gewünschtenfalls nachträglich die Hydroxygruppe freigesetzt werden. Methoxygruppen können mit zur Spaltung von Methoxyarylethern geeigneten Methoden auf an sich bekannte Weise zu Hydroxygruppen gespalten werden. Beispielsweise kann die Etherspaltung durch Behandeln mit Jodwasserstoff oder Bromwasserstoff in einem unter den Reaktionsbedingungen inerten Lösungsmittel, z. B. Acetanhydrid oder Essigsäure, oder mit Jodtrimethylsilan oder Bortribromid in einem halogenierten Kohlenwasserstoff wie Dichlormethan erfolgen. Allfällige niedere Alkanoyloxygruppen werden unter Bedingungen der Etherspaltung ebenfalls gespalten.

Die Verbindungen der Formel I können auf an sich bekannte Weise aus dem Rektionsgemisch isoliert und gereinigt werden. Säureadditionssalze können in üblicher Weise in die freien Basen überführt werden und diese gewünschtenfalls in bekannter Weise in pharmakologisch verträgliche Säureadditionssalze überführt werden.

Als pharmakologisch annehmbare Säureadditionssalze der Verbindungen der Formel I eignen sich beispielsweise deren Salze mit anorganischen Säuren, z. B. Halogenwasserstoffsäuren, insbesondere Chlorwasserstoffsäure, Schwefelsäure oder Phosphorsäuren, oder mit organischen Säuren, beispielsweise niederen aliphatischen Mono- oder Dicarbonsäuren wie Maleinsäure, Fumarsäure, Milchsäure, Weinsäure oder Essigsäure, N-Acetylglutaminsäure, oder Sulfonsäuren, beispielsweise Niederalkansulfonsäuren wie Methansulfonsäure oder gegebenenfalls im Benzolring durch Halogen oder niederes Alkyl substituierte Benzolsulfonsäuren wie p-Toluolsulfonsäure, oder Cyclohexylaminsulfonsäure.

Falls in den Verbindungen der Formel I die Gruppe Q eine Hydroxypropylenkette oder eine durch niederes Alkyl substituierte Alkylenkette bedeutet, enthalten die Verbindungen ein Chiralitätszentrum und können in zwei optisch aktiven Formen oder als Racemat vorliegen. Die vorliegende Erfindung umfaßt sowohl die racemischen Gemische wie auch die optischen Isomeren dieser Verbindungen der Formel I. Die optisch aktiven Verbindungen können aus den racemischen Gemischen in an sich bekannter Weise durch übliche Trennverfahren erhalten werden, z. B. durch chromatographische Trennung an chiralen Trennmaterialien oder fraktionierte Kristallisation geeigneter Salze mit optisch aktiven Säuren. Enantiomerenreine Verbindungen können auch durch Synthese aus entsprechenden enantiomerenreinen Ausgangsverbindungen hergestellt werden.

Die Ausgangsverbindungen der Formel II können auf an sich bekannte Weise erhalten werden, indem man entsprechende Verbindungen der Formel IVa mit Verbindungen der allgemeinen Formel X

X^{a}―Q―T X

worin Q obige Bedeutung besitzt, X^{a} für Halogen steht und T für eine aminolytisch abspaltbare Gruppe Y oder für Hydroxy steht, umsetzt und anschließend die Gruppe T, sofern diese Hydroxy darstellt, in eine aminolytisch abspaltbare Gruppe Y überführt und/oder einen allfälligen Methoxysubstituenten in dem Rest R^{4b} gewünschtenfalls auf an sich bekannte Weise in Hydroxy überführt. Die Umsetzung der Verbindungen der Formel IVa mit den Verbindungen der Formel X kann unter den zur Umsetzung von Verbindungen der Formel IVa mit Verbindungen der Formel V gemäß Verfahrensvariante b) angegebenen Bedingungen erfolgen. Zur Vermeidung von Nebenreaktionen ist es zweckmäßig, einen Überschuß an Verbindungen der Formel X einzusetzen. Sofern in Verbindungen der Formel X der Rest T eine aminolytisch abspaltbare Fluchtgruppe Y darstellt, ist es vorteilhaft, wenn die beiden in der Verbindung der Formel X enthaltenen Fluchtgruppen unterschiedliche Reaktivität aufweisen. Sofern Verbindungen der Formel X eingesetzt werden, worin T Hydroxy bedeutet, kann die Hydroxy-Gruppe in den erhaltenen Verbindungen anschließend auf an sich bekannte Weise in eine Gruppe Y überführt werden. So können die Hydroxy-Verbindungen beispielsweise zur Einführung eines Halogenrestes Y mit Thionylchlorid oder mit Phosphorhalogeniden auf an sich bekannte Weise umgesetzt werden. Sulfonsäurereste Y können auf an sich bekannte Weise eingeführt werden, indem man die Hydroxy-Verbindungen mit einem entsprechenden Sulfonsäurehalogenid acyliert. Die Spaltung von Methoxysubstituenten zu Hydroxy kann auf an sich bekannte Weise beispielsweise unter den vorstehend zur Freisetzung von Hydroxygruppen aus Methoxysubstituenten der Verbindungen der Formel I angegebenen Bedingungen durchgeführt werden.

Verbindungen der allgemeinen Formel IVc 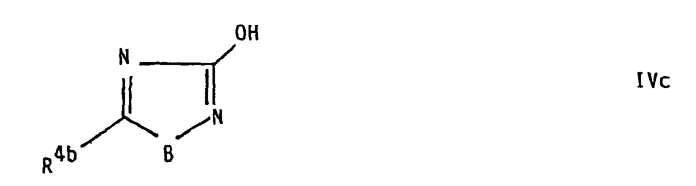 worin R^{4b} und B obige Bedeutung besitzen, sind bekannt oder können auf an sich bekannte Weise ausgehend von Verbindungen der allgemeinen Formel XI 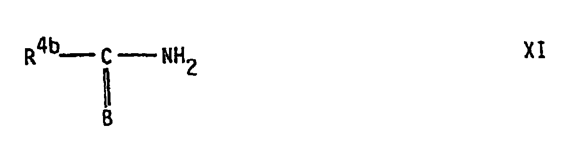 worin R^{4b} und B obige Bedeutung besitzen, erhalten werden, indem man die Amide oder Thioamide der Formel XI mit Oxalylchlorid der Formel XII 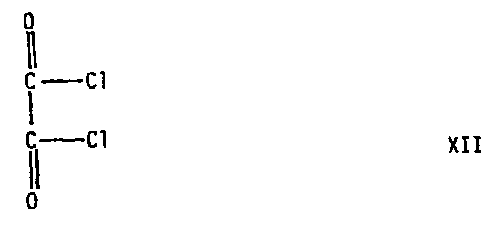 umsetzt und die erhaltenen Reaktionsprodukte mit Trimethylsilylazid weiter umsetzt. Sofern B für Schwefel steht, werden die Thioamide der Formel XI zunächst mit Oxalylchlorid bei Temperaturen zwischen -30° und +30 °C in einem unter den Reaktaionsbedingungen inerten organischen Lösungsmittel, beispielsweise einem aromatischem Kohlenwasserstoff oder Aceton, umgesetzt. Dabei bilden sich die cyclischen Dion-Verbindungen der allgemeinen Formel XIII 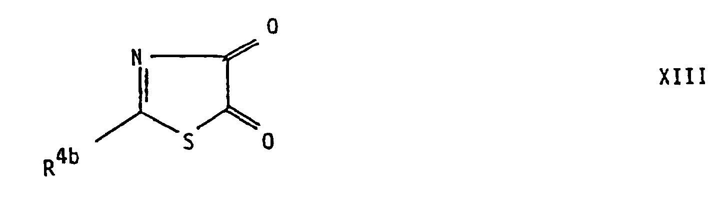 worin R^{4b} obige Bedeutung besitzt, welche gewünschtenfalls isoliert oder direkt in situ unter Erhitzen mit Trimethylsilylazid weiter umgesetzt werden können. Die Umsetzung mit Trimethylsilylazid erfolgt in einem unter den Reaktionsbedingungen inerten organischen Lösungsmittel, beispielsweise einem aromatischen Kohlenwasserstoff, bei Temperaturen zwischen 90 und 130 °C. Dabei wird das cyclische Dion durch Abspaltung von CO intermediär in ein instabiles Thioacylisocyanat überführt, welches mit dem Azid unter Cyclisierung zu entsprechenden Verbindungen der Formel IVc reagiert. Falls B für Sauerstoff steht, kann die Umsetzung von Amiden der Formel XI mit Oxalylchlorid in einem inerten organischen Lösungsmittel, beispielsweise einem halogenierten Kohlenwasserstoff, bei Temperaturen zwischen 30 und 50 °C erfolgen. Hierbei werden direkt die entsprechenden Acylisocyanate der Formel XIV 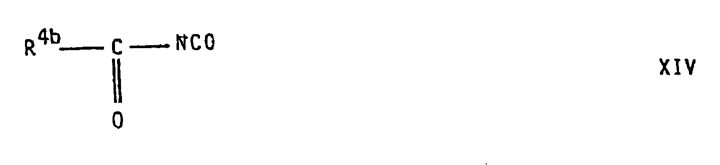 worin R^{4b} obige Bedeutung besitzt, erhalten, von denen viele bekannt sind. Die Acylisocyanate der Formel XIV können isoliert werden oder direkt in situ mit Trimethylsilylazid weiter umgesetzt werden. Beim Erhitzen der Acylisocyanate mit dem Trimethylsilylazid auf Temperaturen von 40 bis 60 °C, vorzugsweise Siedetemperatur des Reaktionsgemisches, reagieren die Acylisocyanate mit dem Azid unter Cyclisierung zu entsprechenden Verbindungen der Formel IVc.
Die Amide und Thioamide der Formel XI sind bekannt oder können nach an sich bekannten Methoden erhalten werden. Beispielsweise können Säurechloride der allgemeinen Formel XV

R^{4b}―CO―Cl XV

worin R^{4b} obige Bedeutung besitzt, auf an sich bekannte Weise in entsprechende Amide der Formel XI umgesetzt werden. Thioamide der Formel XI können aus den entsprechenden Amiden nach an sich zum Austausch von Sauerstoff gegen Schwefel üblichen Methoden hergestellt werden, beispielsweise nach der von Lawesson et al. beschriebenen Methode (siehe Tetrahedron 35, 2433 - 2437) durch Umsetzen mit 2,4-Bis(H-Methoxyphenyl)-1,3-dithia-2,4-diphosphetan-2,4-disulfid (bekannt als Lawessons Reagenz).

Verbindungen der allgemeinen Formel IVb 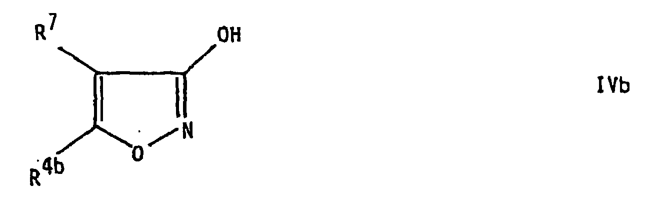 worin R^{4b} und R⁷ obige Bedeutung besitzen, können auf an sich bekannte Weise ausgehend von Verbindungen der allgemeinen Formel XVIa oder XVIb 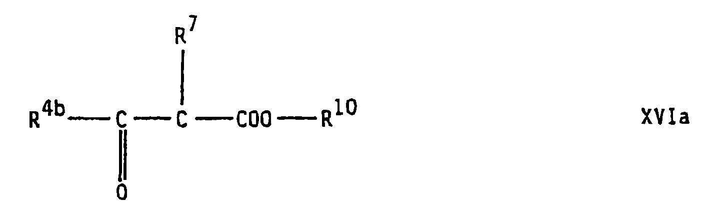 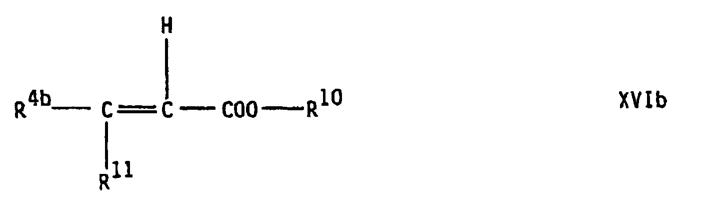 worin R^{4b} und R⁷ obige Bedeutung besitzen, R¹⁰ niederes Alkyl bedeutet und R¹¹ für den Imidazolylrest steht, erhalten werden, indem man die Verbindungen der Formeln XVIa oder XVIb durch Umsetzung mit Hydroxylamin und anschließende Säurebehandlung zu Verbindungen der Formel IVb cyclisiert. Die Umsetzung mit Hydroxylamin erfolgt zweckmäßigerweise in einem niederen Alkanol in Gegenwart einer starken Base beispielsweise einem Alkalimetallhydroxid oder -alkoholat. Bei der Cyclisierung von Verbindungen der Formel XIVa können Gemische der Isoxazol-3-ol Verbindungen der Formel IVb mit entsprechenden regioisomeren Isoxazol-5-onen und deren Tautomeren auftreten, deren Zusammensetzung je nach Art der Reste R^{4b} und R⁷ variieren kann. Um die Ausbeute an Verbindungen der Formel IVb möglichst hoch zu halten, ist es zweckmäßig die Behandlung der β-Ketoester der Formel XIVa bei tiefen Temperaturen, z. B. Temperaturen im Bereich von -50 bis -75 °C, vorzunehmen und anschließend das Reaktionsgemisch schnell in die Säure, z. B. in konzentrierte Salzsäure, zu geben.

Verbindungen der Formeln XVIa und XVIb sind bekannt oder können auf an sich bekannte Weise hergestellt werden. Beispielsweise können Verbindungen der Formel XVIb auf an sich bekannte Weise durch Einführung des Restes R¹¹ in ungesättigte Ester der allgemeinen Formel XVII 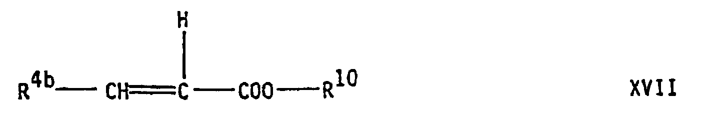 worin R^{4b} und R¹⁰ obige Bedeutung besitzen, erhalten werden. Hierzu können die ungesättigten Ester zunächst zu entsprechenden Dibromcarbonsäureestern bromiert werden, und diese anschließend unter HBr-abspaltenden Bedingungen mit dem Imidazol umgesetzt werden.

Verbindungen der allgemeinen Formel IVd 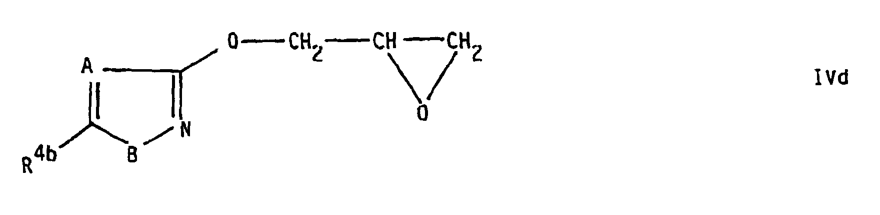 worin R^{4b}, A und B obige Bedeutung besitzen, können auf an sich bekannte Weise aus Verbindungen der Formel IVa durch Umsetzung mit Epichlorhydrin erhalten werden. Die Umsetzung kann unter den für die Umsetzung von Verbindungen der Formel IV mit Verbindungen der Formel III angegebenen Bedingungen erfolgen. Zweckmäßig wird hierbei ein Überschuß an Epichlorhydrin eingesetzt.

Verbindungen der Formel IIIa sind bekannt oder können nach an sich bekannten Methoden oder analog zu an sich bekannten Methoden hergestellt werden. Beispielsweise können Verbindungen der Formel IIIa ausgehend von entsprechenden Säuren der allgemeinen Formel XVIII 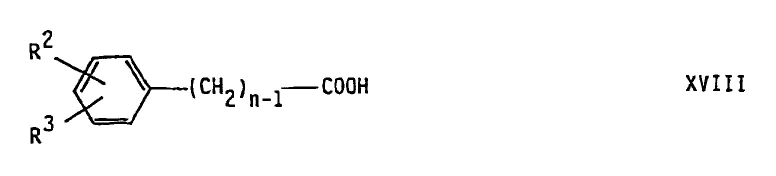 worin R², R³ und n obige Bedeutung besitzen, erhalten werden. So können reaktionsfähige Säurederivate der Säuren der Formel XVIII mit Aminen der allgemeinen Formel XIX

R¹―NH₂ XIX

worin R¹ obige Bedeutung besitzt, zu entsprechenden Amiden umgesetzt und diese anschließend zu Verbindungen der Formel IIIa reduziert werden. Die Reduktion kann beispielsweise unter den zur Reduktion der Verbindungen der Formel VIII gemäß Verfahrensvariante e) angegebenen Bedingungen erfolgen. Säuren der Formel XVIII sind bekannt oder können auf an sich bekannte Weise erhalten werden.

Verbindungen der Formel III, worin Z^{a} für einen 2,3-Epoxypropylrest steht, können aus entsprechenden Verbindungen der Formel IIIa auf an sich bekannte Weise durch Umsetzen mit Epichlorhydrin erhalten werden.

Verbindungen der Formel V sind bekannt oder können nach an sich bekannten Methoden oder analog zu an sich bekannten Methoden hergestellt werden. Beispielsweise können Verbindungen der Formel V erhalten werden, indem man Amine der Formel IIIa mit Verbindungen der allgemeinen Formel Xa

X^{a}―Q―T^{a} Xa

worin X^{a} und Q obige Bedeutung besitzen und T^{a} für eine Fluchtgruppe X oder für Hydroxy steht, umsetzt, und anschließend die Gruppe T^{a} gewünschtenfalls auf an sich bekannte Weise in eine andere Fluchtgruppe X umwandelt. Die Umsetzung kann nach an sich zu Aminoalkylierung üblichen Methoden beispielsweise unter den zur Umsetzung von Verbindungen der Formel II mit Verbindungen der Formel IIIa gemäß Verfahrensvariante a) angegebenen Bedingungen erfolgen. Zur Vermeidung von Nebenreaktionen ist es zweckmäßig, einen Überschuß an Verbindungen der Formel Xa einzusetzen. Sofern in den Verbindungen der Formel Xa der Rest T^{a} eine Fluchtgruppe X darstellt, ist es vorteilhaft, wenn die beiden in der Verbindung der Formel Xa enthaltenen Fluchtgruppen unterschiedliche Reaktivität aufweisen, um eine gleichzeitige Reaktion beider Fluchtgruppen mit Verbindungen der Formel IIIa zu vermeiden. Sofern die Verbindungen der Formel IIIa mit Halogenalkoholen der Formel Xa umgesetzt wurden, kann in dem Reaktionsprodukt anschließend die Hydroxygruppe auf an sich bekannte Weise gegen einen Rest X ausgetauscht werden. Dies kann beispielsweise in der vorstehend bei der Herstellung von Verbindungen der Formel II beschriebenen Weise erfolgen.

Verbindungen der Formel VI können erhalten werden, indem man Verbindungen der Formel IVb mit Verbindungen der allgemeinen Formel XX 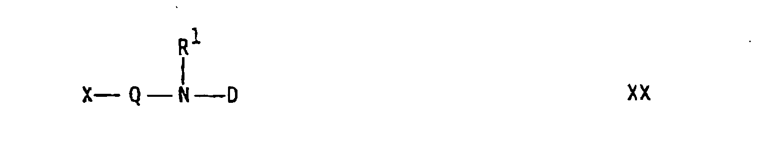 worin R¹, X und Q obige Bedeutung besitzen und D eine abspaltbare Aminoschutzgruppe bedeutet, umsetzt und aus den erhaltenen Reaktionsprodukten die Schutzgruppe D wieder abspaltet. Die Umsetzung von Verbindungen der Formel IVb mit den Verbindungen der Formel XX kann auf an sich bekannte Weise beispielsweise unter den für die Umsetzung der Verbindungen der Formel IVb mit Verbindungen der Formel V gemäß Verfahrensvariante b) angegebenen Reaktionsbedingungen erfolgen.

Verbindungen der Formel XX sind bekannt oder können auf an sich bekannte Weise, beispielsweise analog der Herstellung der Verbindungen der Formel V erhalten werden. So können Verbindungen der Formel XX erhalten werden durch Einführung einer Aminoschutzgruppe D in Amine der Formel XIX und nachfolgende Umsetzung der geschützten Amine mit Verbindungen der Formel Xa mit gegebenenfalls anschließendem Austausch einer Hydroxygruppe T^{a} gegen einen Rest X.

Die Ausgangsverbindungen der Formel VIII sind neue Verbindungen, welche wertvolle Zwischenprodukte zur Herstellung von pharmakologisch wirksamen Verbindungen, beispielsweise den Verbindungen der Formel Ie, darstellen.

Verbindungen der Formel VIII können nach an sich zur Amidbildung üblichen Methoden erhalten werden, indem man Amine der Formel IIIa mit Säuren der allgemeinen Formel XXI 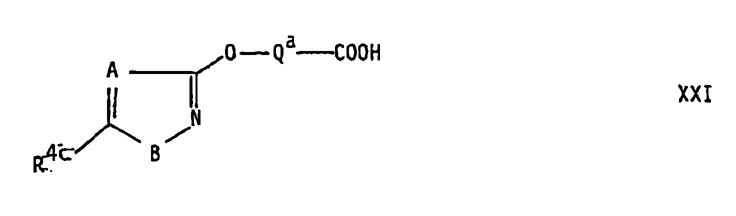 worin R^{4c}, A, B und Q^{a} obige Bedeutung besitzen, oder deren reaktionsfähigen Säurederivaten unter zur Aminoacylierung üblichen Bedingungen umsetzt. So können die Amine der Formel IIIa mit reaktionsfähigen Derivaten der Säuren der Formel XXI zweckmäßig in einem unter den Reaktionsbedingungen inerten organischen Lösungsmittel und in Gegenwart eines säurebindenden Reagenzes umgesetzt werden. Als reaktionsfähige Derivate der Säuren der Formel XXI kommen insbesondere Säurehalogenide und gegebenenfalls gemischte Säureanhydride oder Ester in Frage. Als Lösungsmittel eignen sich halogenierte Kohlenwasserstoffe wie Dichlormethan, aromatische Kohlenwasserstoffe wie Benzol, Toluol oder Chlorbenzol, cyclische Ether wie Tetrahyrofuran oder Dioxan, Dimethylformamid oder Gemische dieser Lösungsmittel. Als säurebindende Reagenzien eignen sich organische oder anorganische Basen. Beispiele geeigneter organischer Basen sind tertiäre organische Amine, insbesondere tertiäre niedere Alkylamine wie Triethyiamin, Tripropylamine oder N-Niederalkylpiperidine. Geeignete anorganische Basen sind insbesondere Alkalimetallcarbonate oder -bicarbonate. Falls als Acylierungsmittel die Säuren selbst oder auch deren Ester eingesetzt werden, kann die Umsetzung der Verbindungen der Formel IIIa mit den Säuren der Formel XXI zweckmäßigerweise in Gegenwart eines dehydratisierenden Reagenzes, beispielsweise eines aus der Peptidchemie als zur Amidbildung geeignet bekannten Kopplungsreagenzes, durchgeführt werden. Als Beispiele derartiger Reagenzien, welche die Acylierung auch dadurch fördern, daß sie mit der Säure in situ unter Bildung eines reaktionsfähigen Säurederivates reagieren, seien insbesondere genannt Alkyl-, vorzugsgweise Cycloalkylcarbodiimide wie Dicyclohexylcarbodiimid, Carbonylimidazol oder N-Niederalkyl-2-halogenpyridiniumsalze, insbesondere Halogenide oder Tosylate, vorzugsweise N-Methyl-2-chlorpyridiniumjodid (= Mukajama Reagenz). Die Umsetzung in Gegenwart eines derartigen Kopplungsreagenzes kann zweckmäßigerweise bei Temperatauren von -30 °C bis +50 °C unter neutralen Reaktionsbedingungen in Lösungsmitteln wie halogenierten Kohlenwasserstoffen und/oder aromatischen Kohlenwasserstoffen durchgeführt werden.

Die Säuren der Formel XXI können nach an sich bekannten Methoden erhalten werden, indem man entsprechende Verbindungen der Formel IVa mit Halogencarbonsäureestern oder -nitrilen der allgemeinen Formeln XXIIa und XXIIb

X^{a}―Q^{a}―COO―R¹² XXIIa

x^{a}―Q^{a}―CN XXIIb

worin X^{a} und Q^{a} obige Bedeutung besitzen und R¹² niederes Alkyl bedeutet, umsetzt und die erhaltenen Ester oder Nitrile anschließend zu den Säuren der Formel XXI hydrolysiert. Die Umsetzung der Verbindungen der Formel IVa mit den Verbindungen der Formel XXIIa oder XXIIb kann auf an sich bekannte Weise, beispielsweise unter den für die Verfahrensvariante b) angegebenen Bedingungen, durchgeführt werden. Zweckmäßig wird die Reaktion in Dimethylformamid in Gegenwart einer starken Base, beispielsweise Natriumhydrid, bei erhöhten Temperaturen, beispielsweise Temperaturen zwischen 80 und 100 °C durchgeführt. Die anschließende Hydrolyse zu den Säuren der Formel XXI erfolgt vorzugsweise unter alkalischen Bedingungen. Die Säuren der Formel XXI können auf an sich bekannte Weise in ihre reaktiven Säurederivate überführt werden.

Verbindungen der Formel IX stellen mit Ausnahme derjenigen Verbindungen worin R^{1a} eine Aminoschutzgruppe darstellt, entsprechende Verbindudngen der Formel I dar. Verbindungen der Formel IX worin R^{1a} eine Aminoschutzgruppe darstellt, können aus entsprechenden Verbindungen der Formel I worin R¹ Wasserstoff bedeutet, durch Einführung einer Aminoschutzgruppe auf an sich bekannte Weise erhalten werden.

Die Verbindungen der Formel I und ihre pharmakologisch akzeptablen Säureadditionssalze zeichnen sich durch interessante pharmakologische Eigenschaften aus. Insbesondere zeigen die Verbindungen Herz-Kreislauf-wirksame Eigenschaften und zeichnen sich durch zytoprotektive Wirkungen am Herzen aus. Dabei besitzen sie ein zur Behandlung ischämischer Zustände wie z. B. bei koronarer Herzkrankheit günstiges Wirkungsprofil, da in der Substanzgruppe auch herzfrequenzsenkende Wirkungen vorhanden sind.

Die herzwirksamen Eigenschaften der Verbindungen lassen sich in pharmakologischen Standardtestmethoden in vitro und in vivo nachweisen.

Beschreibung der pharmakologischen Untersuchungsmethoden:
1. Bestimmung der minimalen toxischen Dosis.
   Männlichen Mäusen von 20 bis 25 g Gewicht werden p. o. maximal Dosen von 300 mg/kg der Testsubstanz verabreicht. Die Tiere werden 3 Stunden lang sorgfältig auf Toxizitätssymptome beobachtet. Über einen Zeitraum von 72 Stunden nach der Applikation werden zusätzlich alle Symptome und Todesfälle registriert. Begleitsymptome werden ebenfalls beobachtet und registriert. Wenn Tod oder starke toxische Symptome beobachtet werden, werden weiteren Mäusen zunehmend geringere Dosen verabreicht, bis keine toxischen Symptome mehr auftreten. Die niedrigste Dosis, welche Tod oder stark toxische Symptome hervorruft, wird in der nachfolgenden Tabelle A als minimale toxische Dosis angegeben. Die in Tabelle A angegebenen Beispielsnummern beziehen sich auf die nachstehenden Herstellungsbeispiele. 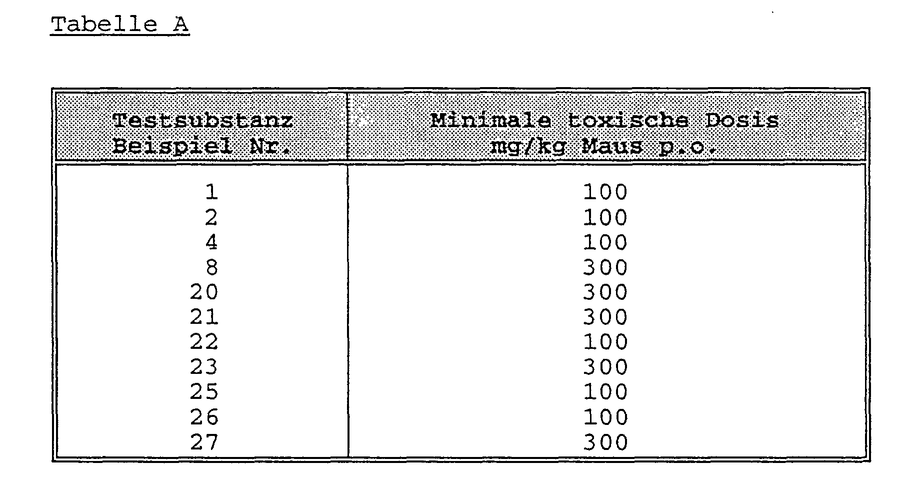
2. In vitro Nachweis der zytoprotektiven Wirkung
   Die zytoprotektive Wirkung wurde an isolierten in einer Nährlösung gehaltenen elektrisch gereizten linken Vorhöfen von männlichen Pirbright-White Meerschweinchen der Gewichtsklasse 250 bis 300 g geprüft. Durch Begasung der Nährlösung mit Stickstoff für 60 Minuten wurde eine zeitweilige Hypoxie herbeigeführt. Infolge der Hypoxie kam es zu einer Kontraktur der Vorhöfe. Nach der Hypoxie wurde reoxygeniert. Als Meßparameter dienten das Integral der Kontraktur über den Zeitraum der Hypoxie, und die Erholung der Kontraktionskraft der stimulierten Vorhöfe nach der Hypoxie. In der nachfolgenden Tabelle B ist die effektive Konzentration in µmol/l angegeben, bei der das Kontrakturintegral auf 50 % des Kontrollwertes reduziert wurde. Als Maß für die Krafterholung ist in Tabelle B die Kontraktionskraft nach der Hypoxie in % des ursprünglichen Ausgangswertes angegeben. 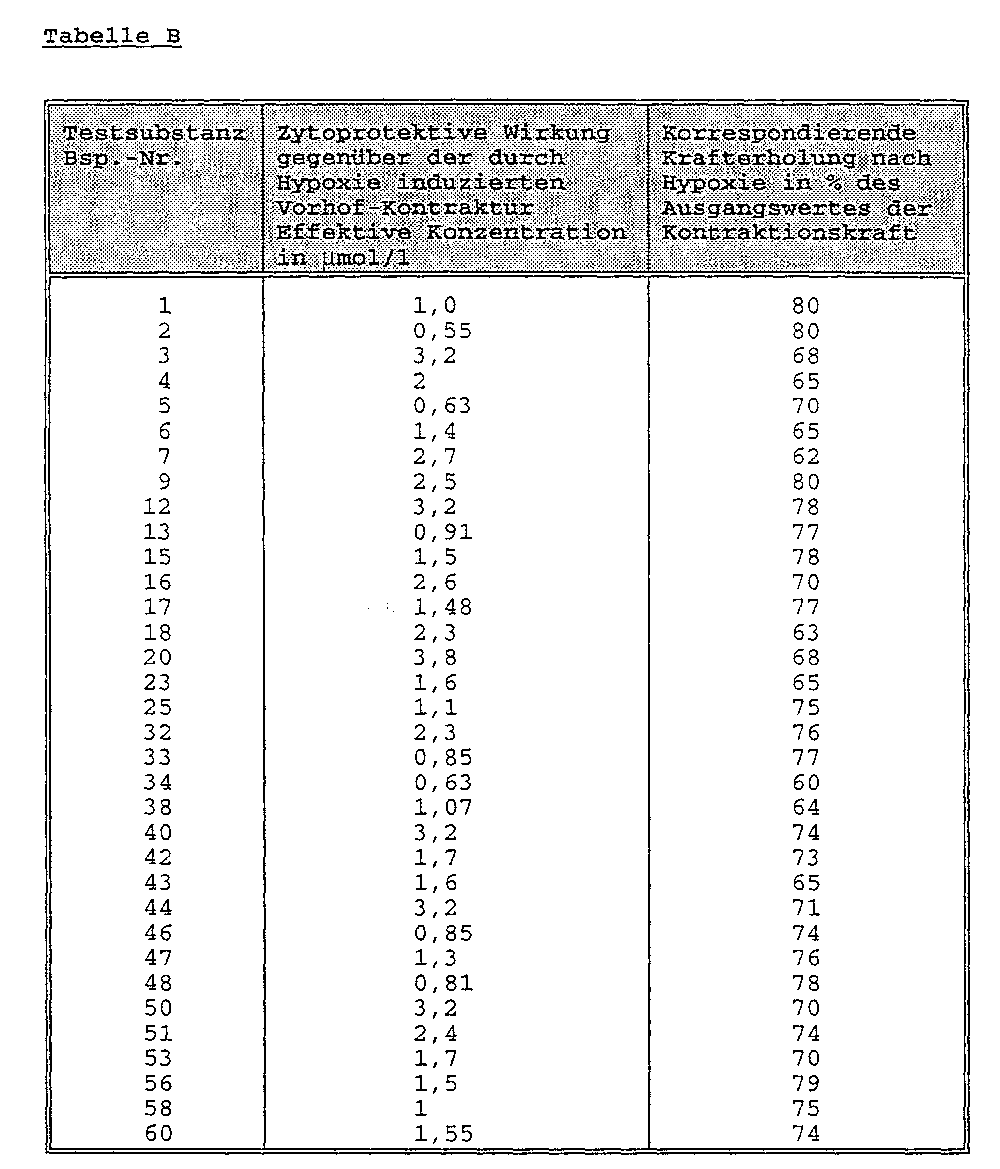
   In in vitro-Versuchen an isolierten Herzmuskelzellen zeigten die Verbindungen cytoprotektive Wirkungen gegenüber cytotoxischen Substanzen wie Veratridin oder einer Kombination aus Oligomycin und Desoxyglucose.
3. In vitro-Nachweis der herzfrequenzsenkenden Wirkung.
   Der direkte Einfluß der Wirksubstanzen auf die Herzfrequenz (FRQ) wurde an spontan schlagenden, isolierten rechten Vorhöfen von männlichen Pirbright-white Meerschweinchen der Gewichtsklasse 250 - 300 g geprüft. In der nachfolgenden Tabelle C ist als FRQ 75 diejenige Konzentration in µmol/l angegeben, bei der es 20 Minuten nach der Substanzgabe zu einer Abnahme der Frequenz auf 75 % des Ausgangswertes kommt. Die für die Testsubstanzen in der Tabelle C angegebenen Beispielnummern beziehen sich auf die nachstehenden Herstellungsbeispiele.

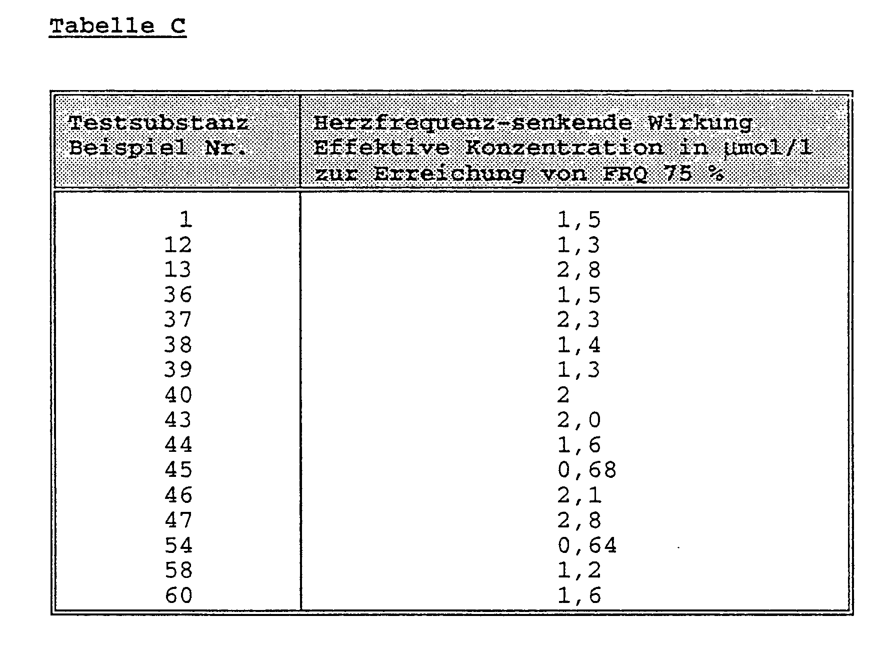

In in vivo-Versuchen lassen sich die cytoprotektiven Wirkungen der Substanzen unter ischämischen Bedingungen an anästhesierten Ratten nachweisen, bei denen die Blutversorgung des Herzmuskels kurzfristig durch eine Coronarligatur unterbrochen und so eine Unterversorgung mit Sauerstoff herbeigeführt wird. Nach Einnahme der Substanzen sind die bei 5-minütiger Unterbrechung der Blutversorgung des Herzmuskels auftretenden Arrhythmien gegenüber einem Kontrollversuch deutlich verringert und ischämiebedingte EKG-Veränderungen sind weniger ausgeprägt.

Aufgrund ihrer vorstehend beschriebenen Wirkungen sind die Verbindungen der Formel I als herzwirksame Arzneimittel für größere Säugetiere, insbesondere Menschen, geeignet zur Behandlung von ischämischen Zuständen wie z. B. bei koronaren Herzkrankheiten und deren Folgeerscheinungen wie z. B. Herzinsuffizienz. Die zu verwendenden Dosen können individuell verschieden sein und variieren naturgemäß je nach Art des zu behandelnden Zustandes, der verwendeten Substanz und der Applikationsform. Zum Beispiel werden parenterale Formulierungen im allgemeinen weniger Wirkstoffe enthalten als orale Präparate. Im allgemeinen eignen sich jedoch für Applikationen an größeren Säugetieren, insbesondere Menschen, Arzneiformen mit einem Wirkstoffgehalt von 1 bis 200 mg pro Einzeldosis.

Als Heilmittel können die Verbindungen der Formel I mit üblichen pharmazeutischen Hilfsstoffen in galenischen Zubereitungen wie z. B. Tabletten, Kapseln, Suppositorien oder Lösungen enthalten sein. Diese galenischen Zubereitungen können nach an sich bekannten Methoden hergestellt werden unter Verwendung üblicher fester oder flüssiger Trägerstoffe wie z. B. Milchzucker, Stärke oder Talkum oder flüssigen Paraffinen und/oder unter Verwendung von üblichen pharmazeutischen Hilfsstoffen, beispielsweise Tablettensprengmitteln, Lösungsvermittlern oder Konservierungsmitteln.

Die nachfolgenden Beispiele sollen die Erfindung näher erläutern, jedoch deren Umfang in keiner Weise beschränken.

Die Strukturen der neuen Verbindungen wurden durch spektroskopische Untersuchungen, insbesondere durch Analyse der NMR-,Massen-, IR-/oder UV-Spektren gesichert.

### Beispiel 1:

### 3-{3-[N-(2-(3,4-Dimethoxyphenyl)-ethyl)-N-methylamino]-propyloxy}-5-phenyl-1,2,4-thiadiazol.

A) 38,7 g Thiobenzamid wurden in 380 ml Xylol aufgeschlämmt. Die Aufschlämmung wurde auf 5 °C abgekühlt und dann wurden 24,8 ml Oxalylchlorid so zugetropft, daß die Temperatur auf 4 bis 6 °C gehalten wurde. Nach 30-minütigem Rühren bei 40 °C und weiterem 90-minütigem Rühren bei 90 °C wurden 58 ml Azidotrimethylsilan so zugetropft, daß die Temperatur 110 °C nicht überschritt. Anschließend wurde das Gemisch weitere 2 Stunden bei 100 °C gerührt und dann auf Raumtemperatur abgekühlt. Zur Aufarbeitung wurden sodann 200 ml Isopropanol zugegeben und das Reaktionsgemisch wurde mehrere Stunden kristallisieren gelassen. Das gebildete Kristallisat wurde abgesaugt, mit Isopropanol gewaschen und getrocknet. Es wurden 35 g 5-Phenyl-1,2,4-thiadiazol-3-ol als gelber Feststoff erhalten.
B) 20,6 g der vorstehend erhaltenen Substanz wurden in 275 ml Dimethylformamid gelöst. Zu der Lösung wurden 14,3 g Kalium-tert.-butylat gegeben und das Reaktionsgemisch wurde anschließend auf 80 °C erhitzt und 2 Stunden bei dieser Temperatur gerührt. Anschließend wurde auf 60 °C abgekühlt und es wurden 12,6 ml 1-Brom-3-chlorpropan zugegeben. Die Zugabe erfolgte so, daß die Temperatur von 60 °C nicht überschritten wurde. Anschließend wurde noch weitere 2 Stunden bei 60 °C gerührt. Dann wurde das Reaktionsgemisch zur Aufarbeitung auf Eiswasser gegossen und zweimal mit Methyl-tert.-butylether extrahiert. Die organischen Phasen wurden mit Wasser gewaschen und eingeengt. Das als Rückstand verbleibende Öl wurde anschließend mit 100 ml eines Gemisches aus Petrolether/Methyltert.-butylether 1:1 und 20 g Kieselgel gerührt. Nach Abfiltrieren des Kieselgels wurde die Lösung eingeengt. Es wurden 28 g 3-(5-Phenyl-1,2,4-thiadiazol-3-yloxy)-propylchlorid als Öl erhalten.
C) 28 g der vorstehend erhaltenen Substanz wurden mit 21,4 g N-Methylhomoveratrylamin versetzt und das Gemisch wurde auf 100 °C erhitzt. Nach 15-minütigem Rühren wurden 15,2 ml Triethylamin und 15,2 ml Dimethylformamid zugegeben und das Reaktionsgemisch wurde weitere 2 Stunden bei 120 °C gerührt. Zur Aufarbeitung wurde das Reaktionsgemisch abgekühlt und mit 100 ml eines Gemisches Essigsäureethylester/Methanol 9:1 und 100 ml Wasser versetzt und 30 Minuten bei Raumtemperatur gerührt. Anschließend wurde die organische Phase abgetrennt, die wäßrige Phase mit 50 ml eines Gemisches Essigsäureethylester/Methanol 9:1 ausgeschüttelt, die organischen Phasen vereinigt, getrocknet und bis zur Trockene eingeengt. Als Rückstand wurden 33 g der Titelverbindung als Öl erhalten.
   Zur Salzbildung wurde die ölige Titelverbindung in 200 ml Isopropanol gelöst, und in die Lösung wurde unter Eiskühlung Chlorwasserstoffgas eingeleitet. Das erhaltene Kristallisat wurde abfiltriert und getrocknet. Es wurden 23,6 g 3-{3-[N-(2-(3,4-Dimethoxyphenyl)-ethyl)-N-methylamino]-propyloxy}-5-phenyl-1,2,4-thiadiazol-hydrochlorid als farbloses Kristallisat mit einem Schmelzpunkt von 156 bis 158 °C erhalten.

### Beispiel 2:

### 3-{3-[N-(2-(3,4-Dimethoxyphenyl)-ethyl)-N-methylamino]-propyloxy}-5-phenyl-1,2,4-oxadiazol.

A) 26 g Benzoylisocyanat wurden mit 40 g Azidotrimethylsilan versetzt und das Reaktionsgemisch wurde 7 Stunden unter Rühren am Rückfluß erhitzt, anschließend mehrere Stunden bei Raumtemperatur stehengelassen und nochmals 7 Stunden am Rückfluß erhitzt. Zur Aufarbeitung wurde das Reaktionsgemisch bis zur Trockene eingeengt und der verbleibende Rückstand wurde in 30 ml Methanol gelöst. Es setzte spontan eine Kristallisation ein. Die ausgefallenen Kristalle wurden abgesaugt und getrocknet. Es wurden 20,2 g 5-Phenyl-1,2,4-oxadiazol-3-ol als farbloser Feststoff erhalten.
B) 1,6 g des vorstehend erhaltenen Produktes wurden in 16 ml Dimethylformamid gelöst. Zu der Lösung wurden portionsweise 0,5 g Natriumhydrid zugegeben, und das Reaktionsgemisch wurde auf 80 °C erhitzt und 30 Minuten gerührt. Anschließend wurden 2 ml 1-Brom-3-chlorpropan zugetropft und das Gemisch wurde weitere 30 Minuten bei 80 °C gerührt. Zur Aufarbeitung wurde das Reaktionsgemisch anschließend auf Raumtemperatur gekühlt, mit wenig Wasser versetzt und mit Essigsäureethylester ausgeschüttelt. Die vereinigten organischen Phasen wurden über Natriumsulfat getrocknet und dann zur Trockene eingeengt. Das als Rückstand verbleibende Öl wurde durch Mitteldruckchromatographie an feinkörnigem Kieselgel (Lichroprep Si 60^{R}) bei einem Druck von 20 bar unter Verwendung von Cyclohexan/-Ethylacetat 9:1 als Elutionsmittel gereinigt. Es wurden 1 g 3-(5-Phenyl-1,2,4-oxadiazol-3-yloxy)-propylchlorid als Öl erhalten.
C) 1 g des vorstehend erhaltenen Produktes wurden mit 1,5 g N-Methylhomoveratrylamin versetzt und das Reaktionsgemisch wurde 1 Stunde auf 130 °C und anschließend 30 Minuten auf 140 °C erhitzt. Zur Aufarbeitung wurde das Reaktionsgemisch auf Raumtemperatur abgekühlt, mit wenig Wasser versetzt und mit Essigsäureethylester ausgeschüttelt. Die organischen Phasen wurden vereinigt, über Natriumsulfat getrocknet und zur Trockene eingeengt. Die erhaltene rohe ölige Titelverbindung wurde durch Mitteldruckchromatographie an feinkörnigem Kieselgel (Lichroprep Si 60^{R}) bei einem Druck von 12 bar unter Verwendung von Essigsäureethylester/Methanol 99:1 als Elutionsmittel gereinigt. Es wurden 1,07 g der Titelverbindung als Öl erhalten.
   Zur Salzbildung wurde die ölige Titelverbindung in Methanol gelöst und die Lösung wurde durch Zugabe von Chlorwasserstoffgas auf pH 1 eingestellt. Anschließend wurde bis zur Trübung Diethylether zugegeben und das Gemisch zur Kristallisation stehengelassen. Die ausgefallenen Kristalle wurden abgesaugt und getrocknet. Es wurden 1 g 3-{3-[N-(2-(3,4-Dimethoxyphenyl)-ethyl)-N-methylamino]-propyloxy}-5-phenyl-1,2,4-oxadiazol-hydrochlorid als farbloser Feststoff mit einem Schmelzpunkt von 160 bis 162 °C erhalten.

### Beispiel 3:

### 3-{3-[N-(2-(3,4-Dimethoxyphenyl)-ethyl)-N-methylamino]-2-hydroxypropyloxy}-5-phenyl-1,2,4-thiadiazol.

A) 5 g 5-Phenyl-1,2,4-thiadiazol-3-ol (Herstellung siehe Beispiel 1 A) wurden in 100 ml Dimethylformamid gelöst. Die Lösung wurde portionsweise mit 1 g Natriumhydrid versetzt und auf 60 °C erhitzt. Dann wurde eine Lösung von 2,4 ml Epichlorhydrin in 10 ml Dimethylformamid zugetropft und das Reaktionsgemisch wurde 3 Stunden bei 80 °C gerührt. Zur Aufarbeitung wurde das Reaktionsgemisch auf Raumtemperatur abgekühlt, auf Wasser gegossen und zweimal mit Essigsäureethylester extrahiert. Die organische Phase wurde abgetrennt, über Natriumsulfat getrocknet und zur Trockene eingeengt. Das als Rückstand erhaltene Öl wurde durch Chromatographie über feinkörnigem Kieselgel bei leicht erhöhtem Druck (Flash-Chromatographie) unter Verwendung von Methyl-tert.-butylether/Petrolether 1:4 als Elutionsmittel gereinigt. Es wurden 4 g 2-(5-Phenyl-1,2,4-thiadiazol-3-yloxymethyl)-oxiran erhalten.
B) 3 g des vorstehend erhaltenen Produktes wurden in 30 ml Ethanol gelöst. Zu der Lösung wurden 2 g N-Methylhomoveratrylamin und 0,2 ml Pyridin gegeben und das Reaktionsgemisch wurde 2 Stunden bei 80 °C gerührt. Zur Aufarbeitung wurde das Reaktionsgemisch auf Wasser gegossen, zweimal mit Dichlormethan extrahiert, die organischen Phasen vereinigt, getrocknet und eingeengt. Die als Rückstand verbleibende rohe ölige Titelverbindung wurde durch Chromatographie über feinkörnigem Kieselgel bei leicht erhöhtem Druck (Flash-Chromatographie) unter Verwendung von Essigsäureethylester/Methanol 20:1 als Elutionsmittel gereinigt. Es wurden 7 g der Titelverbindung als Öl erhalten.
   Zur Salzbildung wurde die ölige Titelverbindung in Isopropanol gelöst, und die Lösung wurde mit Chlorwasserstoffgas gesättigt. Das ausgefallene Kristallisat wurde abgesaugt und getrocknet. Es wurden 5,9 g 3-{3-[N-(2-(3,4-Dimethoxyphenyl)-ethyl)-N-methylamino]-2-hydroxypropyloxy}-5-phenyl 1,2,4-thiadiazol-hydrochlorid als farbloser Feststoff mit einem Schmelzpunkt von 143 °C erhalten.

### Beispiel 4:

### 3-{3-[N-(2-(3,4-Methylendioxyphenyl)-ethyl)-N-methylamino]-propyloxy}-5-phenyl-1,2,4-thiadiazol.

A) 25 g Methylendioxyphenylessigsäure wurden mit 25 ml Thionylchlorid vermischt und das Reaktionsgemisch wurde 2 Stunden bei 80 °C gerührt. Anschließend wurde überschüssiges Thionylchlorid abdestilliert und der Rückstand wurde zweimal in Toluol aufgenommen und erneut eingeengt. Es wurden 25 g rohes 3,4-Methylendioxyphenylessigsäurechlorid als gelbes Öl erhalten.
B) Eine Lösung von 25 g des vorstehend erhaltenen Säurechlorides in 50 ml Tetrahydrofuran wurde unter Eiskühlung zu einem Gemisch aus 100 ml einer 40 %-igen wäßrigen Methylamin-Lösung und 200 ml Tetrahydrofuran getropft. Nach Beendigung der Zugabe wurde das Reaktionsgemisch weitere 2 Stunden bei Raumtemperatur gerührt und anschließend auf 10 % des ursprünglichen Volumens eingeengt. Der verbleibende Rückstand wurde dreimal mit Essigsäureethylester extrahiert. Die vereinigten Essigsäureethylesterextrakte wurden eingeengt und der Rückstand wurde in Methyl-tert.butylether aufgenommen. Nach einiger Zeit setzte Kristallisation ein. Die Kristalle wurden abgesaugt, gewaschen und getrocknet. Es wurden 17 g N-Methyl-3,4-methylendioxyphenylessigsäureamid erhalten.
C) Zu einer Lösung von 17 g des vorstehend erhaltenen Produktes in 300 ml Tetrahydrofuran wurden portionsweise 17 g Natriumborhydrid zugesetzt. Anschließend wurde das Reaktionsgemisch 20 Minuten gerührt, dann wurden 26 ml Eisessig zugetropft und das Gemisch wurde nochmals 3 Stunden bei 80 °C gerührt. Zur Aufarbeitung wurde das Reaktionsgemisch auf Raumtemperatur abgekühlt, auf Wasser gegossen und mit 20 %-iger Salzsäurelösung auf pH 1 eingestellt. Das Gemisch wurde 30 Minuten gerührt und anschließend durch Zugabe von 20 %-iger wäßriger Natriumhydroxid-Lösung basisch gestellt und dreimal mit Dichlormethan extrahiert. Die organischen Phasen wurden vereinigt, getrocknet und eingeengt. Es wurden 13 g N-[2-(3,4-Methylendioxyphenyl)-ethyl]-N-methylamin als Öl erhalten.
D) 4,5 g des vorstehend erhaltenen Produktes wurden mit 6 g 3-(5-Phenyl-1,2,4-thiadiazol-3-yloxy)-propylchlorid (Herstellung siehe Beispiel 1 B)) nach der in Beispiel 1 C) beschriebenen Methode umgesetzt. Die erhaltene rohe ölige Titelverbindung wurde durch Chromatographie über feinkörnigem Kieselgel bei leicht erhöhtem Druck (Flash-Chromatographie) unter Verwendung von Essigsäureethylester/Cyclohexan 1:1 als Elutionsmittel gereinigt. Es wurden 5 g der Titelverbindung als Öl erhalten.
   Zur Salzbildung wurde die ölige Titelverbindung in Isopropanol gelöst und die Lösung wurde mit Chlorwasserstoffgas gesättigt. Das ausgefallene Kristallisat wurde abgesaugt und getrocknet. Es wurden 2,71 g 3-(3-[N-(2-(3,4-Methylendioxyphenyl)-ethyl)-N-methylamino]-propyloxy}-5-phenyl-1,2,4-thiadiazol-hydrochlorid als farbloser Feststoff mit einem Schmelzpunkt von 153 °C erhalten.

### Beispiel 5:

### 3-{3-[N-(2-(3,4-Dimethoxyphenyl)-ethyl)-N-methylamino]-propyloxy}-5-phenyl-isoxazol.

A) Zu einer Lösung von 100 g trans-Zimtsäureethylester in 500 ml Dichlormethan wurden bei 0 °C 91 g Brom so langsam über einen Zeitraum von 7 Stunden verteilt zugetropft, daß größere Bromkonzentrationen vermieden wurden. Das Reaktionsgemisch wurde mehrere Stunden bei Raumtemperatur stehengelassen, dann wurden noch weitere 2 ml Brom langsam zugefügt und das Reaktionsgemisch nochmals 3 Stunden bei Raumtemperatur gerührt. Anschließend wurde unter vermindertem Druck eingeengt. Es wurden 193 g 2,3-Dibrom-3-phenyl-propionsäureethylester erhalten.
B) 164,5 g des vorstehend erhaltenen Produktes, 46,6 g Imidazol und 339 ml Triethylamin wurden in 2 1 Toluol gelöst, und die Lösung wurde mehrere Stunden bei 100 °C gerührt. Anschließend wurden die ausgefallenen Salze abfiltriert, die organische Phase mit gesättigter wäßriger Natriumchloridlösung gewaschen, über Magnesiumsulfat getrocknet und eingeengt. Der erhaltene Rückstand wurde aus Diethylether kristallisiert. Es wurden 81,2 g 3-(Imidazol-1-yl)-zimtsäureethylester erhalten.
C) 72 g Hydroxylammoniumchlorid und 550 g einer 30 %-igen Natriummethylatlösung wurden in 2,5 l Methanol gelöst und die Lösung wurde 1 Stunde am Rückfluß erhitzt. Dann wurden 82 g 3-(Imidazol-1-yl)-zimtsäureethylester zugegeben und das Reaktionsgemisch wurde weitere 3 Stunden am Rückfluß erhitzt. Anschließend wurde das Reaktionsgemisch abgekühlt und durch Zugabe von 3 n Salzsäurelösung ein pH-Wert von 1 eingestellt. Das Reaktionsprodukt wurde mit Dichlormethan extrahiert, die organische Phase mit Magnesiumsulfat getrocknet und eingeengt. Beim Einengen kristallisierten 30,3 g 3-Hydroxy-5-phenyl-isoxazol aus.
D) 29 ml 1-Brom-3-chlorpropan wurden in 300 ml Dimethylformamid gelöst und zu der Lösung wurden 24 g Kaliumcarbonat zugefügt. Zu dem Reaktionsgemisch wurde eine Lösung von 18 g N-[2-(3,4-Dimethoxyphenyl)-ethyl]-N-methylamin in 50 ml Dimethylformamid unter Rühren innerhalb von 2 Stunden zugetropft. Anschließend wurde das Reaktionsgemisch noch 1 Stunde gerührt. Dann wurden die gebildeten Salze abfiltriert. Das Filtrat wurde bei einer Badtemperatur von maximal 50 °C eingeengt. Der Rückstand wurde in 200 ml 0,5 m Zitronensäurelösung gelöst und die Lösung wurde zur Entfernung von unumgesetztem Bromchlorpropan mit Methyl-tert.-butylether extrahiert. Die wäßrige Phase wurde sodann durch Zugabe von Natriumhydrogencarbonat leicht alkalisch gestellt und mehrmals mit Essigsäureethylester extrahiert. Anschließend wurden die vereinigten Essigsäureethylesterextrakte mit Magnesiumsulfat getrocknet und eingeengt. Als Rückstand wurden 21 g 3-[N-(2-(3,4-Dimethoxyphenyl)-ethyl)-N-methylamino]-propylchlorid erhalten.
E) 3,5 g 3-Hydroxy-5-phenyl-isoxazol wurden in 100 ml Dimethylformamid gelöst. Der Lösung wurden 3 g feingepulvertes Kaliumcarbonat zugesetzt und das Reaktionsgemisch wurde 15 Minuten bei 100 °C unter Stickstoffatmosphäre erhitzt. Dann wurden bei 60 °C 5,8 g 3-[N-(2-(3,4-Dimethoxyphenyl)-ethyl)-N-methylamino]-propylchlorid portionsweise zugegeben. Das Reaktionsgemisch wurde 4 Stunden bei 100 °C gerührt, anschließend wurden die gebildeten Salze abfiltriert, das Filtrat bis zur Trockene eingeengt und der Rückstand in Methyl-tert.-butylether gelöst. Die Lösung wurde anschließend mit wäßriger Natriumcarbonatlösung gewaschen, mit Magnesiumsulfat getrocknet und zur Trockene eingeengt. Die als Rückstand verbleibende rohe ölige Titelverbindung wurde durch Flash-Chromatographie unter Verwendung von Essigsäureethylester als Elutionsmittel gereinigt. Es wurden 4,75 g der Titelverbindung erhalten.
   Zur Salzbildung wurde die vorstehend erhaltene ölige Titelverbindung in Aceton gelöst und zu dieser Lösung wurde tropfenweise eine etherische Salzsäurelösung zugegeben. Das Gemisch wurde mehrere Stunden bei 5 °C stehengelassen und das gebildete Kristallisat wurde abgesaugt und getrocknet. Es wurden 4,6 g 3-{3-[N-(2-(3,4-Dimethoxyphenyl)-ethyl)-N-methylamino]-propyloxy}-5-phenylisoxazol-hydrochlorid mit einem Schmelzpunkt von 156 °C erhalten.

### Beispiel 6:

### 3-{3-[N-(2-(4-(Methylsulfonylamino)-phenyl)-ethyl)-N-methylamino]-propyloxy}-5-phenyl-1,2,4-thiadiazol.

A) Zu einer Lösung von 25 g 2-(4-Aminophenyl)-ethanol in 200 ml Pyridin wurden unter Eiskühlung 30 ml Methansulfonylchlorid zugetropft. Nach beendigter Zugabe wurde 6 Stunden bei Raumtemperatur gerührt. Anschließend wurde das Pyridin im Rotationsverdampfer abgezogen und der verbleibende ölige Rückstand wurde auf Wasser gegossen und mit Dichlormethan extrahiert. Die organische Phase wurde getrocknet und eingeengt. Das als Rückstand verbleibende Öl wurde durch Flash-Chromatographie unter Verwendung von Essigsäureethylester/Cyclohexan 1:1 als Elutionsmittel gereinigt. Es wurden 29,9 g eines Öls erhalten, welches zur Kristallisation in Methyl-tert.-butylether gelöst wurde. Die ausgefallenen Kristalle wurden abgesaugt und getrocknet. Es wurden 25,2 g 2-[4-(Methylsulfonyl)-phenyl]-ethyl-methylsulfat erhalten.
B) Zu einer Lösung von 14 g des vorstehend erhaltenen Produktes in 27 ml absolutem Ethanol wurde 33 %-ige ethanolische Methylamin-Lösung im Laborautoklaven zugesetzt. Anschließend wurde das Reaktionsgemisch zweimal 8 Stunden auf 85 °C Lösungstemperatur erhitzt. Zur Aufarbeitung wurde das Reaktionsgemisch eingeengt, und das erhalten Öl wurde durch Flash-Chromatographie unter Verwendung von Dichlormethan/Methanol/konzentrierte wäßrige Ammoniaklösung 35:15:2 als Elutionsmittel gereinigt. Das gereinigte Produkt wurde zur Kristallisation in Isopropanol gelöst. Die ausgefallenen Kristalle wurden abgesaugt und getrocknet. Es wurden 9,8 g N-[2-(4-(Methylsulfonylamino)-phenyl)-ethyl]-N-methylamin erhalten.
C) 3,5 g des vorstehend erhaltenen Produktes wurden mit 3,5 g 3-(5-Phenyl-1,2,4-thiadiazol-3-yloxy)-propylchlorid (Herstellung siehe Beispiel 1 B)) nach der in Beispiel 1 C) beschriebenen Methode umgesetzt. Die erhaltene ölige rohe Titelverbindung wurde durch Flash-Chromatographie unter Verwendung von Essigsäureethylester/Methanol 10:3 als Elutionsmittel gereinigt. Es wurden 3 g der Titelverbindung erhalten.
   Zur Salzbildung wurde die erhaltene ölige Titelverbindung in 200 ml Isopropanol gelöst und in die Lösung wurde unter Eiskühlung Chlorwasserstoffgas eingeleitet. Das gebildete Kristallisat wurde abgesaugt und getrocknet. Es wurden 1,15 g 3-{3-[N-(2-(4-(Methylsulfonylamino)-phenyl)-ethyl)-N-methylamino]-propyloxy}-5-phenyl-1,2,4-thiadiazol-hydrochlorid als farbloser Feststoff mit einem Schmelzpunkt von 186 °C erhalten.

### Beispiel 7:

### 3-{3-[N-(2-(3,5-Dimethoxyphenyl)-ethyl)-N-methylamino]-propyloxy}-5-phenyl-1,2,4-thiadiazol.

A) 22 g 3,5-Dimethoxybenzylalkohol wurden mit 50 ml Phosphortribromid unter Eiskühlung versetzt und das Reaktionsgemisch wurde 30 Minuten auf 80 °C erhitzt. Zur Aufarbeitung wurde das Reaktionsgemisch auf Eiswasser gegossen und mit Methyl-tert.-butylether extrahiert. Die organischen Phasen wurden vereinigt getrocknet und eingeengt. Es wurden 23 g 3,5-Dimethoxybenzylbromid als Öl erhalten.
B) 23 g des vorstehend erhaltenen Produktes wurden mit 100 ml Triethylenglycol und 10 g Kaliumcyanid versetzt. Das Reaktionsgemisch wurde 1 Stunde auf 140 °C erhitzt. Anschließend wurde das Reaktionsgemisch abgekühlt und zur Aufarbeitung in Wasser gegossen, mit Essigsäureethylester extrahiert, die organische Phase getrocknet und eingeengt. Das erhaltene Öl wurde durch Flash-Chromatographie unter Verwendung von Essigsäureethylester/Cyclohexan 2:1 als Elutionsmittel gereinigt. Es wurden 18 g rohes 3,5-Dimethoxybenzylcyanid als Öl erhalten.
C) 18 g des vorstehend erhaltenen Produktes wurden in 100 ml Ethanol gelöst. Zu der Lösung wurden 100 ml 20 %-ige wäßrige Natriumhydroxid-Lösung gegeben. Das Reaktionsgemisch wurde 3 Stunden unter Rückfluß gekocht. Zur Aufarbeitung wurde das Reaktionsgemisch anschließend abgekühlt und eingeengt. Der Rückstand wurde durch Zugabe von 20 %-iger wäßriger Salzsäurelösung sauergestellt und mit Dichlormethan extrahiert. Die organische Phase wurde bis zur Trokkene eingeengt. Es wurden 15 g 3,5-Dimethoxyphenylessigsäure als farbloser Feststoff erhalten.
D) 15 g des vorstehend erhaltenen Produktes wurden in 150 ml Dichlormethan gelöst und der Lösung wurde eine katalytische Menge Dimethylformamid zugesetzt. Anschließend wurden 6,7 ml Oxalylchlorid unter Eiskühlung zugetropft. Das Reaktionsgemisch wurde 2 Stunden unter Rückfluß gekocht und dann eingeengt. Der verbleibende Rückstand wurde in 100 ml Essigsäureethylester aufgenommen und zu einem Gemisch von 450 ml Essigsäureethylester und 100 ml einer 40 %-igen wäßrigen Methylamin-Lösung getropft. Das Reaktionsgemisch wurde 2 Stunden bei Raumtemperatur gerührt und dann eingeengt. Zu dem verbleibenden Rückstand wurden nacheinander Wasser, Essigsäureethylester und einige Tropfen Methanol zugegeben und das Reaktionsgemisch wurde bei Raumtemperatur gerührt. Anschließend wurde die organische Phase abgetrennt, getrocknet und eingeengt. Der Rückstand wurde aus Methyl-tert.-butylether umkristallisiert. Es wurden 14 g N-Methyl-3,5-dimethoxyphenylessigsäureamid als farbloser Feststoff erhalten.
E) 14 g des vorstehend erhaltenen Produktes wurden in 200 ml Tetrahydrofuran gelöst. Zu der Lösung wurden 12 g Natriumborhydrid gegeben und anschließend 19 ml Eisessig langsam zugetropft. Das Reaktionsgemisch wurde 3 Stunden unter Rückfluß gekocht und dann auf Raumtemperatur abgekühlt. Zur Aufarbeitung wurde das Reaktionsgemisch auf Wasser gegossen und dreimal mit Essigsäureethylester, welches einige Tropfen Methanol enthielt, extrahiert. Der Essigsäureethylesterextrakt wurde eingeengt und der Rückstand durch Zugabe von 20 %-iger wäßriger Salzsäurelösung auf pH 1 eingestellt. Anschließend wurde 30 Minuten gerührt und das Gemisch durch Zugabe von Natriumcarbonat alkalisch gestellt. Dann wurde dreimal mit Dichlormethan extrahiert, die organischen Phasen vereinigt, getrocknet und eingeengt. Das verbleibende Öl wurde durch Flash-Chromatographie unter Verwendung von Essigsäureethylester/Methanol/konzentrierte wäßrige Ammoniaklösung 15:3:0,1 als Elutionsmittel gereinigt. Es wurden 4,3 g N-[2-(3,5-Dimethoxyphenyl)-ethyl]-N-methylamin als gelbes Öl erhalten.
F) 2,5 g des vorstehend erhaltenen Produktes wurden mit 3,3 g 3-(5-Phenyl-1,2,4-thiadiazol-3-yloxy)-propylchlorid (Herstellung siehe Beispiel 1 B)) nach der in Beispiel 1 C) beschriebenen Methode umgesetzt. Die erhaltene rohe ölige Titelverbindung wurde durch Flash-Chromatographie unter Verwendung von Essigsäureethylester/Methanol/konzentrierte wäßrige Ammoniaklösung 45:5:0,1 als Elutionsmittel gereinigt. Es wurden 4 g der Titelverbindung als Öl erhalten.
   Zur Salzbildung wurde die ölige Titelverbindung in Isopropanol gelöst und eine ausreichende Menge Chlorwasserstoffgas in diese Lösung eingeleitet. Anschließend wurde die Lösung zur Trockene eingeengt und das erhalten Öl mit Diethylether und einer kleinen Menge Isopropanol gerührt. Die entstandenen Kristalle wurden abgesaugt und getrocknet. Es wurden 2 g 3-{3-[N-(2-(3,5-Dimethoxyphenyl)-ethyl)-N-methylamino]-propyloxy}-5-phenyl-1,2,4-thiadiazol-hydrochlorid als farbloses Kristallisat mit einem Schmelzpunkt von 129 °C erhalten.

### Beispiel 8:

### 3-{3-[N-(2-(4-Nitrophenyl)-ethyl)-N-methylamino]-propyloxy}-5-phenyl-1,2,4-thiadiazol.

A) 25 g 2-(4-Nitrophenyl)-ethylamin-hydrochlorid wurden in 500 ml Dichlormethan aufgeschlämmt und mit 33 ml Triethylamin versetzt. Zu dem Reaktionsgemisch wurden 52 g Trifluoressigsäureanhydrid bei 0 °C langsam zugetropft. Nach beendeter Zugabe wurde das Reaktionsgemisch 2 Stunden bei Raumtemperatur gerührt und anschließend zweimal mit je 200 ml 5 %-iger wäßriger Salzsäurelösung und zweimal mit gesättigter wäßriger Natriumhydrogencarbonat-Lösung ausgeschüttelt. Dann wurde die Dichlormethan-Phase mit Natriumsulfat getrocknet und eingeengt. Der Rückstand wurde in 500 ml Tetrahydrofuran aufgenommen und mit 44 ml Methyliodid versetzt. Anschließend wurden portionsweise 10 g Natriumhydrid zugegeben und das Reaktionsgemisch 2 Stunden bei Raumtemperatur gerührt. Nach Beendigung der Reaktion wurde das Reaktionsgemisch über Kieselgel filtriert und eingeengt. Der erhaltene Rückstand wurde durch Flash-Chromatographie unter Verwendung von Methyl-tert.butylether/Cyclohexan 2:1 als Elutionsmittel gereinigt. Das erhalten Öl wurde in 300 ml Methanol gelöst. Zu der Lösung wurde 50 ml einer 20 %-igen wäßrigen Natriumhydroxid-Lösung gegeben und das Gemisch wurde 2 Stunden bei Raumtemperatur gerührt. Anschließend wurde die Hauptmenge Methanol abdestilliert und es wurden 100 ml Wasser zugegeben. Die Lösung wurde zweimal mit je 240 ml Dichlormethan ausgeschüttelt. Die organischen Phasen wurden vereinigt, getrocknet und eingeengt. Das verbleibende ölige Reaktionsprodukt wurde durch Flash-Chromatographie unter Verwendung von Dichlormethan/Methanol/konzentrierte wäßrige Ammoniaklösung als Elutionsmittel gereinigt. Es wurden 8,9 g N-[2-(4-Nitrophenyl)-ethyl]-N-methylamin als Öl erhalten.
B) 3,5 g des vorstehend erhaltenen Produktes wurden mit 3,5 g 3-(5-Phenyl-1,2,4-thiadiazol-3-yloxy)-propylchlorid (Herstellung siehe Beispiel 1 B) nach der in Beispiel 1 C) beschriebenen Methode umgesetzt. Die erhaltene rohe ölige Titelverbindung wurde durch Flash-Chromatographien zunächst unter Verwendung von Essigsäureethylester/Cyclohexan 1:2 und dann unter Verwendung von Essigsäureethylester/Methanol 25:1 als Elutionsmittel gereinigt. Es wurden 5 g der Titelverbindung als Öl erhalten.
   Zur Salzbildung wurde die ölige Titelverbindung in Isopropanol gelöst und zu der Lösung wurde eine etherische Salzsäurelösung zugetropft. Das ausgefallene Kristallisat wurde anschließend abgesaugt und getrocknet. Es wurden 3,11 g 3-{3-[N-(2-(4-Nitrophenyl)-ethyl)-N-methylamino]-propyloxy}-5-phenyl-1,2,4-thiadiazol-hydrochlorid erhalten.

### Beispiel 9:

### 3-{3-[N-(2-(4-Aminophenyl)-ethyl)-N-methylamino]-propyloxy}-5-phenyl-1,2,4-thiadiazol.

2,1 g 3-{3-[N-(2-(4-Nitrophenyl)-ethyl)-N-methylamino]-propyloxy}-5-phenyl-1,2,4-thiadiazol (Herstellung siehe Beispiel 8)) wurden in einem Gemisch von 75 ml Ethanol und 75 ml Methanol gelöst und mit einer katalytischen Menge Raney-Nickel versetzt. Das Reaktionsgemisch wurde anschließend 2,5 Stunden bei Raumtemperatur mit Wasserstoff bei einem Wasserstoffdruck von 5 bar hydriert. Der Katalysator wurde abfiltriert und das Filtrat zur Trockene eingeengt. Die erhaltene ölige rohe Titelverbindung wurde durch Mitteldruckchromatographie über feinkörnigem Kieselgel (Lichroprep Si 60^{R}) unter Verwendung von Essigsäureethylester/Methanol 95:5 als Elutionsmittel gereinigt. Es wurden 1,1 g der Titelverbindung als Öl erhalten.

Zur Salzbildung wurde die ölige Titelverbindung in Ethanol gelöst und der Lösung wurde etherische Salzsäurelösung zugesetzt. Die ausgefallenen Kristalle wurden abgesaugt und getrocknet. Es wurden 0,48 g 3-{3-[N-(2-(4-Aminophenyl)-ethyl)-N-methylamino]-propyloxy}-5-phenyl-1,2,4-thiadiazol-hydrochlorid als farbloser Feststoff mit einem Schmelzpunkt von 183 bis 186 °C erhalten.

### Beispiel 10:

### 3-{3-[N-(2-(4-Acetaminophenyl)-ethyl)-N-methylamino]-propyloxy)-5-phenyl-1,2,4-thiadiazol.

300 mg 3-{3-[N-(2-(4-Aminophenyl)-ethyl)-N-methylamino]-propyloxy}-5-phenyl-1,2,4-thiadiazol (Herstellung siehe Beispiel 9) wurden in 4 ml Pyridin gelöst. Zu der Lösung wurde 1 ml Essigsäureanhydrid gegeben und das Reaktionsgemisch wurde 2 Stunden bei Raumtemperatur gerührt. Zur Aufarbeitung wurde auf Eis gegossen und das Gemisch durch Zugabe von Natriumcarbonat-Lösung alkalisch gestellt. Anschließend wurde mit Essigsäureethylester extrahiert, die organische Phase abgetrennt und zur Trockene eingeengt. Die erhaltene rohe ölige Titelverbindung wurde durch Mitteldruckchromatographie über feinkörnigem Kieselgel (Lichroprep Si 60^{R}) unter Verwendung von Essigsäureethylester/Methanol 95:5 als Elutionsmittel gereinigt. Es wurden 0,12 g der Titelverbindung als Öl erhalten.

Zur Salzbildung wurde die ölige Titelverbindung in Isopropanol gelöst und die Lösung wurde durch Zugabe von etherischer Salzsäurelösung auf pH 1 eingestellt. Die ausgefallenen Kristalle wurden anschließend abgesaugt und getrocknet. Es wurden 150 mg 3-{3-[N-(2-(4-Acetaminophenyl)-ethyl)-N-methylamino]-propyloxy}-5-phenyl-1,2,4-thiadiazol-hydrochlorid mit einem Schmelzpunkt von 211 bis 213 °C erhalten.

### Beispiel 11:

### 3-{3-[N-(2-(3,4-Dimethoxyphenyl)-ethyl)-N-methylamino]-propyloxy}-4-methyl-5-phenylisoxazol.

A) Zu einer Lösung von 5,2 g Natriumhydroxid in einer Mischung aus 5 ml Wasser und 100 ml Methanol wurden bei 70 °C tropfenweise unter Rühren 28,2 g 2-Benzoylpropionsäureethylester zugefügt. Zu dieser Lösung wurde dann eine auf -70 °C abgekühlte filtrierte Lösung von 10,3 g Natriumhydroxid und 17,1 Hydroxylamin-Hydrochlorid in einer Mischung aus 10 ml Wasser und 100 ml Methanol tropfenweise zugegeben. Dann wurde die Kühlung entfernt und weitere 2 Stunden gerührt, wobei die Temperatur auf ca. 10 °C anstieg. Anschließend wurden zu dem Reaktionsgemisch 9 ml Aceton gegeben und das Reaktionsgemisch wurde bei einer Temperatur von 80 °C in 90 ml konzentrierte Salzsäurelösung gegeben. Das Gemisch wurde noch weitere 30 Minuten bei 80 °C gerührt und dann auf die Hälfte eingeengt und noch mehrere Stunden bei 5 °C gerührt. Die ausgefallenen Kristalle wurden abfiltriert, in Dichlormethan aufgenommen und mit verdünnter wäßriger Salzsäurelösung versetzt. Die organische Phase wurde abgetrennt, mit Magnesiumsulfat getrocknet und eingeengt. Es wurden 18 g kristallines 4-Methyl-5-phenylisoxazol-3-ol erhalten.
B) 5,2 g des vorstehend erhaltenen Produktes wurden in 100 ml Dimethylformamid gelöst. Der Lösung wurden 4,15 g feingepulvertes wasserfreies Kaliumcarbonat zugesetzt, und das Reaktionsgemisch wurde anschließend 1 Stunde bei 100 °C unter Stickstoffatmosphäre erhitzt. Dann wurde bei 60 °C eine Lösung von 8,4 g 3-[N-(2-(3,4-Dimethoxyphenyl)-ethyl)-N-methylamino]-propylchlorid (Herstellung siehe Beispiel 5 D) in ca. 50 ml Dimethylformamid zugetropft. Das Reaktionsgemisch wurde 5 Stunden bei 100 °C gerührt. Dann wurden die gebildeten Salze abfiltriert und das Filtrat wurde bis zur Trockene eingeengt und der Rückstand in Methyl-tert.-butylether gelöst. Die Lösung wurde anschließend mit wäßriger Natriumcarbonat-Lösung gewaschen, mit Magnesiumsulfat getrocknet und zur Trockene eingeengt. Die als Rückstand verbleibende rohe ölige Titelverbindung wurde durch Flash-Chromatographie unter Verwendung einer Mischung aus Diethylether und Hexan im Verhältnis 1:1, der 5 % Triethylamin und 1 bis 5 % Methanol zugesetzt wurden, als Elutionsmittel gereinigt. Es wurden 8,8 g der Titelverbindung als Öl erhalten.
   Zur Salzbildung wurde die vorstehend erhaltene ölige Titelverbindung in Ethanol gelöst und zu dieser Lösung wurde tropfenweise eine ethanolische Oxalsäure-Lösung zugegeben. Das gebildete Kristallisat wurde abgetrennt. Es wurden 9,9 g 3-{3-[N-(2-(3,4-Dimethoxyphenyl)-ethyl)-N-methylamino]-propyloxy}-4-methyl-5-phenyl-isoxazol-hydrogenoxalat mit einem Schmelzpunkt von 183 °C erhalten.

### Beispiel 12:

### 3-{3-[N-(2-(3,4-Dimethoxyphenyl)-ethyl)-N-methylamino]-propyloxy}-5-(3-methoxyphenyl)-1,2,4-thiadiazol.

A) 17 g 3-Methoxybenzoylchlorid wurden in 200 ml Aceton gelöst. Zu der Lösung wurden 15,5 g Ammoniumacetat zugegeben und das Reaktionsgemisch wurde mehrere Stunden bei Raumtemperatur gerührt. Anschließend wurde in Dichlormethan gelöst. Die Dichlormethanphase wurde durch Zugabe von Natriumcarbonat getrocknet und eingeengt. Es wurden 7,5 g 3-Methoxybenzamid als farbloser Feststoff erhalten.
B) 15 g 3-Methoxybenzamid (Herstellung analog Beispiel 12A) wurden in 80 ml Toluol gelöst. Zu der Lösung wurden 20,4 g Lawessons Reagenz (= 2,4-bis(Methoxyphenyl)-1,3-dithia-2,4-diphosphetan-2,4-disulfid) zugegeben. Das Reaktionsgemisch wurde dann unter Stickstoffatmosphäre 3 Stunden auf 100 °C erhitzt. Anschließend wurde das Reaktionsgemisch zur Aufarbeitung eingeengt und der ölige Rückstand wurde durch Flash-Chromatographie unter Verwendung von Dichlormethan als Elutionsmittel gereinigt. Es wurden 2,7 g 3-Methoxyphenyl-thiobenzamid als gelber Feststoff erhalten.
C) 7,3 g 3-Methoxyphenylthiobenzamid (Herstellung analog Beispiel 12B) wurden in 50 ml Aceton gelöst und unter Stickstoffatmosphäre auf -20 °C abgekühlt. Zu dieser Lösung wurde eine Lösung von 4 ml Oxalylchlorid in 40 ml Aceton langsam zugetropft. Dann wurde noch 1 Stunde bei 20 °C gerührt. Die ausgefallenen Kristalle wurden anschließend abgesaugt und die Mutterlauge auf ungefähr ein Drittel des ursprünglichen Volumens eingeengt. Es wurde eine weitere Kristallfraktion erhalten. Insgesamt wurden 5 g 2-(3-Methoxyphenyl)-1,3-thiazol-4,5-dion als gelber Feststoff erhalten.
D) 5 g des vorstehend erhaltenen Produktes wurden in 60 ml Xylol gelöst. Anschließend wurden zu der Lösung langsam 5,3 g Azidotrimethylsilan zugetropft, und dann wurde das Reaktionsgemisch 4 Stunden auf 120 °C erhitzt. Anschließend wurde das Reaktionsgemisch durch Stehen bei Raumtemperatur abgekühlt, wobei Kristallisation eintrat. Die ausgefallenen Kristalle wurden abgesaugt und getrocknet. Die Mutterlauge wurde auf ein Drittel des ursprünglichen Volumens eingeengt, wobei eine weitere Kristallfraktion erhalten wurde. Insgesamt wurden 2,7 g 5-(3-Methoxyphenyl)-1,2,4-thiadiazol-3-ol als gelber Feststoff erhalten.
E) 2,7 g des vorstehend erhaltenen Produktes wurden mit 1,3 ml 1-Brom-3-chlorpropan analog Beispiel 1 B) umgesetzt. Das erhaltene Öl wurde durch Flash-Chromatographie unter Verwendung von Petrolether/Methyl-tert.-butylether 4:1 als Elutionsmittel gereinigt. Es wurden 2 g rohes 3-[5-(3-Methoxyphenyl)-1,2,4-thiadiazol-3-oxy]-propylchlorid als Öl erhalten.
F) 2,7 g des vorstehend erhaltenen Produktes wurden mit 1,9 g N-Methylhomoveratrylamin nach der in Beispiel 1 C) beschriebenen Methode umgesetzt. Die erhaltene rohe ölige Titelverbindung wurde durch Flash-Chromatographie unter Verwendung von Essigsäureethylester/Methanol 9:1 als Elutionsmittel gereinigt. Es wurden 2,9 g der Titelverbindung als Öl erhalten.
   Zur Salzbildung wurde die ölige Titelverbindung in Isopropanol gelöst und eine ausreichende Menge gasförmiger Chlorwasserstoff in die Lösung eingeleitet. Die ausgefallenen Kristalle wurden abgesaugt und getrocknet. Es wurden 2,3 g 3-{3-[N-(2-(3,4-Dimethoxyphenyl)-ethyl)-N-methylamino]-propyloxy}-5-(3-methoxyphenyl)-1,2,4-thiadiazol-hydrochlorid als farbloser Feststoff mit einem Schmelzpunkt von 175 °C erhalten.

### Beispiel 13:

### 3-{3-[N-(2-(3,4-Dimethoxyphenyl)-ethyl)-N-methylamino]-propyloxy}-5-(3-hydroxyphenyl)-1,2,4-thiadiazol.

A) 2 g 3-[5-(3-Methoxyphenyl)-1,2,4-thiadiazol-3-oxy]-propylchlorid (Herstellung siehe Beispiel 12 E) wurden in 100 ml Dichlormethan gelöst. Zu der Lösung wurde eine Lösung von 20 ml Bortribromid in Dichlormethan gegeben und das Reaktionsgemisch wurde bei Raumtemperatur unter Stickstoffatmosphäre 8 Stunden gerührt. Anschließend wurde das Reaktionsgemisch zur Aufarbeitung in Wasser gegossen und durch Zugabe von Natriumcarbonat alkalisch eingestellt. Dann wurde mit Dichlormethan extrahiert, die organische Phase getrocknet und eingeengt. Es wurden 1,9 g rohes 3-[5-(3-Hydroxyphenyl)-1,2,4-thiadiazol-3-yloxy]-propylchlorid als gelbes Öl erhalten.
B) 1,9 g des vorstehend erhaltenen Produktes wurden mit 1,9 g N-Methylhomoveratrylamin nach der in Beispiel 1 C) beschriebenen Methode umgesetzt. Die erhaltene ölige Titelverbindung wurde durch Flash-Chromatographie an Kieselgel unter Verwendung von Dichlormethan/Methanol 9:1 als Elutionsmittel gereinigt. Es wurden 2,9 g der Titelverbindung als Öl erhalten.
   Zur Salzbildung wurde die erhaltene ölige Titelverbindung in Ethanol gelöst und eine ausreichende Menge gasförmiger Chlorwasserstoff in die Lösung eingeleitet. Die ausgefallenen Kristalle wurden abgesaugt und getrocknet. Es wurden 2,3 g 3-{3-[N-(2-(3,4-Dimethoxyphenyl)-ethyl)-N-methylamino]-propyloxy}-5-(3-hydroxyphenyl)-1,2,4-thiadiazol-hydrochlorid als farbloser Feststoff mit einem Schmelzpunkt von 152 °C erhalten.

### Beispiel 14:

### 3-{3-[N-(2-(3,4-Dimethoxyphenyl)-ethyl)-N-methylamino]-propyloxy}-5-(4-nitrophenyl)-1,2,4-oxadiazol.

A) Zu einer Lösung von 50 g 4-Nitrobenzamid in 1,2 1 1,2-Dichlorethan wurden 48 ml Oxalylchorid portionsweise zugegeben und das Reaktionsgemisch wurde 17 Stunden unter Rückfluß gekocht. Zur Aufarbeitung wurde das Reaktionsgemisch eingeengt und der verbleibende Rückstand im Hochvakuum destilliert. Es wurden 56 g 4-Nitrobenzoylisocyanat als farblose Kristalle erhalten.
B) 56 g des vorstehend erhaltenen Produktes wurden mit 67 g Azidotrimethylsilan versetzt und das Reaktionsgemisch wurde 7 Stunden unter Rückfluß erhitzt. Anschließend wurde das Reaktionsgemisch nach der in Beispiel 2 A) beschriebenen Methode aufgearbeitet. Es wurden 49,8 g rohes 5-(4-Nitrophenyl)-1,2,4-oxadiazol-3-ol erhalten.
C) 15 g des vorstehend erhaltenen Produktes wurden in 300 ml Dimethylformamid gelöst und mit 9,7 g Kalium-tert.-butylat und 7,8 ml 1-Brom-3-chlorpropan analog der in Beispiel 2 B) beschriebenen Methode umgesetzt. Das erhaltene kristalline Rohprodukt wurde mit Methanol ausgerührt, das gebildete Kristallisat abgesaugt und getrocknet. Es wurden 10,2 g 3-[5-(4-Nitrophenyl)-1,2,4-oxadiazol-3-yloxy]-propylchlorid als farbloses Kristallisat erhalten.
D) 10,2 g des vorstehend erhaltenen Produktes wurden mit 7 g N-Methylhomoveratrylamin und 5 ml Triethylamin versetzt und das Reaktionsgemisch wurde im Ölbad 20 Minuten auf eine Ölbadtemperatur von 140 °C erhitzt. Zur Aufarbeitung wurde das Reaktionsgemisch abgekühlt, auf Eiswasser gegossen und mit Essigsäureethylester ausgeschüttelt. Die vereinigten organischen Phasen wurden eingeengt und die als Rückstand verbleibende rohe Titelverbindung wurde durch Mitteldruckchromatographie an feinkörnigem Kieselgel unter Verwendung von Essigsäureethylester/Methanol 9:1 als Elutionsmittel gereinigt. Es wurden 6,8 g der Titelverbindung als leicht gelbes Öl erhalten. Dünnschichtchromatographie an Kieselgel: Rf-Wert = 0,5 (Eluens Methanol/Essigsäureethylester 1:9)

### Beispiel 15:

### 3-{3-[N-(2-(3,4-Dimethoxyphenyl)-ethyl)-N-methylamino]-propyloxy}-5-(4-aminophenyl)-1,2,4-oxadiazol.

5 g 3-{3-[N-(2-(3,4-Dimethoxyphenyl)-ethyl)-N-methylamino]-propyloxy}-5-(4-nitrophenyl)-1,2,4-oxadiazol (Herstellung siehe Beispiel 14) wurden in 500 ml Ethanol gelöst. Zu der Lösung wurden 4 ml Hydraziniumhydroxid und eine katalytische Menge Raney-Nickel zugegeben und das Reaktionsgemisch wurde 30 Minuten auf 50 °C erhitzt. Der Katalysator wurde anschließend abfiltriert und das Filtrat wurde zur Trockene eingeengt. Der verbleibende Rückstand wurde mit 10 %-iger wäßriger Natriumcarbonatlösung versetzt und mit Essigsäureethylester ausgeschüttelt. Die organische Phase wurde mit Natriumsulfat getrocknet, filtriert und eingeengt. Die als Rückstand verbleibende rohe ölige Titelverbindung wurde durch Mitteldruckchromatographie über Kieselgel (Lichroprep Si 60^{R}) unter Verwendung von Essigsäureethylester/Methanol 99:1 als Elutionsmittel gereinigt. Es wurden 1,1 g der Titelverbindung als Öl erhalten.

Zur Salzbildung wurden 270 mg der öligen Titelverbindung und 246 mg N-Acetyl-L-glutaminsäure in wenig Methanol gelöst. Die Lösung wurde filtriert und anschließend zur Trockene eingeengt. Als Rückstand wurden 240 mg 3-{3-[N-(2-(3,4-Dimethoxyphenyl)-ethyl)-N-methylamino]-propyloxy}-5-(4-aminophenyl)-1,2,4-oxadiazol-N-Acetyl-L-glutamat mit einem Schmelzpunkt von 107 bis 110 °C erhalten.

### Beispiel 16:

### 3-{3-[2-(3,4-Dimethoxyphenyl)-ethylamino]-propyloxy}-5-phenyl-1,2,4-oxadiazol.

A) 17,5 g Benzoylisocyanat wurden mit 27 ml Azidotrimethylsilan nach der in Beispiel 2 A) angegebenen Methode umgesetzt. Es wurden 21 g 5-Phenyl-1,2,4-oxadiazol-3-ol als farbloser Feststoff erhalten.
B) 10,7 g des vorstehend erhaltenen Produktes wurden in 300 ml Dimethylformamid mit 2,4 g Natriumhydrid und 10,9 g 3-Brom-1-chlorpropan nach der in Beispiel 2 B) beschriebenen Methode umgesetzt. Zur Aufarbeitung wurde das Reaktionsgemisch anschließend zur Trockene eingeengt, der verbleibende Rückstand in Methyl-tert.-butylether aufgenommen und mit Wasser ausgeschüttelt. Die vereinigten organischen Phasen wurden mit Natriumsulfat getrocknet, filtriert und eingeengt. Der verbleibende Rückstand wurde in Diethylether gelöst und durch Mitteldruckchromatographie an feinkörnigem Kieselgel unter Verwendung von Cyclohexan/Essigsäureethylester 9:1 als Elutionsmittel gereinigt. Es wurden 6,7 g 3-(5-Phenyl-1,2,4-thiadiazol-3-yloxy)-propylchlorid als farbloses Kristallisat erhalten.
C) 6,7 g des vorstehend erhaltenen Produktes wurden mit 4,75 g 2-(3,4-Dimethoxyphenyl)-ethylamin nach der in Beispiel 2 C) beschriebenen Methode umgesetzt. Zur Aufarbeitung wurde das Reaktionsgemisch anschließend zur Trockene eingeengt und der Rückstand in Essigsäureethylester gelöst. Zu dieser Lösung wurde soviel Wasser zugefügt, daß eine deutliche Phasentrennung auftrat. Das Gemisch wurde ausgeschüttelt und die Essigsäureethylesterphase wurde verworfen. Die verbleibende wäßrige Phase wurde mit Zitronensäure angesäuert und mit Essigsäurethylester extrahiert. Diese Essigsäureethylesterphase wurde ebenfalls verworfen und die verbleibende wäßrige Phase wurde durch Zugabe von Natriumcarbonat alkalisch gestellt und anschließend zweimal mit Dichlormethan extrahiert. Die vereinigten Dichlormethanextrakte wurden mit Natriumsulfat getrocknet, filtriert und eingeengt. Die erhaltene ölige rohe Titelverbindung wurde durch Mitteldruckchromatographie an feinkörmigem Kieselgel unter Verwendung von Essigsäure-ethylester/Methanol/konzentrierte wäßrige Ammoniaklösung 9:1:0,1 als Elutionsmittel gereinigt. Es wurden 3,3 g der Titelverbindung als Öl erhalten.
   Zur Salzbildung wurden 1,1 g der obigen Titelverbindung in Isopropanol gelöst und bis zur Trübung mit etherischer Salzsäurelösung versetzt. Das ausgefallene Kristallisat wurde abgesaugt, zunächst mit Isopropanol/Diethylether 1:1 und dann mit Isopropanol nachgewaschen und getrocknet. Es wurden 1,2 g 3-{3-[2-(3,4-Dimethoxyphenyl)-ethylamino]-propyloxy}-5-phenyl-1,2,4-oxadiazol-Hydrochlorid als farbloses Kristallisat mit einem Schmelzpunkt von 158 - 160 °C erhalten.

### Beispiel 17:

### 3-{3-[N-(2-(3,4-Dimethoxyphenyl)-ethyl)-N-ethylamino]-propyloxy}-5-phenyl-1,2,4-oxadiazol.

2 g 3-{3-[2-(3,4-Dimethoxyphenyl)-ethylamino]-propylaxy}-5-phenyl-1,2,4-oxadiazol (Herstellung siehe Beispiel 16) wurden in 20 ml Dimethylformamid gelöst. Zu der Lösung wurden 0,9 ml Diethylamin und 0,6 g Ethylbromid gegeben und das Reaktionsgemisch wurde 3,5 Stunden auf 50 °C erhitzt. Zur Aufarbeitung wurde das Reaktionsgemisch anschließend zur Trockene eingeengt und der Rückstand in Dichlormethan gelöst. Zu der Lösung wurde soviel Wasser zugefügt, daß eine deutliche Phasentrennung auftrat. Das Gemisch wurde ausgeschüttelt und die organische Phase abgetrennt, mit Natriumsulfat getrocknet, filtriert und eingeengt. Die erhaltene rohe, ölige Titelverbindung wurde durch Mitteldruckchromatographie an feinkörnigem Kieselgel unter Verwendung von Essigsäureethylester/Methanol/konzentrierte wäßrige Ammoniaklösung 9:1:0,05 als Elutionsmittel gereinigt. Es wurden 1,3 g der Titelverbindung als gelbes Öl erhalten.

Zur Salzbildung wurde die ölige Titelverbindung in Isopropanol gelöst und die Lösung wurde mit einer ätherischen Salzsäurelösung versetzt. Das ausgefallene Kristallisat wurde abgesaugt, mit Diethylether gewaschen und getrocknet. Es wurden 0,65 g 3-{3-[N-(2-(3,4-Dimethoxyphenyl)-ethyl)-N-ethylamino]-propyloxy}-5-phenyl-2,2,4-oxadiazol-Hydrochlorid als farbloses Kristallisat mit einem Schmelzpunkt von 137 bis 139 °C erhalten.

### Beispiel 18:

### 3-{4-[N-(2-(3,4-Dimethoxyphenyl)-ethyl)-N-methylamino]-butyloxy)-5-phenyl-1,2,4-oxadiazol.

A) 10 g 5-Phenyl-1,2,4-oxadiazol-3-ol (Herstellung siehe Beispiel 2 A)) wurden in 150 ml Dimethylformamid gelöst. Zu der Lösung wurden portionsweise 3 g Natriumhydrid zugegeben. Das Reaktionsgemisch wurde 90 Minuten auf 80 °C erhitzt. Anschließend wurden 17,22 ml 4-Chlorbuttersäureethylester zugetropft und das Reaktionsgemisch wurde dann zunächst 30 Minuten auf 90 °C und dann weitere 45 Minuten auf 100 °C erhitzt. Zur Aufarbeitung wurde anschließend das Dimethylformamid abdestilliert, der Rückstand auf Eiswassser gegossen und mit Essigsäureethylester extrahiert. Die organische Phase wurde abgetrennt, getrocknet und eingeengt. Das erhaltene Öl wurde durch Chromatographie über feinkörniges Kieselgel bei leicht erhöhtem Druck (Flash-Chromatographie) unter Verwendung von Cyclohexan/Essigsäureethylester 4:1 als Elutionsmittel gereinigt. Es wurden 11,29 g 4-(5-Phenyl-1,2,4-oxadiazol-3-yloxy)-buttersäureethylester als Öl erhalten.
B) 11,29 g des vorstehend erhaltenen Produktes wurden mit 14,32 g Natriumhydroxid und 20,4 ml Ethanol versetzt. Das Reaktionsgemisch wurde 30 Minuten auf 90 °C erhitzt. Zur Aufarbeitung wurde das Reaktionsgemisch eingeengt und der Rückstand in wenig Wasser gelöst. Anschließend wurde die Lösung durch Zugabe von verdünnter wäßriger Salzsäurelösung auf pH 1 eingestellt. Die ausgefallenen Kristalle wurden abgesaugt, mit wenig Wasser gewaschen und getrocknet. Es wurden 4,04 g 4-(5-Phenyl-1,2,4-oxadiazol-3-yloxy)-buttersäure als farbloser Feststoff erhalten.
C) Zu einer Lösung von 3,42 g 2-Chlor-N-methylpyridiniumjodid in 123 ml Dichlormethan wurden zunächst 1,85 ml Triethylamin und anschließend 2,77 g des vorstehend unter Beispiel 18 B) hergestellten Produktes zugegeben. Das Reaktionsgemisch wurde 15 Minuten bei Raumtemperatur gerührt. Dann wurden 2,19 g N-Methylhomoveratrylamin zugegeben und die Reaktionslösung wurde erneut 30 Minuten bei Raumtemperatur gerührt. Zur Aufarbeitung wurde das Reaktionsgemisch zur Trockene eingegengt, der Rückstand in Wasser aufgenommen und mit Essigsäureethylester ausgeschüttelt. Die organische Phase wurde abgetrennt, mit Natriumsulfat getrocknet und eingeengt. Das erhaltene Öl wurde durch Chromatographie über feinkörnigem Kieselgel bei leicht erhöhtem Druck (Flash-Chromatographie) unter Verwendung von Essigsäureethylester als Elutionsmittel gereinigt. Es wurden 3,96 g 4-(5-Phenyl-1,2,4-oxadiazol-3-yloxy)-buttersäure-N-[2-(3,4-dimethoxyphenyl)-ethyl]-N-methylamid als farbloser Feststoff erhalten.
D) 3,96 g des vorstehend erhaltenen Produktes wurden in 150 ml Tetrahydrofuran gelöst. Zu der Lösung wurden 5 ml Diisobutylaluminiumhydrid zugegeben. Das Reaktionsgemisch wurde 30 Minuten bei Raumtemperatur gerührt. Anschließend wurden erneut zweimal je 5 ml Diisobutylaluminiumhydrid tropfenweise zugegeben. Dann wurde das Reaktionsgemisch langsam auf 50 °C erwärmt und 1 Stunde bei dieser Temperatur gerührt. Anschließend wurden 100 ml konzentrierte wäßrige Zitronensäurelösung zugegeben und weitere 30 Minuten bei 50 °C gerührt. Dann wurde das Tetrahydrofuran abdestilliert und die verbleibende wäßrige Lösung wurde mit Essigsäureethylester ausgeschüttelt. Die organische Phase wurde verworfen und die wäßrige Phase wurde anschließend durch Zugabe von wäßriger Natronlauge neutralisiert und mit Essigsäureethylester ausgeschüttelt. Diese Essigsäureethylesterphase wurde getrocknet und eingeengt. Die als Rückstand verbleibende rohe Titelverbindung wurde durch Chromatographie über feinkrönigem Kieselgel bei leicht erhöhtem Druck (Flash-Chromatographie) unter Verwendung von Essigsäureethylester/Methanol 1:1 als Elutionsmittel gereinigt. Es wurden 1,9 g der Titelverbindung erhalten.

Zur Salzbildung wurden 1,9 g der Titelverbindung in Ethanol gelöst und die Lösung wurde mit ätherischer Salzsäurelösung versetzt. Anschließend wurde zur Trockene eingeengt und der Rückstand in Isopropanol aufgenommen. Die ausgefallenen Kristalle wurden abgesaugt und getrocknet. Es wurden 0,8 g 3-{4-[N-(2-(3,4-Dimethoxyphenyl)-ethyl)-N-methylamino]-butyloxy}-5-phenyl-1,2,4-oxadiazol-hydrochlorid als farbloser Feststoff mit einem Schmelzpunkt von 148,5 bis 150,5 °C erhalten.

### Beispiel 19:

### 3-{3-[N-(2-(4-Hydroxy-3-methoxyphenyl)-ethyl)-N-methylamino]-propyloxy}-5-phenyl-1,2-4-thiadiazol.

A) 5 g 4-Hydroxy-3-methoxyphenylessigsäureethylester wurden in 25 ml Dichlormethan gelöst. Zu der Lösung wurden 15,3 g N-(Ethyl)-diisopropylamin und 12,65 ml 2-(Trimethylsilyl)-ethoxymethylchlorid gegeben. Das Reaktionsgemisch wurde 1,5 Stunden bei Raumtemperatur gerührt. Zur Aufarbeitung wurde das Reaktionsgemisch anschließend mit Wasser ausgeschüttelt. Die Dichlormethan-Phase wurde abgetrennt und zur Trockene eingeengt. Das erhaltene Öl wurde in Methyl-tert.-butylether gelöst und erneut mit Wasser ausgeschüttelt. Die organische Phase wurde mit Natriumsulfat getrocknet, filtriert und eingeengt. Es wurden 7,8 g 4-[2-(Trimethylsilyl)-ethoxymethoxy]-3-methoxyphenylessigsäureethylester als Öl erhalten.
B) 7,8 g des vorstehend erhaltenen Produktes wurden in 100 ml Ethanol gelöst. Zu der Lösung wurden 11,2 ml 40 %ige wäßrige Natronlauge gegeben und das Reaktionsgemisch wurde 1 Stunde bei 42 °C gerührt. Zur Aufarbeitung wurde das Reaktionsgemisch zur Trockene eingeengt und der Rückstand in Essigsäureethylester gelöst. Zu dieser Lösung wurde soviel Wasser zugefügt, daß eine deutliche Phasentrennung auftrat. Das Gemisch wurde ausgeschüttelt und die Essigsäureethylesterphase wurde verworfen. Die verbleibende wäßrige Phase wurde durch Zugabe von Zitronensäure auf pH 4 eingestellt und zweimal mit Dichlormethan extrahiert. Die vereinigten Dichlormethanextrakte wurden mit Natriumsulfat getrocknet, filtriert und eingeengt. Es wurden 6,7 g 4-[2-(Trimethylsilyl)-ethoxymethoxy]-3-methoxyphenylessigsäure erhalten.
C) 6,6 g des vorstehend erhaltenen Produktes wurden in 105 ml absolutem Tetrahydrofuran gelöst. Zu der Lösung wurden 3,8 g 1,1'-Carbonyldiimidazol zugegeben und das Reaktionsgemisch 1 Stunde bei Raumtemperatur gerührt. Anschließend wurden 5,7 g Methylamin-hydrochlorid und 11,7 ml Triethylamin zugegeben und das Reaktionsgemisch weitere 3,5 Stunden bei Raumtemperatur gerührt. Zur Aufarbeitung wurde das Reaktionsgemisch zur Trockene eingeengt und der Rückstand in Methyl-tert.-butylether gelöst. Die Lösung wurde zunächst mit Wasser und dann nacheinander mit wäßriger Natriumcarbonatlösung, verdünnter wäßriger Zitronensäurelösung und Wasser ausgeschüttelt. Dann wurde die organische Phase mit Natriumsulfat getrocknet, filtriert und eingeengt. Es wurden 5,9 g 4-[2-(Trimethylsilyl)-ethoxymethoxy]-3-methoxyphenylessigsäure-N-methylamid als farbloser Feststoff erhalten.
D) 5,8 g des vorstehend erhaltenen Produktes wurden in 200 ml getrocknetem Dioxan gelöst. Zu der Lösung wurden portionsweise 3,4 g Natriumborhydrid unter Stickstoffatmosphäre und Eiskühlung zugegeben. Zu der erhaltenen Suspension wurden vorsichtig 5,1 ml Eisessig zugetropft und das Reaktionsgemisch wurde 2,5 Stunden unter Rückfluß gerührt. Dann wurde das Reaktionsgemisch zur Trockene eingeengt, der verbleibende Rückstand in 50 ml Methanol aufgenommen und 1 ml 10%-ige wäßrige Salzsäurelösung zugefügt. Das Gemisch wurde 15 Minuten bei Raumtemperatur gerührt und dann zur Trockene eingeengt. Der Rückstand wurde in verdünnter wäßriger Zitronensäurelösung gelöst und mit Essigsäureethylester extrahiert. Die Essigsäureethylesterphase wurde mit Natriumsulfat getrocknet, filtriert und eingeengt. Das verbleibende Öl wurde in 50 ml Methanol gelöst. Zu der Lösung wurden 2 ml 10 %-ige wäßrige Salzsäurelösung zugefügt und das Reaktionsgemisch wurde 5 Stunden unter Rückfluß gekocht. Anschließend wurde eine Säure/Base-Trennung des Reaktionsgemisches vorgenommen, die wäßrige Phase abgetrennt und zur Trockene eingeengt. Der verbleibende Rückstand wurde mit verdünnter wäßriger Zitronensäurelösung extrahiert. Dieser Extrakt wurde durch Zugabe von wäßriger Natronlauge alkalisch eingestellt und mit Dichlormethan extrahiert. Die Dichlormethan-Phase wurde mit Natriumsulfat getrocknet, filtriert und eingeengt. Es wurden 0,95 g N-(2-[4-(2-(Trimethylsilyl)-ethoxymethoxy)-3-methoxyphenyl]-ethyl}-N-methylamin als Öl erhalten.
E) 0,85 g des vorstehend erhaltenen Produktes wurden mit 0,7 g 3-(5-Phenyl-1,2,4-thiadiazol-3-yloxy)-propylchlorid (Herstellung siehe Beispiel 1 B)) versetzt und das Reaktionsgemisch wurde 10,5 Stunden bei 65 °C gerührt. Anschließend wurde das Reaktionsgemisch in Dichlormethan gelöst und die Lösung wurde mit Wasser ausgeschüttelt. Die Dichlormethanphase wurde abgetrennt, getrocknet und eingeengt. Das erhaltene Rohprodukt wurde durch Mitteldruckchromatographie an feinkörnigem Kieselgel (Lichroprep Si 60^{R}) unter Verwendung von Essigsäureethylester/Methanol 9:1 als Elutionsmittel gereinigt. Es wurden 0,5 g 3-{3-[N-[2-(4-(2-Trimethylsilyl)-ethoxymethoxy)-3-methoxyphenyl)-ethyl]-N-methylamino]-propyloxy}-5-phenyl-1,2,4-thiadiazol als farbloses Öl erhalten.
F) 0,5 g des vorstehend erhaltenen Produktes wurden mit 5 ml einer 1-molaren Lösung von Tetrabutylammoniumfluorid in Tetrahydrofuran versetzt. Das Reaktionsgemisch wurde zur Trockene eingeengt und der verbliebene Rückstand wurde mit 0,5 g gemörsertem Molekularsieb (Spezifikation: A4, Porenweite 4-8 mesh) und 5 ml 1,3-Dimethyltetrahydro-2-(1H)-pyrimidinon ( = DMPU ) versetzt. Das Gemisch wurde anschließend 1 Stunde bei Raumtemperatur gerührt. Zur Aufarbeitung wurde sodann mit Wasser verdünnt und das Gemisch zweimal mit Dichlormethan extrahiert. Die vereinigten Dichlormethan-Phasen wurden mit Natriumsulfat getrocknet, filtriert und eingeengt. Die erhaltene rohe Titelverbindung wurde durch Mitteldruckchromatographie an feinkörnigem Kieselgel (Lichroprep Si 60^{R}) unter Verwendung von Essigsäureethyl-ester/Methanol 9:1 als Elutionsmittel gereinigt. Nach Einengen der das Produkt enthaltenen Eluatfraktionen wurde die Titelverbindung in Form eines noch leicht verunreinigten gelben Rohöls erhalten. Dieses wurde anschließend am Kugelrohr bei 150 °C und 1 mbar Druck destilliert. Es wurden 320 mg der Titelverbindung als gelbes Öl erhalten.
   Zur Salzbildung wurde die ölige Titelverbindung in Essigsäureethylester gelöst und zuerst mit Wasser und dann mit Zitronensäure ausgeschüttelt. Die Zitronensäurephase wurde mit Natriumcarbonat-Lösung auf pH 9 eingestellt und zweimal mit Essigsäureethylester extrahiert. Das Extrakt wurde mit Natriumsulfat getrocknet und eingeengt. Der Rückstand wurde in Methanol gelöst und mit einer ausreichenden Menge etherischer Salzsäurelösung versetzt. Zur Kristallisation wurde mit Eiswasser gekühlt. Die ausgefallenen Kristalle wurden abgessaugt, mit Methanol/Diethylether 1:1 nachgewaschen und getrocknet. Es wurden 167 mg 3-(3-[N-2-(4-Hydroxy-3-methoxyphenyl)-ethyl)-N-methylamino]-propyloxy}-5-phenyl-1,2,4-thiadiazol als farbloses Kristallisat mit einem Schmelzpunkt von 137 - 138 °C erhalten.
   Nach den in den vorstehenden Beispielen beschriebenen Verfahren können auch die in der folgenden Tabelle aufgeführten Verbindungen der Formel I hergestellt werden. Die in der Tabelle in cm⁻¹ angeführten IR-Banden sind die charakteristischen Banden der IR-Spektren der jeweiligen freien Basen (sofern nicht anders angegeben als Film).

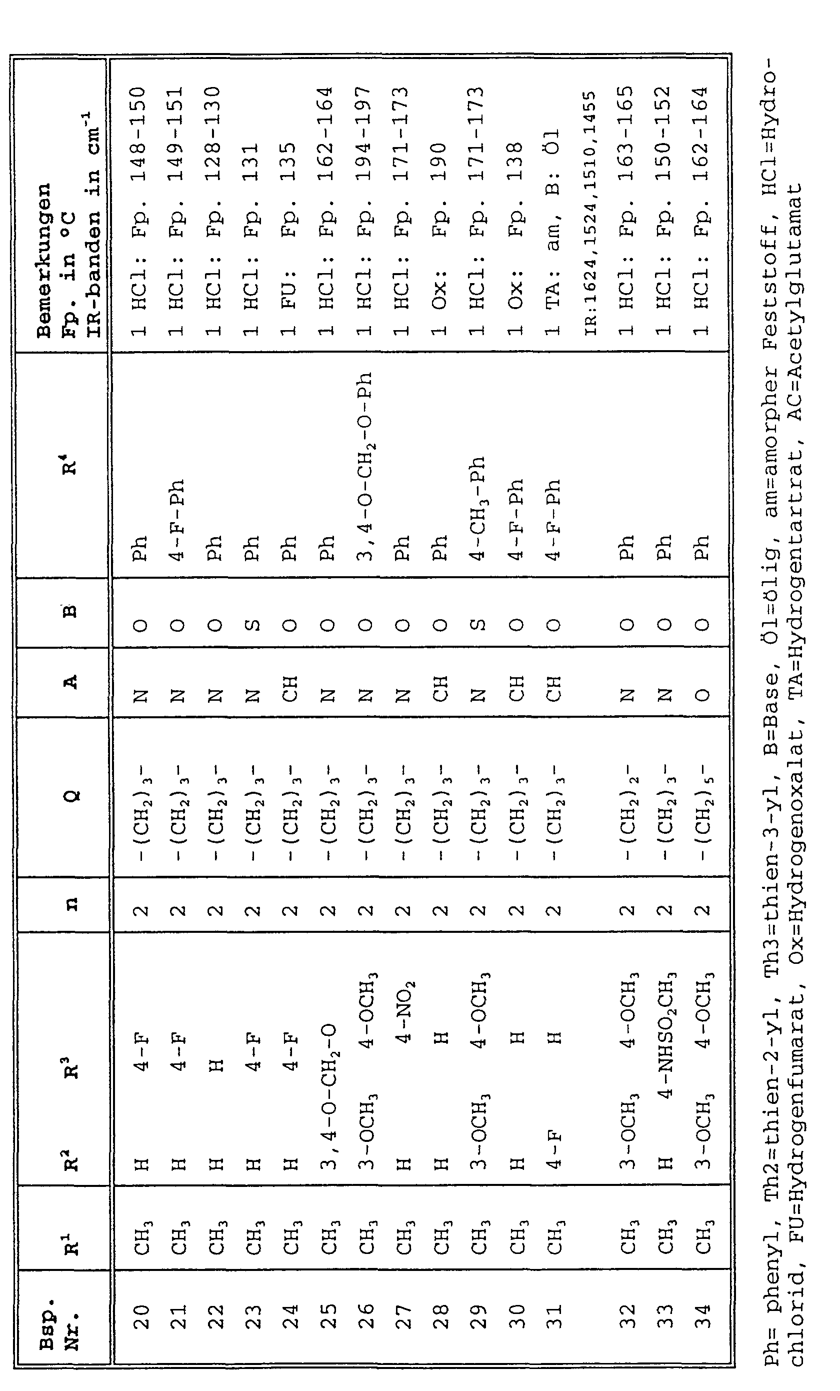

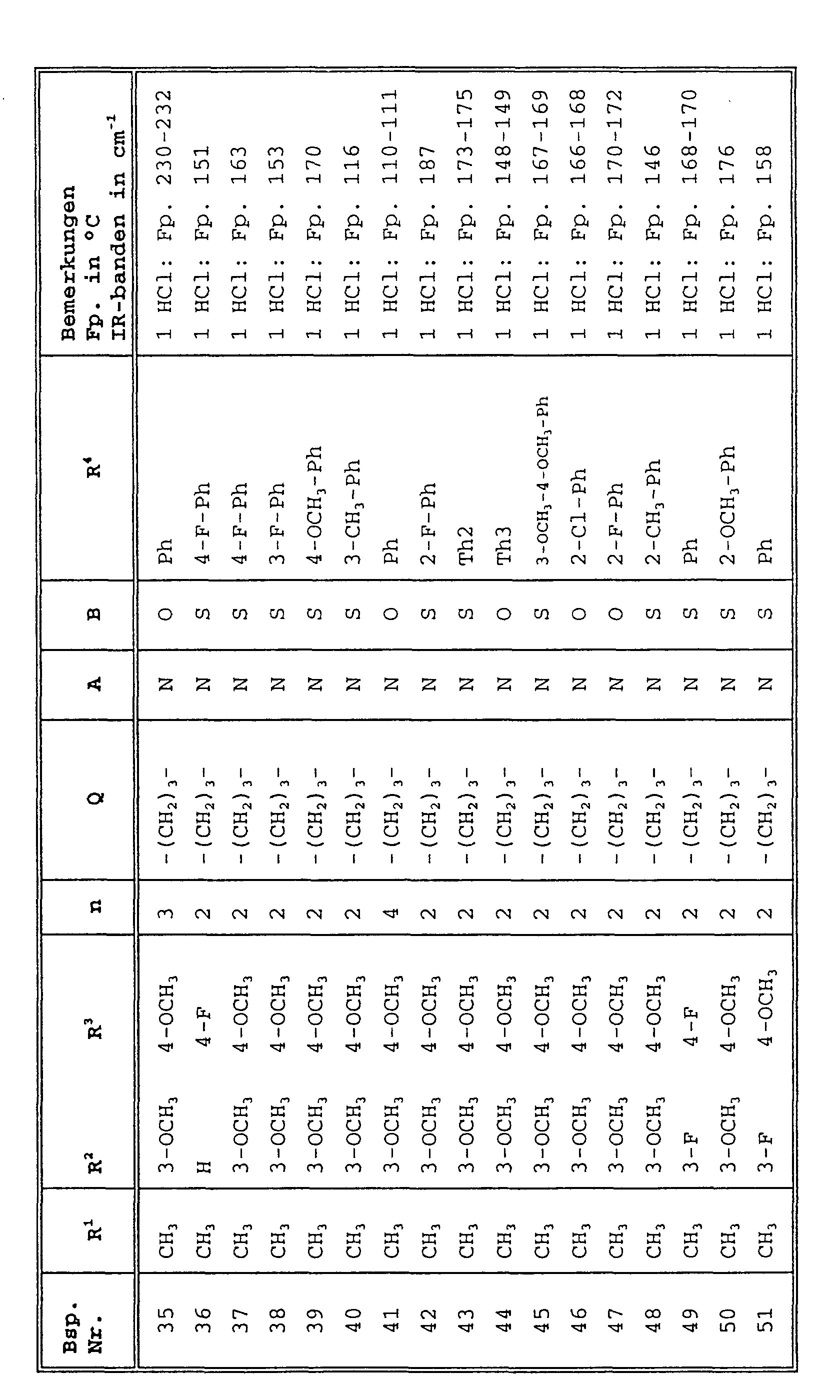

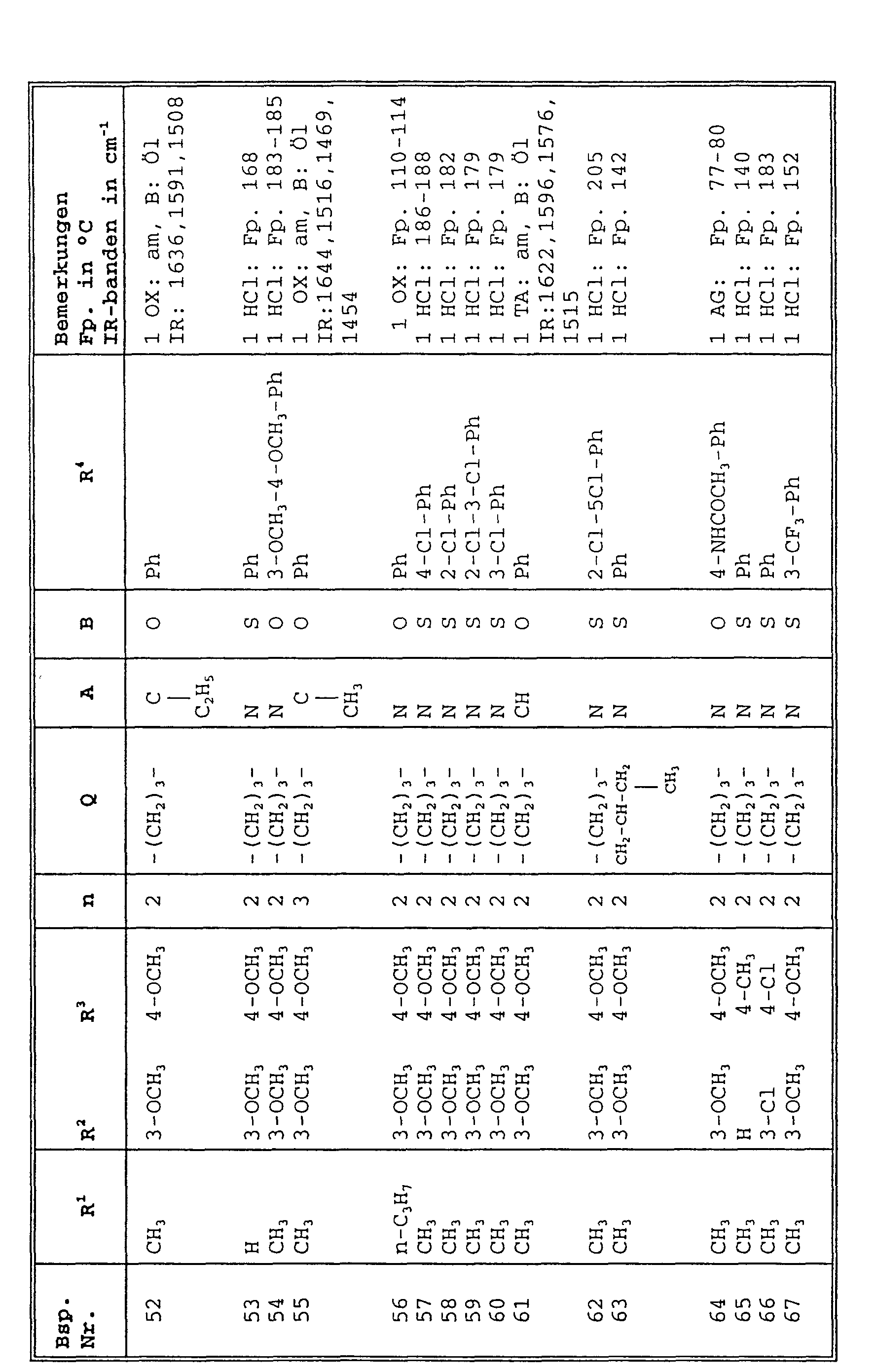

### Beispiel I:

### 3-{3-[N-(2-(3,4 Dimethoxyphenyl)-ethyl)-N-methylamino]-propyloxy}-5-phenyl-1,2,4-thiadiazol-hydrochlorid enthaltende Tabletten

Man stellte Tabletten in folgender Zusammensetzung pro Tablette her:

| | |
|---|---|
| 3-{3-[N-(2-(3,4 Dimethoxyphenyl)-ethyl)-N-methylamino]-propyloxy}-5-phenyl-1,2,4-thiadiazol-hydrochlorid | 20 mg |
| Maisstärke | 60 mg |
| Milchzucker | 35 mg |
| Gelatine (als 10%-ige Lösung) | 6 mg |

Der Wirkstoff, die Maisstärke und der Milchzucker wurden mit der 10 %-igen Gelatine-Lösung eingedickt. Die Paste wurde zerkleinert, und das entstehende Granulat wurde auf ein geeignetes Blech gebracht und bei 45 °C getrocknet. Das getrocknete Granulat wurde durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann zu Tabletten von 240 mg verpreßt.

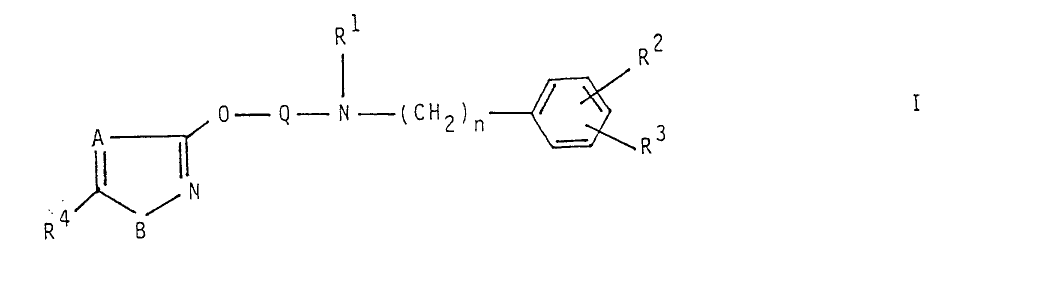

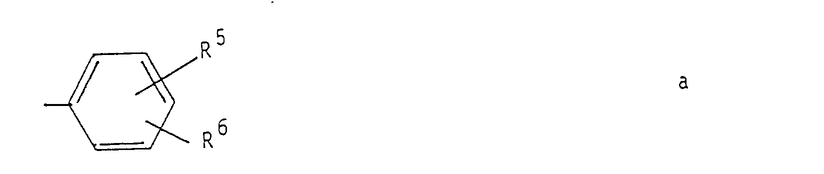

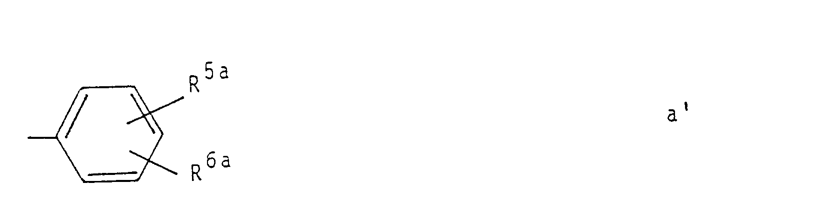

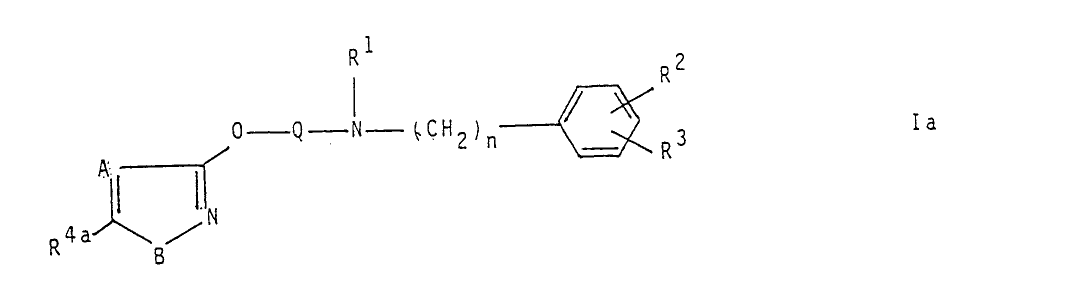

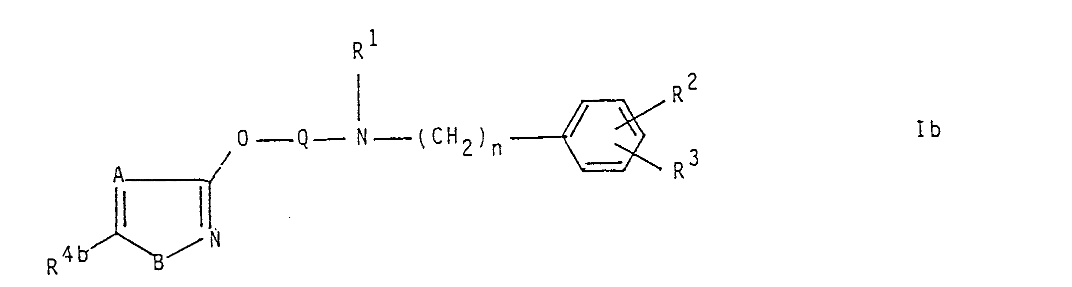

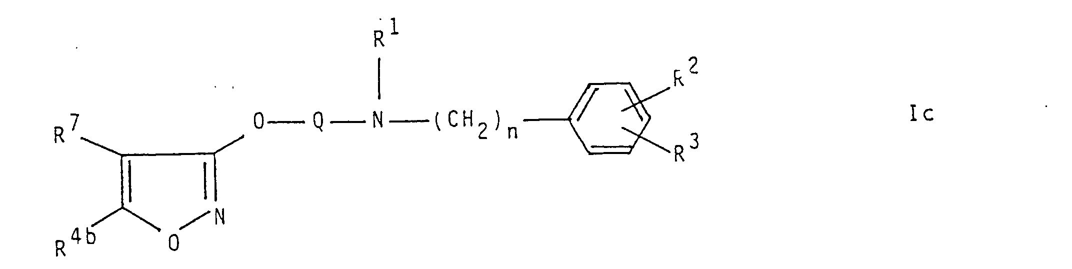

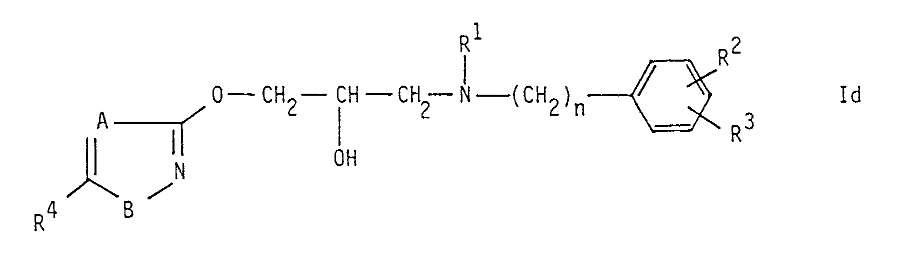

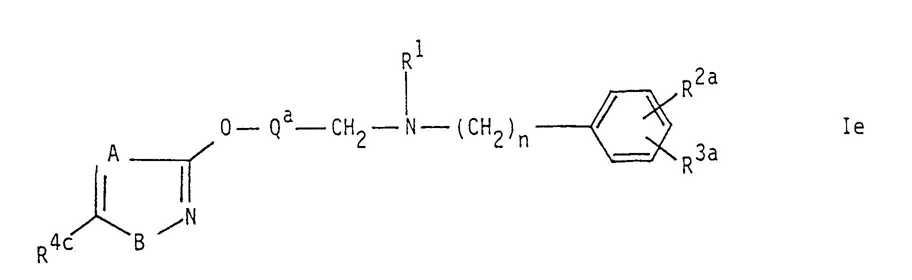

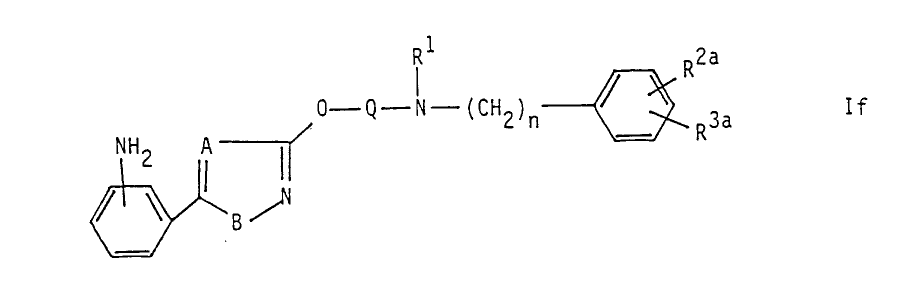

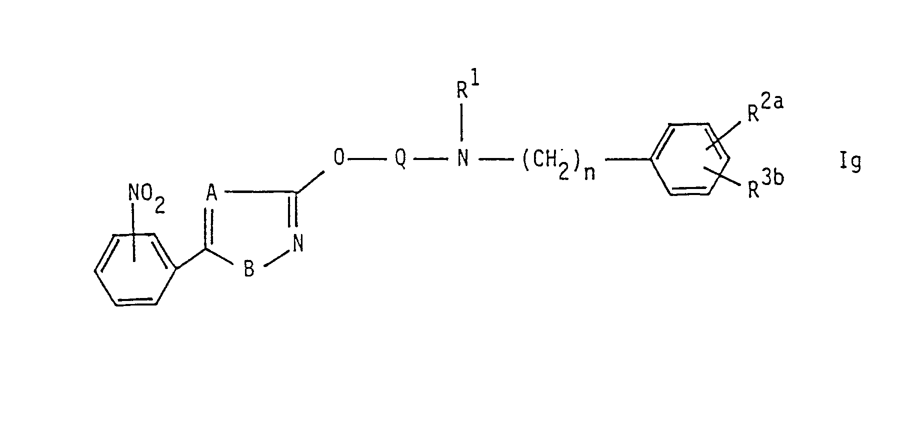

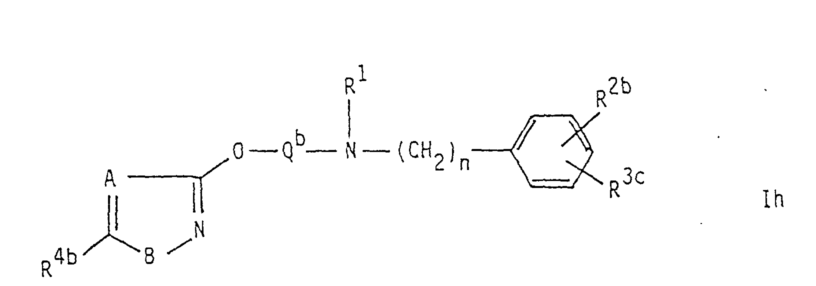

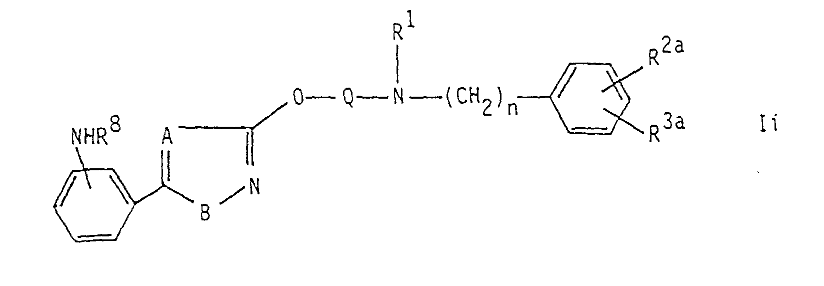

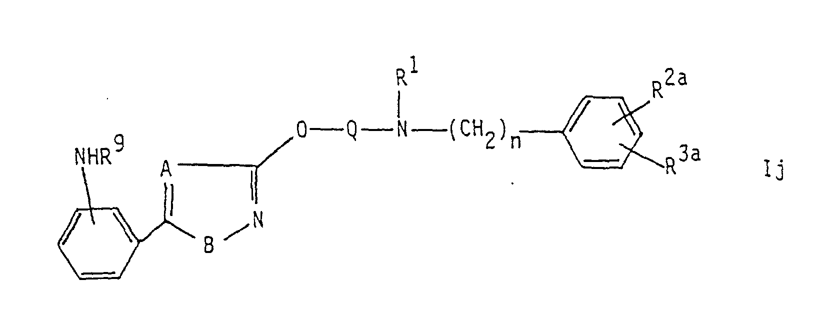

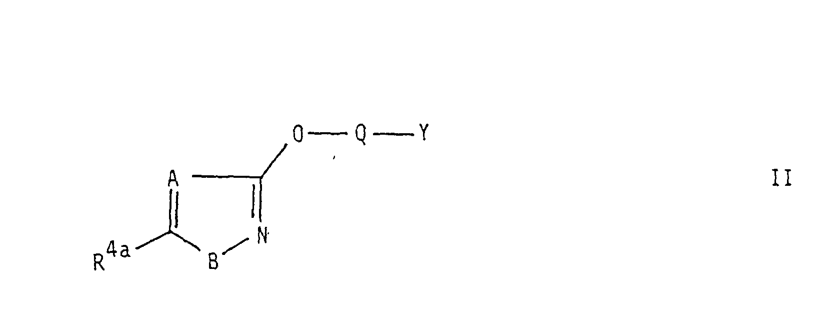

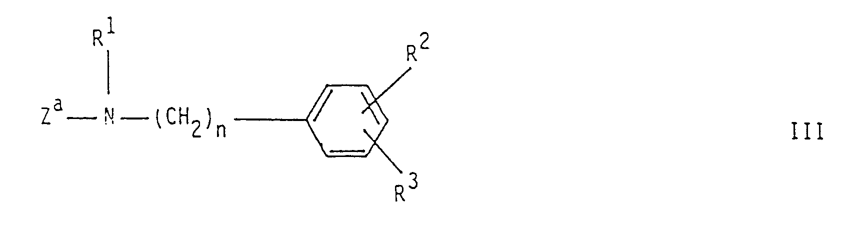

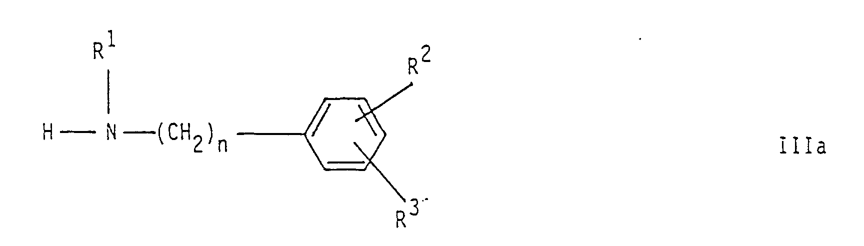

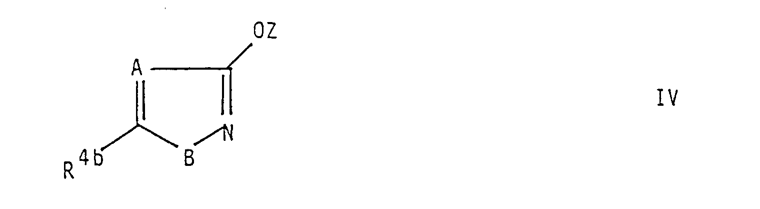

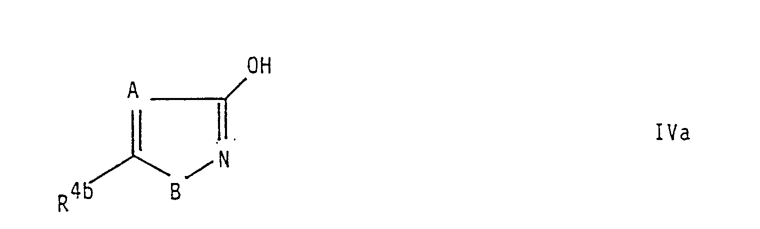

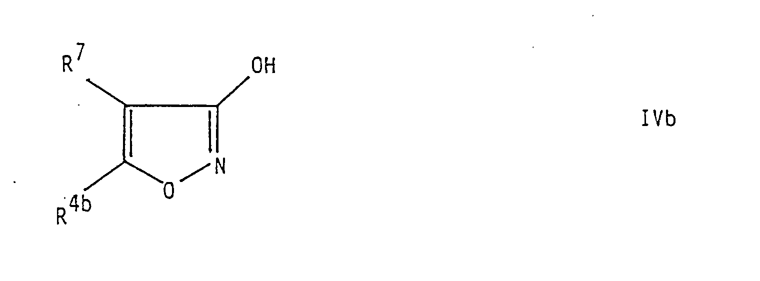

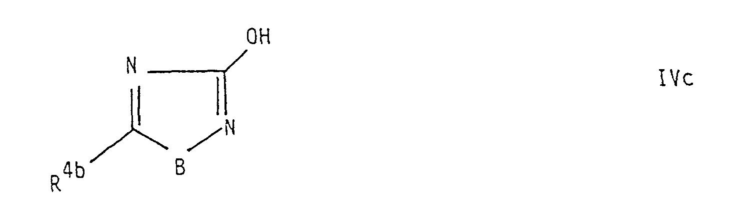

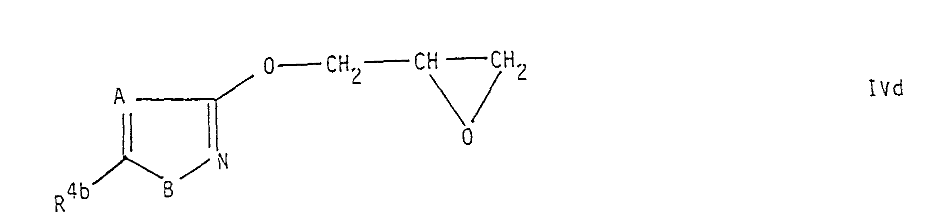

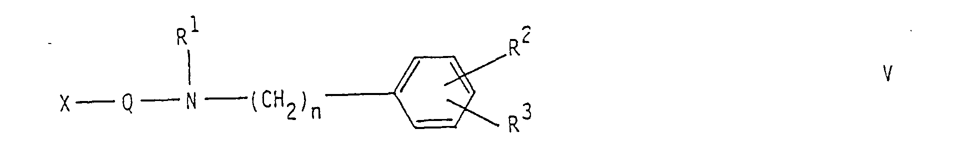

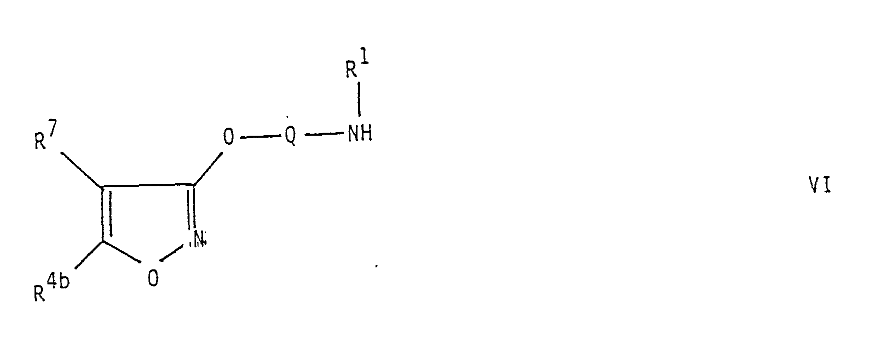

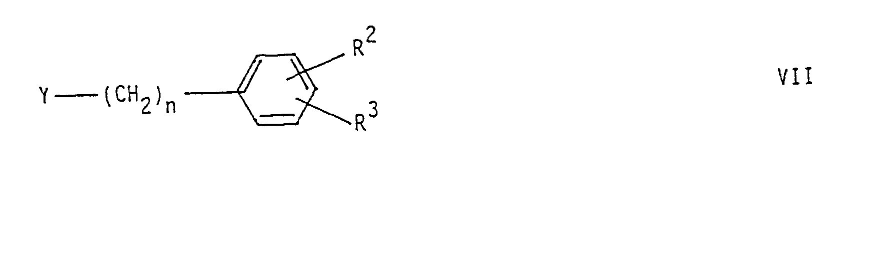

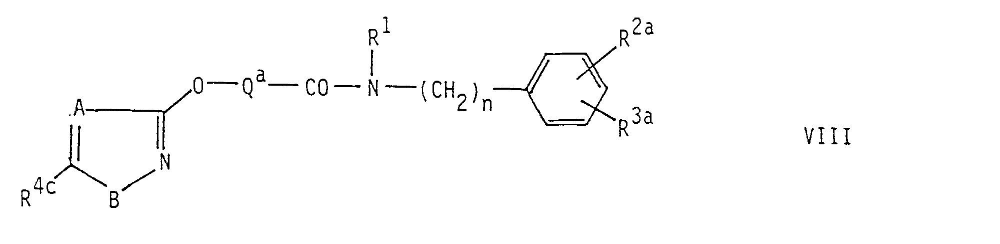

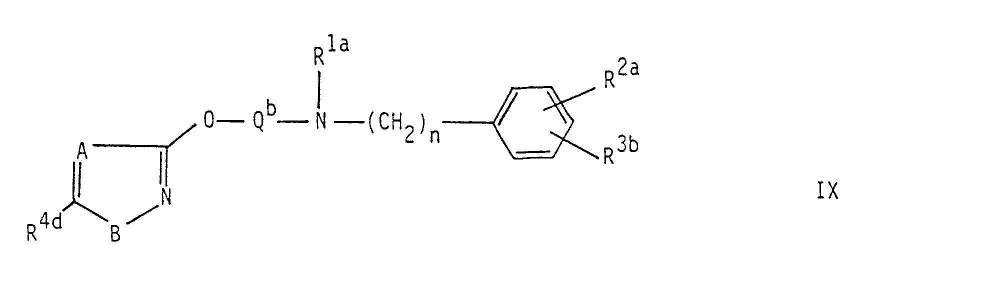

X^{a}―Q―T X

X^{a}―Q―T^{a} Xa

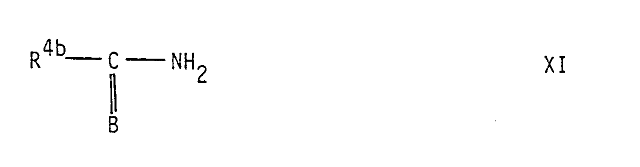

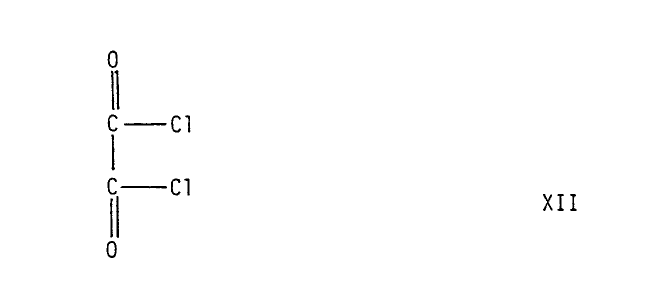

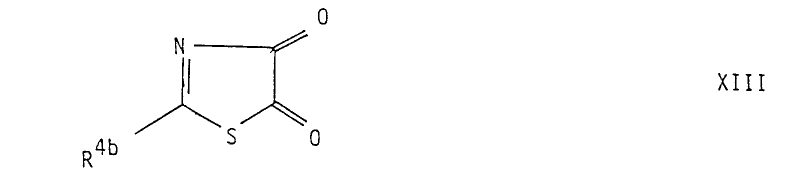

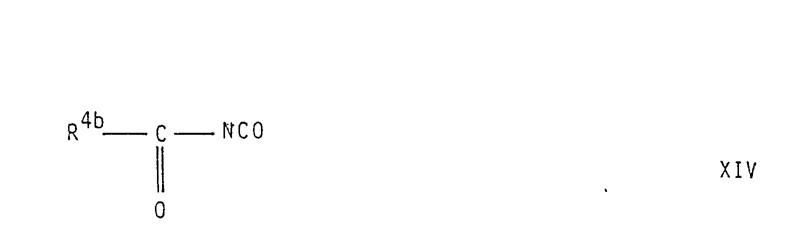

R^{4b}―CO―Cl XV

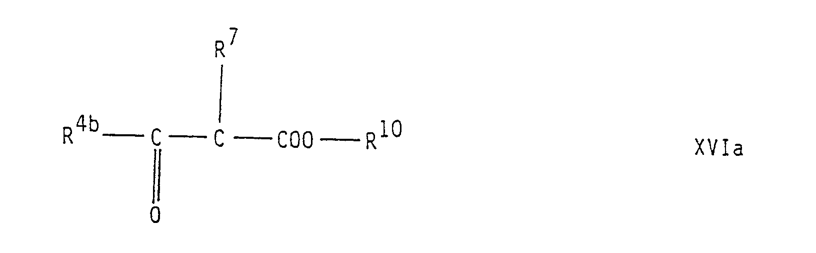

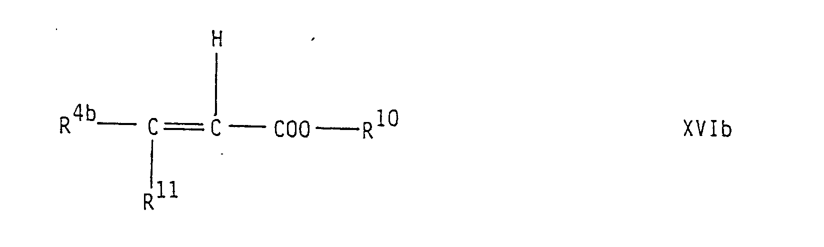

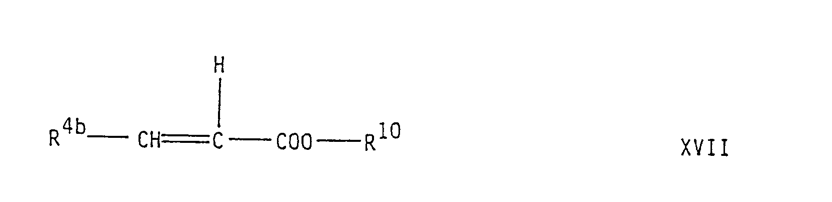

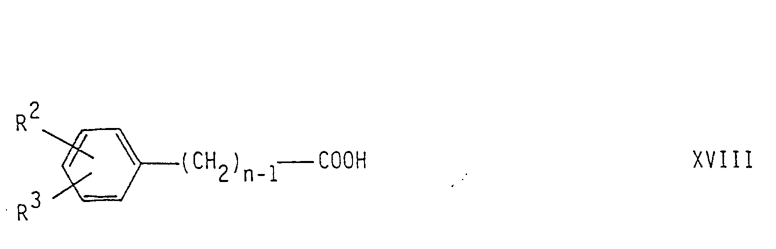

R¹―NH₂ XIX

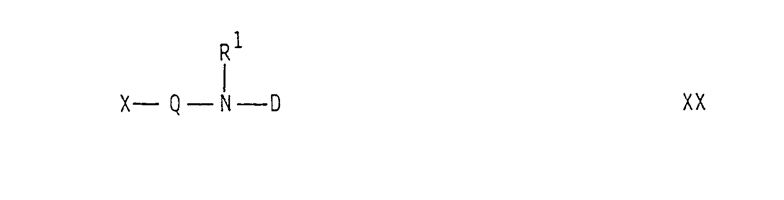

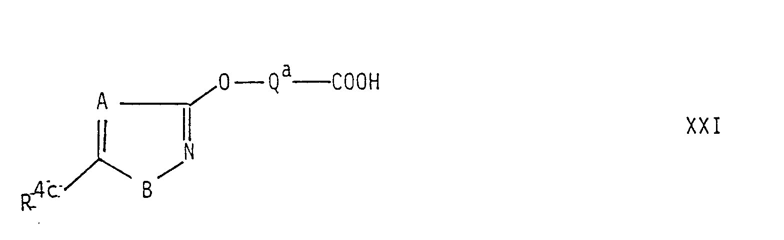

X^{a}― Q^{a}―COO―R¹² XXIIa

x^{a}―Q^{a}―CN XXIIb

## Patentansprüche

1. Verbindungen der allgemeinen Formel I 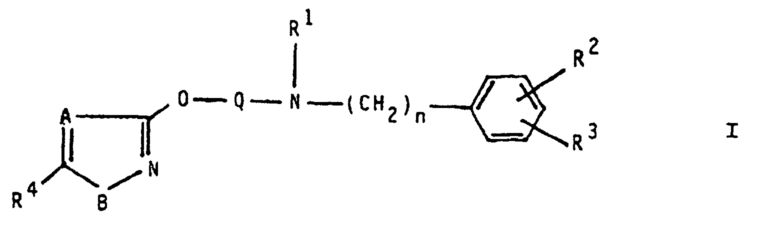 worin
R¹ Wasserstoff oder C₁-C₄-Alkyl bedeutet
R² Wasserstoff, Halogen, C₁-C₄-Alkyl, C₁-C₄-Alkoxy, Hydroxy oder, falls Q und R⁴ keine OH-Gruppe enthalten und R³ nicht Hydroxy ist, auch C₁-C₄-Alkanoyloxy bedeutet und
R³ Wasserstoff, Halogen, C₁-C₄-Alkyl, C₁-C₄-Alkoxy, Hydroxy oder, falls Q und R⁴ keine OH-Gruppe enthalten und R² nicht Hydroxy ist, auch C₁-C₄-Alkanoyloxy oder, falls R² Wasserstoff ist, auch Trifluormethyl, Nitro, Amino, C₁-C₄-Alkylamino, C₁-C₄-Alkylsulfonylamino oder C₁-C₄-Alkanoylamino bedeutet, wobei jedoch R³ nicht Nitro bedeutet, falls R⁶ Amino, C₁-C₄-Alkylamino oder C₁-C₄-Alkanoylamino ist, und nicht C₁-C₄-Alkanoylamino bedeutet, falls R⁶ Amino oder C₁-C₄-Alkylamino ist, oder
R² und R³ an zwei benachbarte Kohlenstoffatome gebunden sind und gemeinsam eine Alkylendioxygruppe mit 1 - 2 Kohlenstoffatomen darstellen,
R⁴ für Thienyl oder für eine gegebenenfalls substituierte Phenylgruppe der allgemeinen Formel a 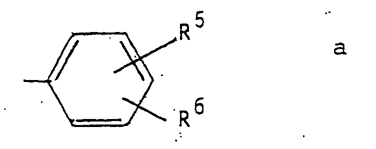
worin
R⁵ Wasserstoff, Halogen, C₁-C₄-Alkyl, C₁-C₄-Alkoxy, Hydroxy oder, falls Q keine OH-Gruppe enthält und R², R³ und R⁶ nicht Hydroxy sind, auch C₁-C₄-Alkanoyloxy bedeutet und
R⁶ Wasserstoff, Halogen, C₁-C₄-Alkyl, C₁-C₄-Alkoxy, Hydroxy oder, falls Q keine OH-Gruppe enthält und R², R³ und R⁵ nicht Hydroxy sind, auch C₁-C₄-Alkanoyloxyoder, falls R⁵ Wasserstoff ist, auch Trifluormethyl, Nitro, Amino, C₁-C₄-Alkylamino oder C₁-C₄-Alkanoylamino bedeutet,
A für Stickstoff oder eine R⁷-C-Gruppe, worin R⁷ Wasserstoff oder C₁-C₄-Alkyl bedeutet, steht,
B für Sauerstoff oder, falls A Stickstoff ist, auch für Schwefel steht,
n eine ganze Zahl von 2 bis 4 bedeutet, und
Q für eine (CH₂)ₘ-Gruppe, worin m eine ganze Zahl von 2 bis 8 bedeutet und welche gegebenenfalls durch C₁-C₄-Alkyl substituiert sein kann, oder für die 2-Hydroxypropylenkette steht,
sowie deren physiologisch verträgliche Säureadditionssalze.

2. Verbindungen gemäß Anspruch 1, worin R⁴ für eine gegebenenfalls substituierte Phenylgruppe der allgemeinen Formel a steht, wobei die Substituenten gemäß Anspruch 1 definiert sind.

3. Verbindungen gemäß Anspruch 2, worin in der gegebenenfalls substituierten Phenylgruppe a R⁵ obige Bedeutung besitzt und in 2- oder 3-Stellung angeordnet ist und R⁶ Wasserstoff bedeutet.

4. Verbindungen gemäß einem der vorstehenden Ansprüche, worin R¹ C₁-C₄-Alkyl bedeutet.

5. Verbindungen gemäß einem der vorstehenden Ansprüche worin R² und R³ in 3- und 4-Stellung stehen und C₁-C₄-Alkoxy oder Wasserstoff bedeuten.

6. Verbindungen gemäß einem der vorstehenden Ansprüche worin R¹ C₁-C₄-Alkyl bedeutet, R² Wasserstoff oder C₁-C₄-Alkoxy bedeutet, R³ C₁-C₄-Alkoxy bedeutet, R⁴ für eine Phenylgruppe der allgemeinen Formel a steht, worin R⁵ die obige Bedeutung besitzt und R⁶ Wasserstoff bedeutet, Q die Propylenkette bedeutet und n für 2 steht.

7. 3-{3-[N-2-(3,4-Dimethoxyphenyl)-ethyl)-N-methylamino]-propyloxy}-5-phenyl-1,2,4-thiadiazol und dessen physiologisch verträgliche Säureadditionssalze.

8. 3-{3-[N-(2-(3,4-Dimethoxyphenyl)-ethyl)-N-methylamino]-propyloxy}-5-phenyl-1,2,4-oxadiazol und dessen physiologisch verträgliche Säureadditionssalze.

9. 3-{3-[N-(2-(3,4-Dimethoxyphenyl)-ethyl)-N-methylamino)-propyloxy}-5-phenyl-isoxazol und dessen physiologisch verträgliche Säureadditionssalze.

10. Arzneimittel enthaltend eine pharmakologisch wirksame Menge einer-Verbindung gemäß. Anspruch 1 und übliche pharmazeutische Hilfs- und Trägerstoffe.

11. Verfahren zur Herstellung von Verbindungen der alLgemeinen Formel I 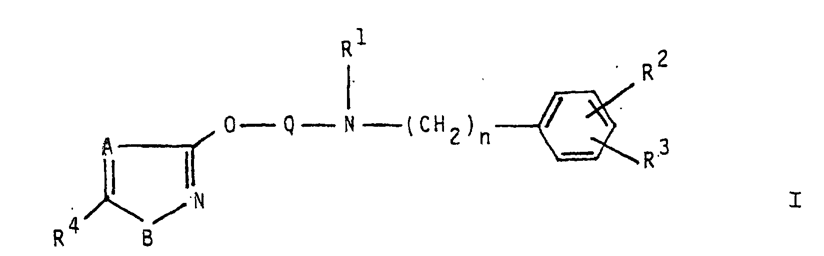 worin
R¹ Wasserstoff oder C₁-C₄-Alkyl bedeutet
R² Wasserstoff, Halogen, C₁-C₄-Alkyl, C₁-C₄-Alkoxy, Hydroxy oder, falls Q und R⁴ keine OH-Gruppe enthalten und R³ nicht Hydroxy ist, auch C₁-C₄-Alkanoyloxy bedeutet und
R³ Wasserstoff, Halogen, C₁-C₄-Alkyl, C₁-C₄-Alkoxy, Hydroxy oder, falls Q und R⁴ keine OH-Gruppe enthalten und R² nicht Hydroxy ist, auch C₁-C₄-Alkanoyloxy oder, falls R² Wasserstoff ist, auch Trifluormethyl, Nitro, Amino, C₁-C₄-Alkylamino, C₁-C₄-Alkylsulfonylamino oder C₁-C₄-Alkanoylamino bedeutet, wobei jedoch R³ nicht Nitro bedeutet, falls R⁶ Amino, C₁-C₄-Alkylamino oder C₁-C₄-Alkanoylamino ist, und nicht C₁-C₄-Alkanoylamino bedeutet, falls R⁶ Amino oder C₁-C₄-Alkylamino ist, oder
R² und R³ an zwei benachbarte Kohlenstoffatome gebunden sind und gemeinsam eine Alkylendioxygruppe mit 1 - 2 Kohlenstoffatomen darstellen,
R⁴ für Thienyl oder für eine gegebenenfalls substituierte Phenylgruppe der allgmeinen Formel a 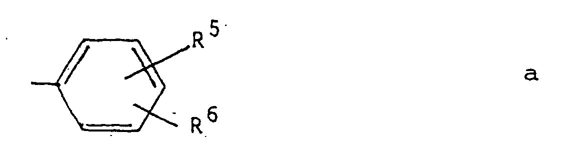
worin
R⁵ wasserstoff, Halogen, C₁-C₄-Alkyl, C₁-C₄-Alkoxy, Hydroxy oder, falls Q keine OH-Gruppe enthält und R², R³ und R⁶ nicht Hydroxy sind, auch C₁-C₄-Alkanoyloxy bedeutet und
R⁶ Wasserstoff, Halogen, C₁-C₄-Alkyl, C₁-C₄-Alkoxy, Hydroxy oder, falls Q keine OH-Gruppe enthält und R², R³ und R⁵ nicht Hydroxy sind, auch C₁-C₄-Alkanoyloxy oder, falls R⁵ Wasserstoff ist, auch Trifluormethyl, Nitro, Amino, C₁-C₄-Alkylamino oder C₁-C₄-Alkanoylamino bedeutet,
A für Stickstoff oder eine R⁷-C-Gruppe, worin R⁷ Wasserstoff oder C₁-C₄-Alkyl bedeutet, steht,
B für Sauerstoff oder, falls A Stickstoff ist, auch für Schwefel steht,
n eine ganze Zahl von 2 bis 4 bedeutet, und
Q für eine (CH₂)ₘ-Gruppe, worin m eine ganze Zahl von 2 bis 8 bedeutet und welche gegebenenfalls durch C₁-C₄Alkyl substituiert sein kann, oder für die 2-Hydroxypropylenkette steht,
sowie deren physiologisch verträglichen Säureadditionssalzen,
**dadurch gekennzeichnet, daß** man
a) zur Herstellung von Verbindungen der allgemeinen Formel Ia 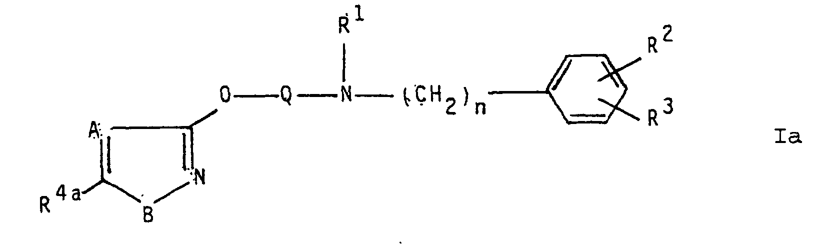 worin R¹, R², R³, A, B, Q und n obige Bedeutung besitzen und R^{4a} die für R⁴ angegebene Bedeutung mit Ausnahme von NH-haltigen Resten besitzt,
Verbindungen der allgemeinen Formel II 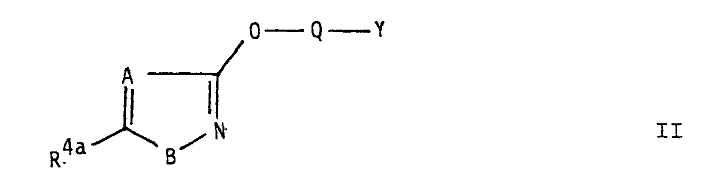 worin R^{4a}, A, B und Q obige Bedeutung besitzen und Y eine aminolytisch abspaltbare Fluchtgruppe bedeutet, mit Verbindungen der allgemeinen Formel IIIa 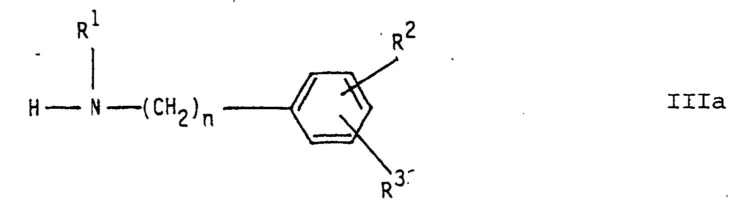 worin R¹, R², R³ und n obige Bedeutung besitzen, wobei jedoch allfällige freie Hydroxy- und/oder Aminogruppen R² und/oder R³ mit einer Schutzgruppe versehen sind, umsetzt und anschließend allfällige Hydroxy- und/oder Aminoschutzgruppen wieder abspaltet, oder
b) zur Herstellung von Verbindungen der allgemeinen Formel Ib 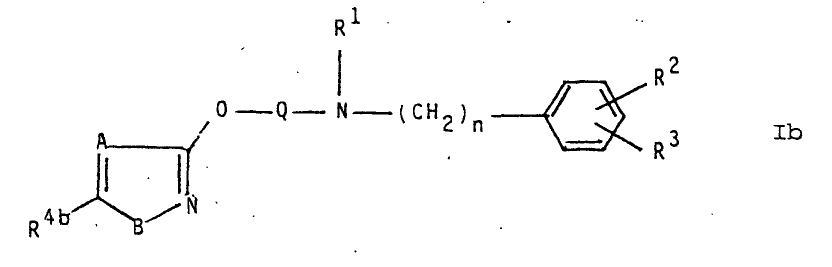 worin R¹, R², R³, A, B, Q und n obige Bedeutung besitzen und R^{4b} die für R⁴ angegebene Bedeutung mit Ausnahme von Hydroxy- und/oder NH-haltigen Resten besitzt,
Verbindungen der allgemeinen Formel IVa 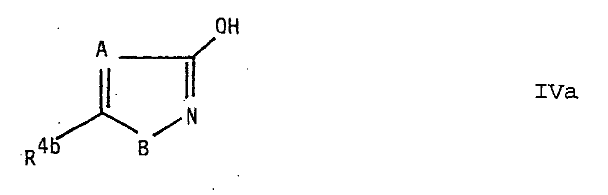 worin R^{4b}, A und B obige Bedeutung besitzen,
mit Verbindungen der allgemeinen Formel V 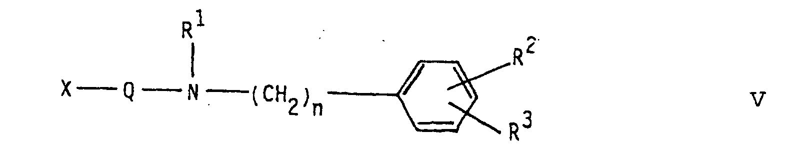 worin R¹, R², R³, n und Q obige Bedeutung besitzen, wobei jedoch allfällige freie Hydroxy- und/oder Aminogruppen mit einer Schutzgruppe versehen sind, und X für eine abspaltbare Fluchtgruppe steht, umsetzt und anschließend allfällige Hydroxy- und/oder Aminoschutzgruppen wieder abspaltet, oder
c) zur Herstellung von Verbindungen der allgemeinen Formel Ic 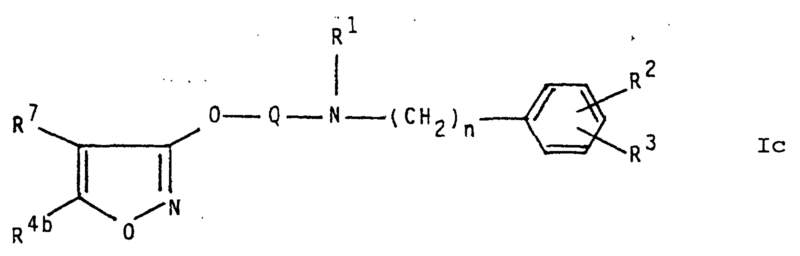 worin R¹, R², R³, R^{4b}, R⁷, Q und n obige Bedeutung besitzen, Verbindungen der allgemeinen Formel VI 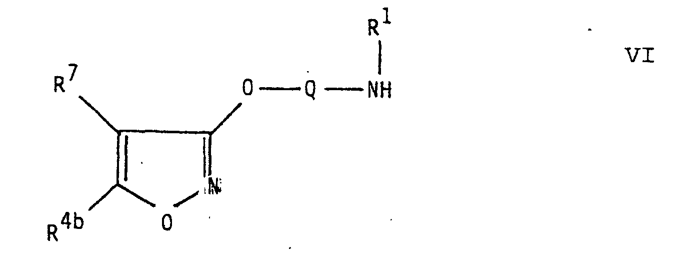 worin R¹, R⁷ und R^{4b} obige Bedeutung besitzen, mit Verbindungen der allgemeinen Formel VII 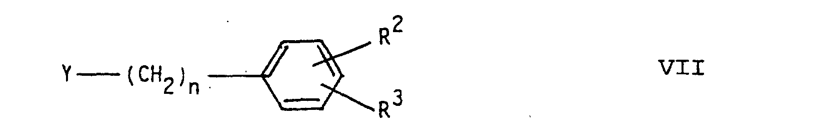 worin Y, n, R² und R³ obige Bedeutung besitzen, wobei jedoch freie Amino- und/oder Hydroxygruppen R² und/oder R³ mit einer Schutzgruppe versehen sind, umsetzt und anschließend allfällige Hydroxy- und/oder Aminoschutzgruppen wieder abspaltet, oder
d) zur Herstellung von Verbindungen der allgemeinen Formel Id 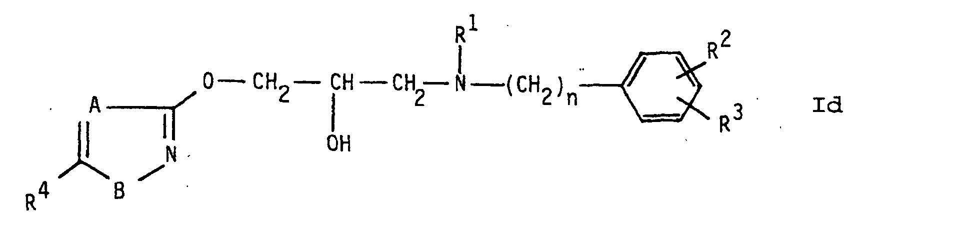 worin R¹, R², R³, R^{4b}, A, B, und n obige Bedeutung besitzen,
Verbindungen der allgmeinen Formel IV 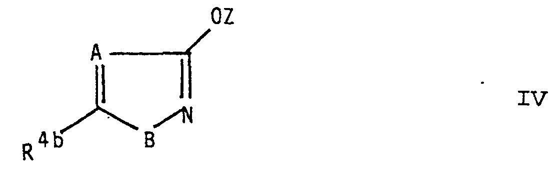 worin R^{4b}, A und B obige Bedeutung besitzen und Z für die 2,3-Epoxypropyl-Gruppe oder, falls A eine R⁷-C-
Gruppe ist, auch für Wasserstoff steht, mit Verbindungen der allgemeinen Formel III 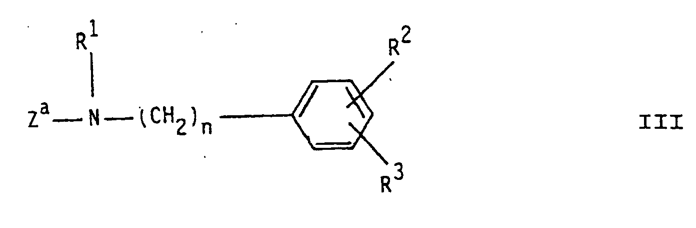 worin R¹, R², R³ und n obige Bedeutung besitzen, wobei jedoch allfällige freie Hydroxy- und/oder Aminogruppen mit einer Schutzgruppe versehen sind, und Z^{a} für Wasserstoff oder, falls Z in den Verbindungen der Formel IV Wasserstoff ist, auch für die 2,3-Epoxypropyl-Gruppe steht, umsetzt und anschließend allfällige Hydroxyund/oder Aminoschutzgruppen wieder abspaltet, oder
e) zur Herstellung von Verbindungen der allgemeinen Formel Ie 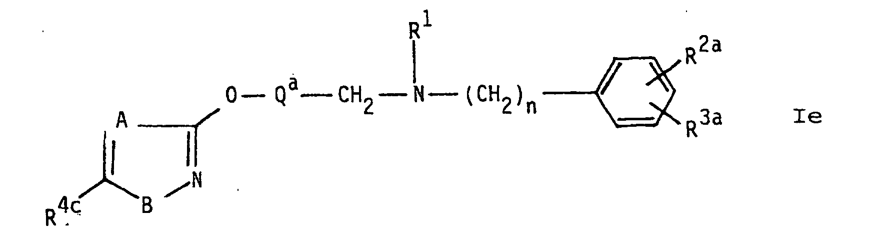 worin R¹, n, A und B obige Bedeutung besitzen, R^{4c} die für R^{4b} angegebene Bedeutung mit Ausnahme von Nitro und/oder C₁-C₄-Alkanoyloxy enthaltenden Resten besitzt, R^{2a} und R^{3a} die für R² und R³ angegebenen Bedeutungen mit Ausnahme von Nitro, C₁-C₄-Alkanoyloxy und C₁-C₄-Alkanoylamino besitzen und Q^{a} für eine (CH₂)ₘ^{a}-Gruppe steht, worin m^{a} 3 oder 4 bedeutet und welche gegebenenfalls durch C₁-C₄-Alkyl substituiert sein kann, steht, Verbindungen der allgemeinen Formel VIII 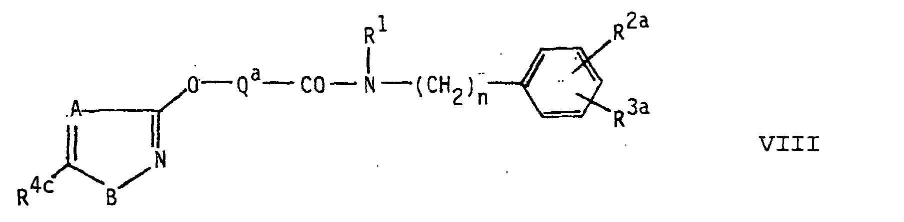 worin R¹, R^{2a}, R^{3a}, R^{4c}, A, B, Q^{a} und n obige Bedeutung besitzen, wobei jedoch allfällige freie Hydroxy- und/oder Aminogruppen mit einer Schutzgruppe versehen sind, reduziert und anschließend allfällige Hydroxy- und/oder Aminoschutzgruppen wieder abspaltet, oder
f) zur Herstellung von Verbindungen der allgemeinen Formel If 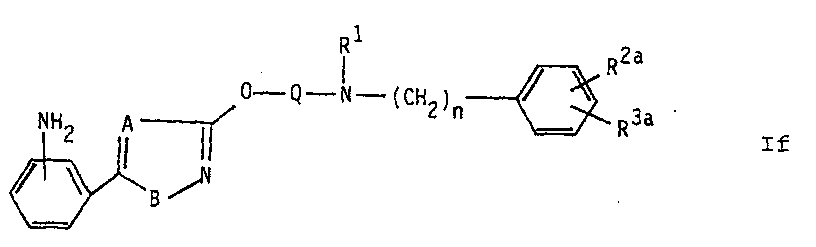 worin R¹, R^{2a}, R^{3a}, A, B, Q und n obige Bedeutung besitzen, Verbindungen der allgemeinen Formel Ig 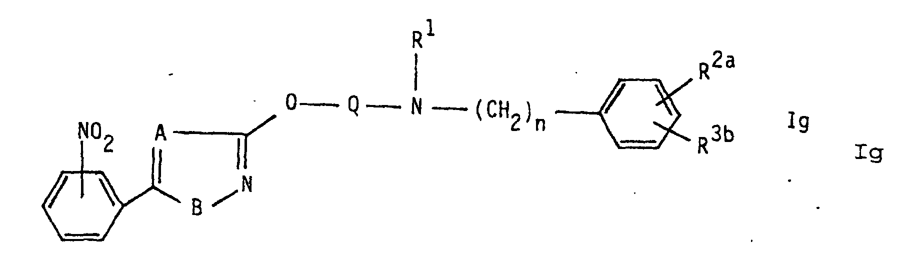 worin R¹, R^{2a}, A, B, Q und n obige Bedeutung besitzen und R^{3b} die für R³ angegebene Bedeutung mit Ausnahme von alkanoylhaltigen Resten besitzt, reduziert, oder
g) zur Herstellung von Verbindungen der allgemeinen Formel Ih 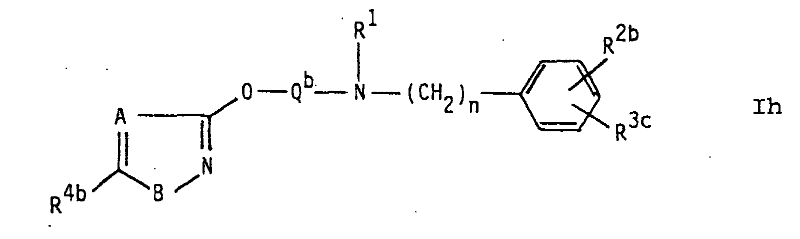 worin R¹, R^{4b}, A, B und n obige Bedeutung besitzen, Q^{b} die für Q angegebene Bedeutung mit Ausnahme der 2-Hydroxypropylenkette besitzt und R^{2b} und R^{3c} die für R² und R³ angegebenen Bedeutungen mit Ausnahme von OH-Gruppen besitzen, wobei jedoch von den Substituenten R^{2b} und R^{3c} und/oder von in R^{4b} enthaltenen Substituenten R⁵ und R⁶ mindestens einer C₁-C₄-Alkanoyloxy oder C₁-C₄-Alkanoylamino bedeutet, Verbindungen der allgemeinen Formel IX 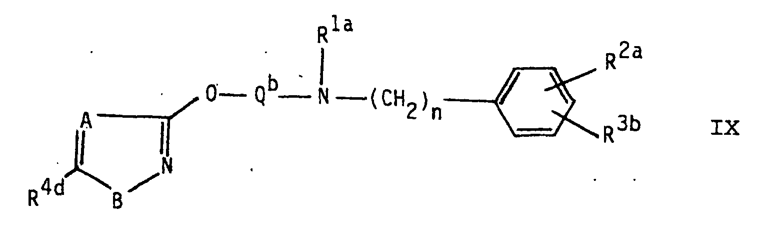 worin R^{2a}, R^{3b}, A, B, Q^{b} und n obige Bedeutung besitzen, R^{1a} C₁-C₄-Alkyl oder eine Aminoschutzgruppe bedeutet und R^{4d} die für R⁴ angegebene Bedeutung mit Ausnahme von eine C₁-C₄-Alkanoylgruppe enthaltenden Resten besitzt und worin gegebenenfalls Hydroxy- und/oder Aminoschutzgruppen enthalten sein können, wobei jedoch von den Substituenten R^{2a} und R^{3b} und/oder von in R^{4d} enthaltenen Substituenten R⁵ und R⁶ mindestens einer eine freie OH- oder NH₂-Gruppe bedeutet, acyliert und anschließend allfällige Schutzgruppen wieder abspaltet, oder
h) zur Herstellung von Verbindungen der allgemeinen Formel Ii 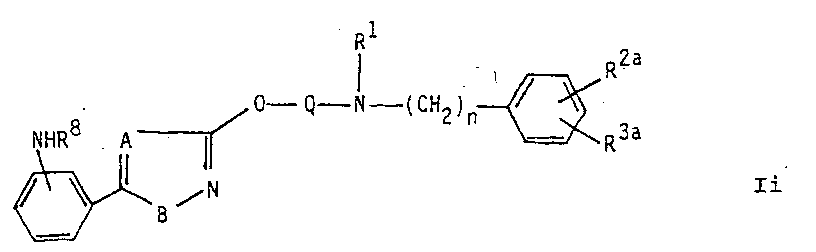
worin A, B, Q, R¹, R^{2a}, R^{3a} und n obige Bedeutung besitzen, und R⁸ C₁-C₄-Alkyl bedeutet,
Verbindungen der allgemeinen Formel Ij 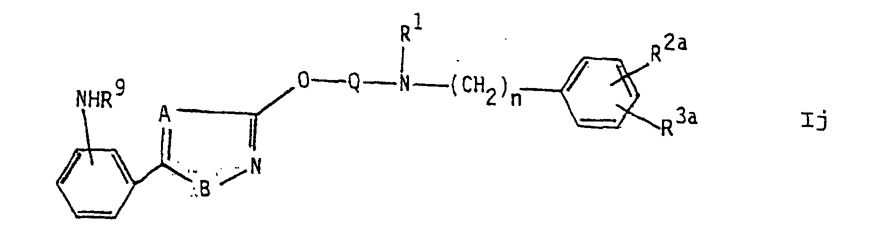 worin R¹, R^{2a}, R^{3a}, A, B und n obige Bedeutung besitzen und R⁹ C₁-C₄-Alkanoyl bedeutet, reduziert, und
gewünschtenfalls in erhaltenen Verbindungen der Formel I, worin R², R³, R⁵ und/oder R⁶ Methoxy bedeuten, aus diesen Gruppen eine Hydroxygruppe freisetzt und/oder gewünschtenfalls erhaltene Verbindungen der Formel I, worin R¹ Wasserstoff bedeutet, zu Verbindungen der Formel I, worin R¹ C₁-C₄-Alkyl bedeutet, alkyliert und gewünschstenfalls freie Verbindungen der Formel I in ihre Säureadditionssalze überführt oder die Säureadditionssalze in die freien Verbindungen der Formel I überführt.

12. Verbindungen der allgemeinen Formel VIII 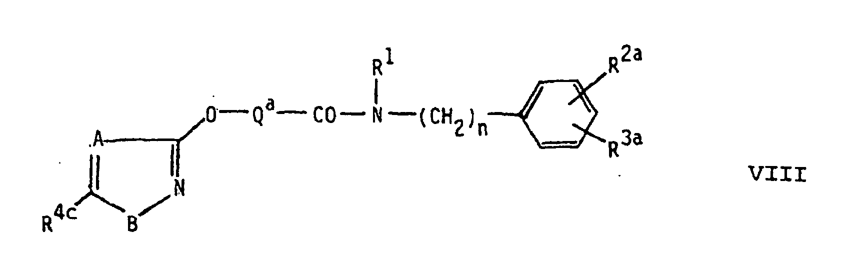 worin
R¹ Wasserstoff oder C₁-C₄-Alkyl bedeutet
R^{2a} Wasserstoff, Halogen, C₁-C₄-Alkyl, C₁-C₄-Alkoxy, oder Hydroxy und
R^{3a} Wasserstoff, Halogen, C₁-C₄-Alkyl, C₁-C₄-Alkoxy, Hydroxy oder, falls R² Wasserstoff ist, auch Trifluormethyl, Amino, C₁-C₄-Alkylamino oder C₁-C₄-Alkylsulfonylamino bedeutet, oder
R² und R³ an zwei benachbarte Kohlenstoffatome gebunden sind und gemeinsam eine Alkylendioxygruppe mit 1-2 Kohlenstoffatomen darstellen,
R^{4c} für Thienyl oder für eine gegebenenfalls substituierte Phenylgruppe der allgemeinen Formel a 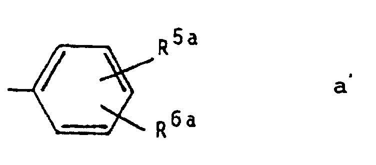
worin
R^{5a} Wasserstoff, Halogen, C₁-C₄-Alkyl, C₁-C₄-Alkoxy oder, falls R²⁸ und R^{3a} nicht Hydroxy sind, auch C₁-C₄-Alkanoyloxy bedeutet und
R^{6a} Wasserstoff, Halogen, C₁-C₄-Alkyl, C₁-C₄-Alkoxy, oder, falls R^{2a} und R^{3a} nicht Hydroxy sind, auch C₁-C₄-Alkanoyloxy oder, falls R⁵ Wasserstoff ist, auch Trifluormethyl oder C₁-C₄-Alkanoylamino bedeutet,
A für Stickstoff oder eine R⁷-C-Gruppe, worin R⁷ Wasserstoff oder C₁-C₄-Alkyl bedeutet, steht
B für Sauerstoff oder, falls A Stickstoff ist, auch für Schwefel steht,
n eine ganze Zahl von 2 bis 4 bedeutet, und
Q^{a} für eine (CH₂)m^{a}-Gruppe, worin m^{a} 3 oder 4 bedeutet und welche gegebenenfalls durch C₁-C₄-Alkyl substituiert sein kann,steht.

## Claims

1. Compounds of the general formula I, 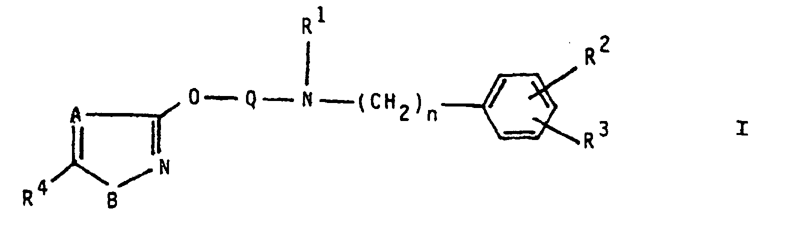 wherein
R¹ is hydrogen or C₁-C₄-alkyl,
R² is hydrogen, halogen, C₁-C₄-alkyl, C₁-C₄-alkoxy, hydroxy or, if Q and R⁴ do not contain an OH group and R³ is not hydroxy, also C₁-C₄-alkanoyloxy, and
R³ is hydrogen, halogen, C₁-C₄-alkyl, C₁-C₄-alkoxy, hydroxy or, if Q and R⁴ do not contain an OH group and R² is not hydroxy, also C₁-C₄-alkanoyloxy or, if R² is hydrogen, also trifluoromethyl, nitro, amino, C₁-C₄-alkylamino, C₁-C₄-alkylsulphonylamino or C₁-C₄-alkanoylamino, in which case however R³ is not nitro if R⁶ is amino, C₁-C₄-alkylamino or C₁-C₄-alkanoylamino, and is not C₁-C₄-alkanoylamino, if R⁶ is amino or C₁-C₄-alkylamino, or
R² and R³ are bonded to two adjacent carbon atoms and together represent an alkylenedioxy group with 1 - 2 carbon atoms,
R⁴ stands for thienyl or for an optionally substituted phenyl group of the general formula a 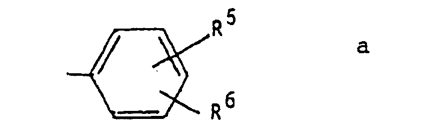
wherein
R⁵ is hydrogen, halogen, C₁-C₄-alkyl, C₁-C₄-alkoxy, hydroxy or, if Q does not contain an OH group and R², R³ and R⁶ are not hydroxy, also C₁-C₄-alkanoyloxy, and
R⁶ is hydrogen, halogen, C₁-C₄-alkyl, C₁-C₄-alkoxy, hydroxy or, if Q does not contain an OH group and R², R³ and R⁵ are not hydroxy, also C₁-C₄-alkanoyloxy or, if R⁵ is hydrogen, also trifluoromethyl, nitro, amino, C₁-C₄-alkylamino or C₁-C₄-alkanoylamino,
A stands for nitrogen or for an R⁷-C group, wherein R⁷ is hydrogen or C₁-C₄-alkyl,
B stands for oxygen or, if A is nitrogen, also for sulphur,
n is a whole number from 2 to 4, and
Q stands for a (CH₂)ₘ-group, wherein m is a whole number from 2 to 8 and which may optionally be substituted by C₁-C₄-alkyl, or for the 2-hydroxypropylene chain,
and their physiologically compatible acid addition salts.

2. Compounds according to Claim 1, wherein R⁴ stands for an optionally substituted phenyl group of the general formula a, the substituents being defined according to Claim 1.

3. Compounds according to Claim 2, wherein in the optionally substituted phenyl group a R⁵ has the above meaning and is in position 2 or 3 and R⁶ is hydrogen.

4. Compounds according to one of the preceding claims, wherein R¹ is C₁-C₄-alkyl.

5. Compounds according to one of the preceding claims, wherein R² and R³ are in position 3 and 4 and are C₁-C₄-alkoxy or hydrogen.

6. Compounds according to one of the preceding claims, wherein R¹ is C₁-C₄-alkyl, R² is hydrogen or C₁-C₄-alkoxy, R³ is C₁-C₄-alkoxy, R⁴ stands for a phenyl group of the general formula a wherein R⁵ has the above meaning and R⁶ is hydrogen, Q is the propylene chain and n stands for 2.

7. 3-{3-[N-2-(3,4-dimethoxyphenyl)-ethyl)-N-methylamino]-propyloxy}-5-phenyl-1,2,4-thiadiazole and the physiologically compatible acid addition salts thereof.

8. 3-{3-[N-(2-(3,4-dimethoxyphenyl)-ethyl)-N-methylamino]-propyloxy}-5-phenyl-1,2,4-oxadiazole and the physiologically compatible acid addition salts thereof.

9. 3-{3-[N-(2-(3,4-dimethoxyphenyl)-ethyl)-N-methylamino)-propyloxy}-5-phenyl-isoxazole and the physiologically compatible acid addition salts thereof.

10. Medicament containing a pharmacologically active quantity of a compound according to Claim 1 and conventional pharmaceutical auxiliaries and/or carriers.

11. A process for the preparation of compounds of the general formula I 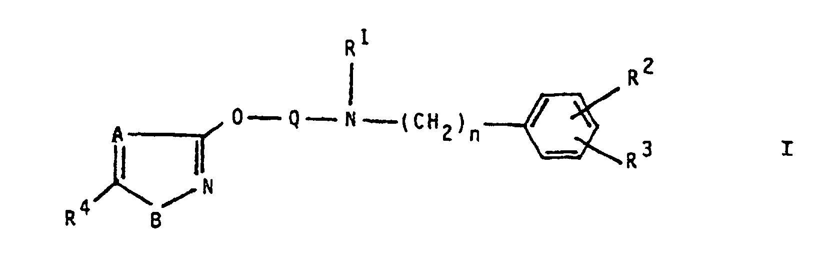 wherein
R¹ is hydrogen or C₁-C₄-alkyl,
R² is hydrogen, halogen, C₁-C₄-alkyl, C₁-C₄-alkoxy, hydroxy or, if Q and R⁴ do not contain an OH group and R³ is not hydroxy, also C₁-C₄-alkanoyloxy, and
R³ is hydrogen, halogen, C₁-C₄-alkyl, C₁-C₄-alkoxy, hydroxy or, if Q and R⁴ do not contain an OH group and R² is not hydroxy, also C₁-C₄-alkanoyloxy or, if R² is hydrogen, also trifluoromethyl, nitro, amino, C₁-C₄-alkylamino, C₁-C₄-alkylsulphonylamino or C₁-C₄-alkanoylamino, in which case however R³ is not nitro if R⁶ is amino, C₁-C₄-alkylamino or C₁-C₄-alkanoylamino, and is not C₁-C₄-alkanoylamino, if R⁶ is amino or C₁-C₄-alkylamino, or
R² and R³ are bonded to two adjacent carbon atoms and together represent an alkylenedioxy group with 1 - 2 carbon atoms,
R⁴ stands for thienyl or for an optionally substituted phenyl group of the general formula a 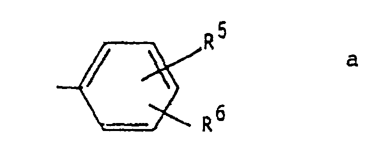
wherein
R⁵ is hydrogen, halogen, C₁-C₄-alkyl, C₁-C₄-alkoxy, hydroxy or, if Q does not contain an OH group and R², R³ and R⁶ are not hydroxy, also C₁-C₄-alkanoyloxy, and
R⁶ is hydrogen, halogen, C₁-C₄-alkyl, C₁-C₄-alkoxy, hydroxy or, if Q does not contain an OH group and R², R³ and R⁵ are not hydroxy, also C₁-C₄-alkanoyloxy or, if R⁵ is hydrogen, also trifluoromethyl, nitro, amino, C₁-C₄-alkylamino or C₁-C₄-alkanoylamino,
A stands for nitrogen or for an R⁷-C group, wherein R⁷ is hydrogen or C₁-C₄-alkyl,
B stands for oxygen or, if A is nitrogen, also for sulphur,
n is a whole number from 2 to 4, and
Q stands for a (CH₂)ₘ-group, wherein m is a whole number from 2 to 8 and which may optionally be substituted by C₁-C₄-alkyl, or for the 2-hydroxypropylene chain,
and also the physiologically compatible acid addition salts thereof, **characterised in that**
a) for the preparation of compounds of the general formula Ia 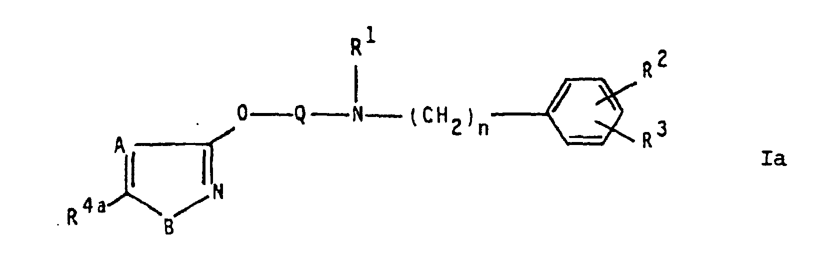 wherein R¹, R², R³, A, B, Q and n have the above meaning and R^{4a} has the meaning given for R⁴ with the exception of NH-containing radicals,
compounds of the general formula II 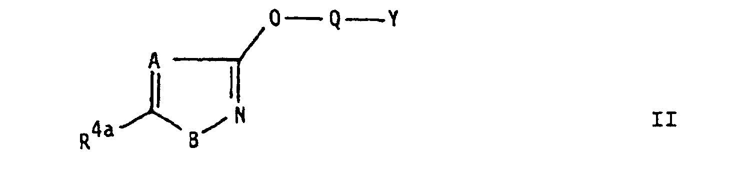 wherein R^{4a}, A, B and Q have the above meaning and Y is an aminolytically cleavable leaving group, are reacted with compounds of the general formula IIIa 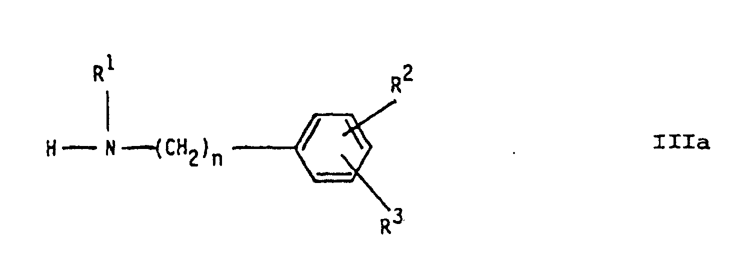 wherein R¹, R², R³ and n have the above meaning, but any free hydroxy and/or amino groups R² and/or R³ are provided with a protective group, and then any hydroxy and/or amino protective groups are cleaved off again, or
b) for the preparation of compounds of the general formula Ib 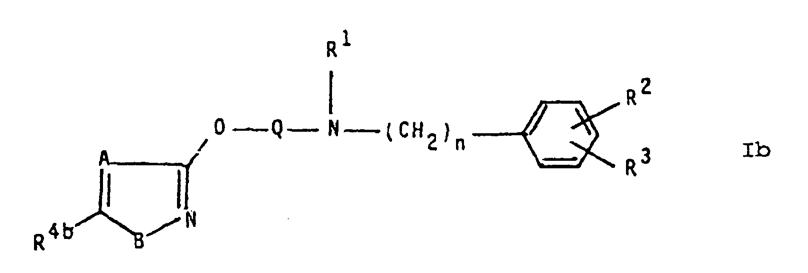 wherein R¹, R², R³, A, B, Q and n have the above meaning and R^{4b} has the meaning given for R⁴ with the exception of hydroxy- and/or NH-containing radicals,
compounds of the general formula IVa 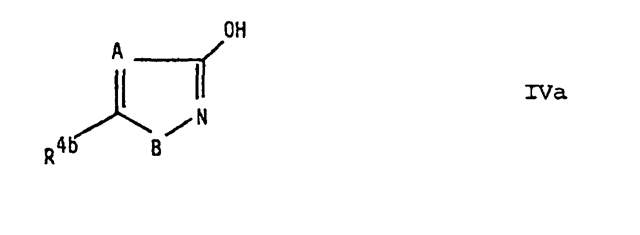 wherein R^{4b}, A and B have the above meaning,
are reacted with compounds of the general formula V 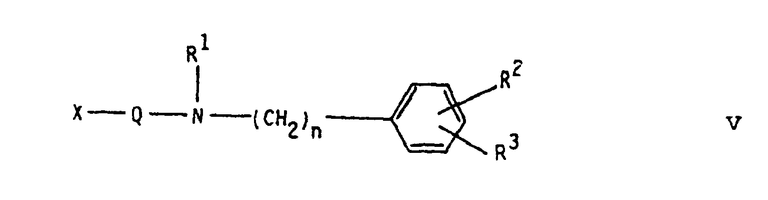 wherein R¹, R², R³, n and Q have the above meaning, but any free hydroxy and/or amino groups are provided with a protective group, and X stands for a cleavable leaving group, and then any hydroxy and/or amino protective groups are cleaved off again, or
c) for the preparation of compounds of the general formula Ic 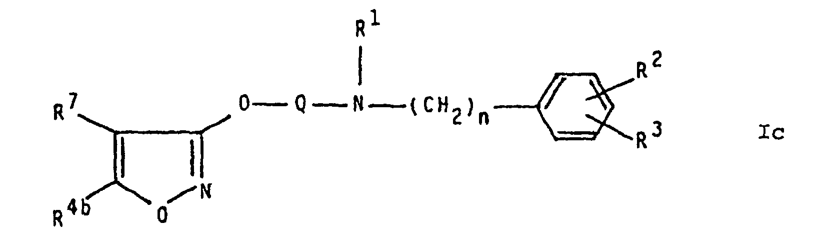 wherein R¹, R², R³, R^{4b}, R⁷, Q and n have the above meaning, compounds of the general formula VI 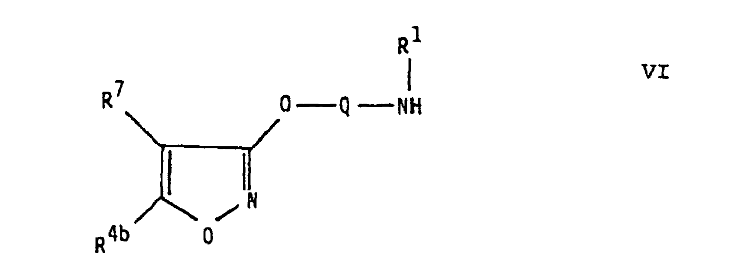 wherein R¹, R⁷ and R^{4b} have the above meaning, are reacted with compounds of the general formula VII 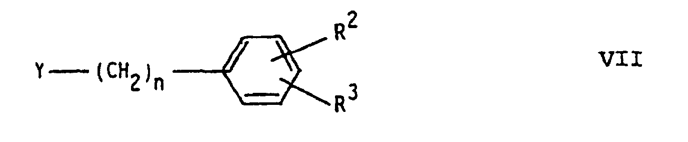 wherein Y, n, R² and R³ have the above meaning, but free amino and/or hydroxy groups R² and/or R³ are provided with a protective group, and then any hydroxy and/or amino protective groups are cleaved off again, or
d) for the preparation of compounds of the general formula Id 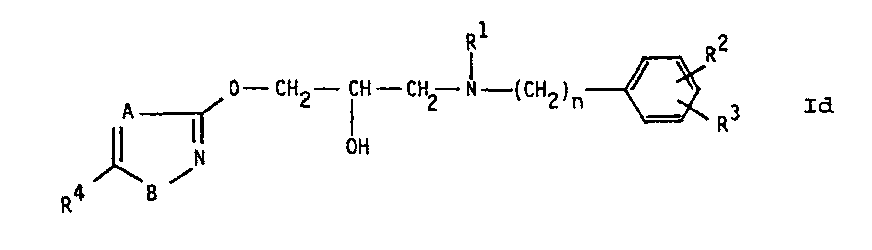 wherein R¹, R², R³, R^{4b}, A, B and n have the above meaning, compounds of the general formula IV 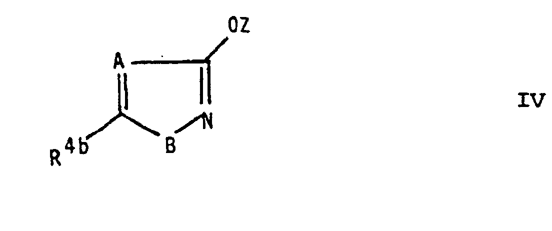 wherein R^{4b}, A and B have the above meaning and Z stands for the 2,3-epoxypropyl group or, if A is an R⁷-C group, also for hydrogen, are reacted with compounds of the general formula III 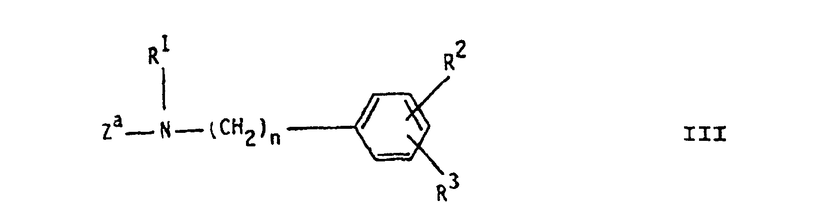 wherein R¹, R², R³ and n have the above meaning, but any free hydroxy and/or amino groups are provided with a protective group, and Z^{a} stands for hydrogen or, if Z in the compounds of Formula IV is hydrogen, also stands for the 2,3-epoxypropyl group, and then any hydroxy and/or amino protective groups are cleaved off again, or
e) for the preparation of compounds of the general formula le 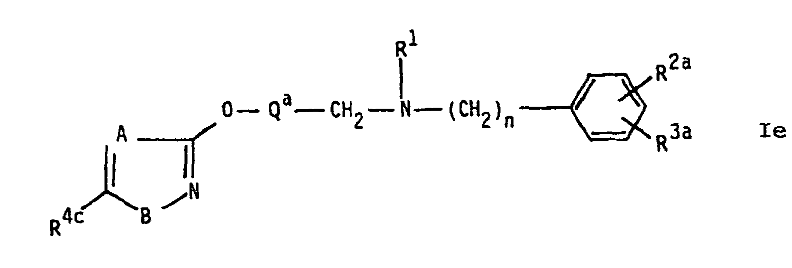 wherein R¹, n, A and B have the above meaning, R^{4c} has the meaning given for R^{4b} with the exception of nitro and/or radicals containing C₁-C₄-alkanoyloxy, R^{2a} and R^{3a} have the meanings given for R² and R³ with the exception of nitro, C₁-C₄-alkanoyloxy and C₁-C₄-alkanoylamino and Q^{a} stands for a (CH₂)ₘ^{a} group wherein m^{a} is 3 or 4 and which may optionally be substituted by C₁-C₄-alkyl, compounds of the general formula VIII 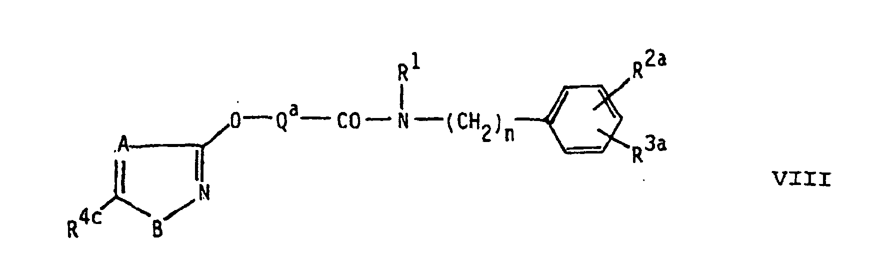 wherein R¹, R^{2a}, R^{3a}, R^{4c}, A, B, Q^{a} and n have the above meaning, but any free hydroxy and/or amino groups are provided with a protective group, are reduced and then any hydroxy and/or amino protective groups are cleaved off again, or
f) for the preparation of compounds of the general formula If 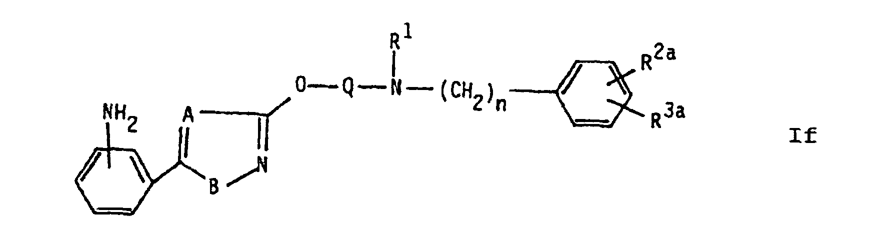 wherein R¹, R^{2a}, R^{3a}, A, B, Q and n have the above meaning, compounds of the general formula Ig 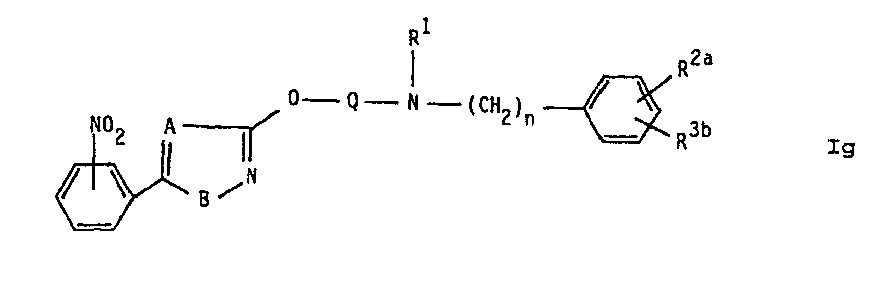 wherein R¹, R^{2a}, A, B, Q and n have the above meaning and R^{3b} has the meaning given for R³ with the exception of alkanoyl-containing radicals, are reduced, or
g) for the preparation of compounds of the general formula Ih 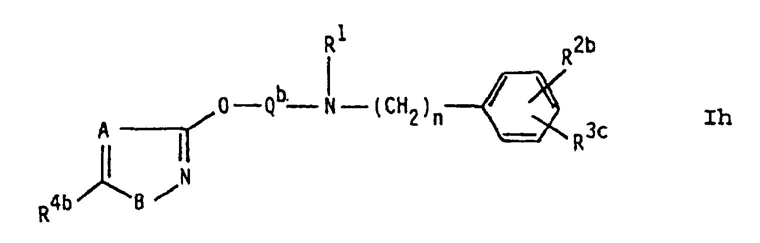 wherein R¹, R^{4b}, A, B and n have the above meanings, Q^{b} has the meaning given for Q with the exception of the 2-hydroxypropylene chain and R^{2b} and R^{3c} have the meanings given for R² and R³ with the exception of OH groups, but of the substituents R^{2b} and R^{3c} and/or of substituents R⁵ and R⁶ contained in R^{4b} at least one is C₁-C₄-alkanoyloxy or C₁-C₄-alkanoylamino, compounds of the general formula IX 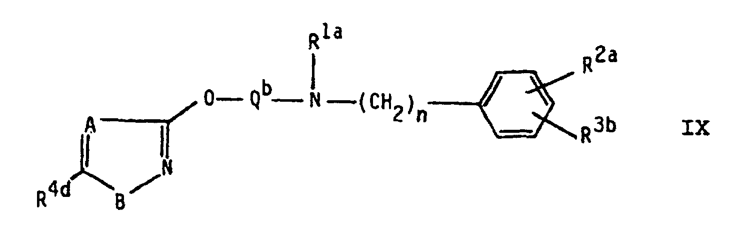 wherein R^{2a}, R^{3b}, A, B, Q^{b} and n have the above meanings, R^{1a} is C₁-C₄-alkyl or an amino protective group and R^{4d} has the meaning given for R⁴ with the exception of radicals containing a C₁-C₄-alkanoyl group and wherein optionally hydroxy and/or amino protective groups may be contained, but of the substituents R^{2a} and R^{3b} and/or of substituents R⁵ and R⁶ contained in R^{4d} at least one is a free OH or NH₂ group, are acylated and then any protective groups are cleaved off again, or
h) for the preparation of compounds of the general formula li 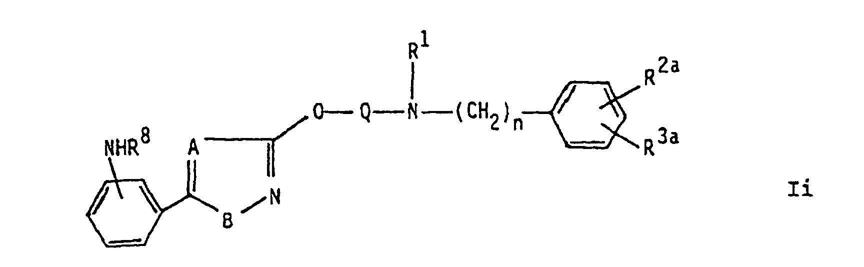
wherein A, B, Q, R¹, R^{2a}, R^{3a} and n have the above meaning, and R⁸ is C₁-C₄-alkyl,
compounds of the general formula Ij 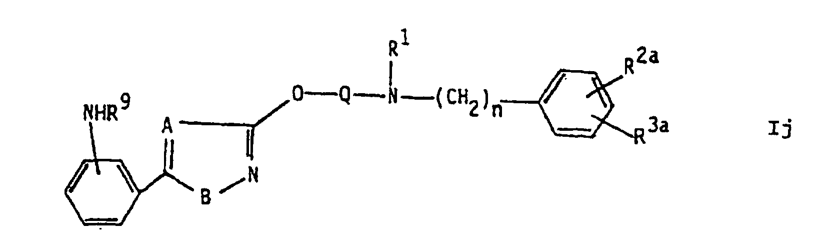 wherein R¹, R^{2a}, R^{3a}, A, B and n have the above meaning, and R⁹ is C₁-C₄-alkanoyl, are reduced, and
if desired in resulting compounds of Formula I, wherein R², R³, R⁵ and/or R⁶ are methoxy, a hydroxy group is released from these groups and/or if desired resulting compounds of Formula I, wherein R¹ is hydrogen, are alkylated to form compounds of Formula I, wherein R¹ is C₁-C₄-alkyl, and if desired free compounds of Formula I are converted into their acid addition salts or the acid addition salts are converted into the free compounds of Formula I.

12. Compounds of the general formula VIII 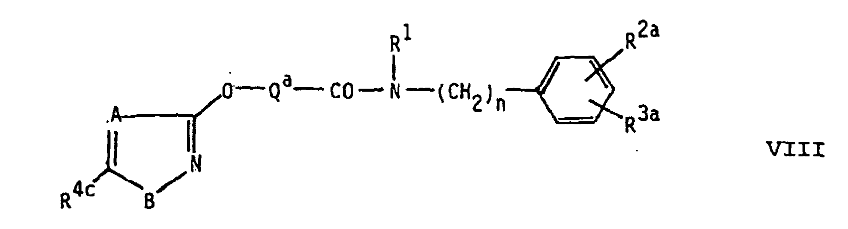 wherein
R¹ is hydrogen or C₁-C₄-alkyl,
R^{2a} is hydrogen, halogen, C₁-C₄-alkyl, C₁-C₄-alkoxy, or hydroxy and
R^{3a} is hydrogen, halogen, C₁-C₄-alkyl, C₁-C₄-alkoxy, hydroxy or, if R² is hydrogen, also trifluoromethyl, amino, C₁-C₄-alkylamino or C₁-C₄-alkylsulphonylamino, or
R² and R³ are bonded to two adjacent carbon atoms and together represent an alkylenedioxy group with 1 - 2 carbon atoms,
R^{4c} stands for thienyl or for an optionally substituted phenyl group of the general formula a' 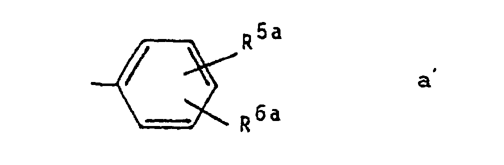
wherein
R^{5a} is hydrogen, halogen, C₁-C₄-alkyl, C₁-C₄-alkoxy or, if R^{2a} and R^{3a} are not hydroxy, also C₁-C₄-alkanoyloxy, and
R^{6a} is hydrogen, halogen, C₁-C₄-alkyl, C₁-C₄-alkoxy, or, if R^{2a} and R^{3a} are not hydroxy, also C₁-C₄-alkanoyloxy, or, if R⁵ is hydrogen, also trifluoromethyl or C₁-C₄-alkanoylamino,
A stands for nitrogen or for an R⁷-C group, wherein R⁷ is hydrogen or C₁-C₄-alkyl,
B stands for oxygen or, if A is nitrogen, also for sulphur,
n is a whole number from 2 to 4, and
Q^{a} stands for a (CH₂)ₘ^{a} group, wherein m^{a} is 3 or 4 and which may optionally be substituted by C₁-C₄ alkyl.

## Revendications

1. Composés de formule générale I 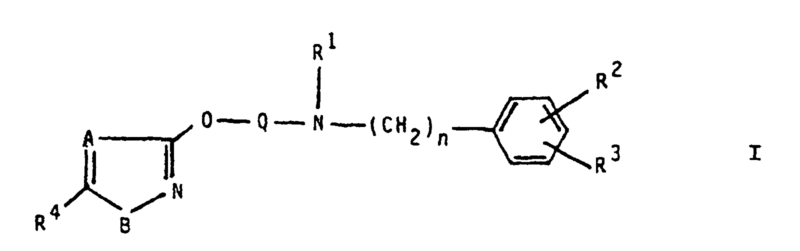 dans laquelle :
R¹ est l'hydrogène ou un alkyle en C₁-C₄,
R² est l'hydrogène, un halogène, un alkyle en C₁-C₄, un alcoxy en C₁-C₄, un hydroxy ou, dans le cas où Q et R⁴ ne contiennent pas de groupe OH et où R³ n'est pas un hydroxy, aussi un (alcanoyle en C₁-C₄)oxy, et
R³ est l'hydrogène, un halogène, un alkyle en C₁-C₄, un alcoxy en C₁-C₄, un hydroxy ou, dans le cas où Q et R⁴ ne contiennent pas de groupe OH et où R² n'est pas un hydroxy, aussi un (alcanoyle en C₁-C₄)oxy ou, dans le cas où R² est l'hydrogène, aussi un trifluorométhyle, un nitro, un amino, un (alkyle en C₁-C₄)amino, un (alkyle en C₁-C₄)sulfonylamino ou un (alcanoyle en C₁-C₄)amino, où cependant R³ ne représente pas un nitro dans le cas où R⁶ est un amino, un (alkyle en C₁-C₄)amino ou un (alcanoyle en C₁-C₄)amino, et ne représente pas un (alcanoyle en C₁-C₄)amino dans le cas où R⁶ est un amino ou un (alkyle en C₁-C₄)amino, ou
R² et R³ sont liés sur deux atomes de carbone adjacents et représentent conjointement un groupe alkylènedioxy comportant 1 à 2 atomes de carbone,
R⁴ est un thiényle ou un groupe phényle éventuellement substitué de formule générale a 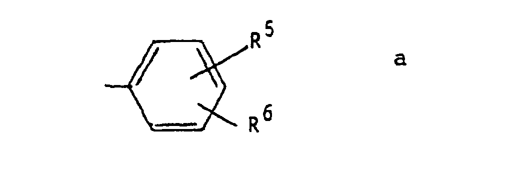
dans laquelle
R⁵ est l'hydrogène, un halogène, un alkyle en C₁-C₄ ou un alcoxy en C₁-C₄, un hydroxy ou, dans le cas où Q ne contient pas de groupe OH et où R², R³ et R⁶ ne sont pas un hydroxy, aussi un (alcanoyle en C₁-C₄)oxy, et
R⁶ est l'hydrogène, un halogène, un alkyle en C₁-C₄, un alcoxy en C₁-C₄, un hydroxy ou, dans le cas où Q ne contient pas de groupe OH et où R², R³ et R⁵ ne sont pas un hydroxy, aussi un (alcanoyle en C₁-C₄)oxy ou, dans le cas où R⁵ est l'hydrogène, aussi un trifluorométhyle, un nitro, un amino, un (alkyle en C₁-C₄)amino ou un (alcanoyle en C₁-C₄)amino,
A représente l'azote ou un groupe R⁷-C, où R⁷ est l'hydrogène ou un alkyle en C₁-C₄,
B est l'oxygène ou, si A est l'azote, aussi le soufre,
n est un nombre entier compris de 2 à 4, et
Q est un groupe (CH₂)ₘ, où m est un nombre entier compris de 2 à 8, et qui peut éventuellement être substitué par alkyle en C₁-C₄, ou est la chaîne 2-hydroxypropylène,
ainsi que leurs sels d'addition d'acide physiologiquement compatibles.

2. Composés selon la revendication 1, dans lesquels R⁴ est un groupe phényle éventuellement substitué de formule générale a, les substituants étant définis selon la revendication 1.

3. Composés selon la revendication 2, dans lesquels, dans le groupe phényle éventuellement substitué a, R⁵ possède la signification précédente et est disposé en position 2- ou 3-, et R⁶ est l'hydrogène.

4. Composés selon l'une des revendications précédentes, dans lesquels R¹ est un alkyle en C₁-C₄.

5. Composés selon l'une des revendications précédentes, dans lesquels R² et R³ sont en position 3- et 4- et représentent un alcoxy en C₁-C₄ ou un hydrogène.

6. Composés selon l'une des revendications précédentes, dans lesquels R¹ est un alkyle en C₁-C₄, R² est l'hydrogène ou un alcoxy en C₁-C₄, R³ est un alcoxy en C₁-C₄, R⁴ est un groupe phényle de formule générale a, dans laquelle R⁵ a la signification précédente et R⁶ est l'hydrogène, Q est la chaîne propylène et n vaut 2.

7. 3-{3-[N-(2-(3,4-diméthoxyphényl)-éthyl)-N-méthylamino]-propyloxy}-5-phényl-1,2,4-thiadiazole et ses sels d'addition d'acide physiologiquement compatibles.

8. 3-{3-[N-(2-(3,4-diméthoxyphényl)-éthyl)-N-méthylamino]-propyloxy}-5-phényl-1,2,4-oxadiazole et ses sels d'addition d'acide physiologiquement compatibles.

9. 3-{3-[N-(2-(3,4-diméthoxyphényl)-éthyl)-N-méthylamino]-propyloxy}-5-phényl-1,2,4-isoxazole et ses sels d'addition d'acide physiologiquement compatibles.

10. Médicament contenant une quantité pharmacologiquement efficace d'un composé selon la revendication 1, et des auxiliaires et supports pharmaceutiques usuels.

11. Procédé de fabrication de composés de formule générale I 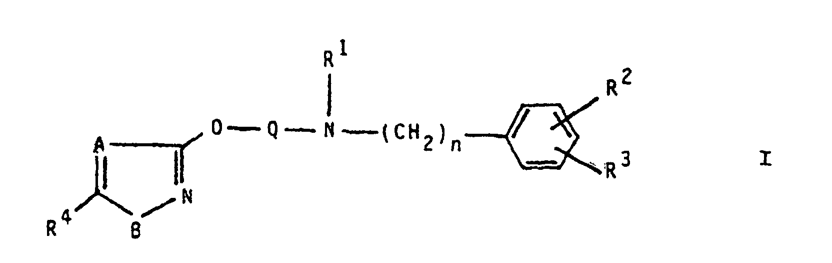 dans laquelle :
R¹ est l'hydrogène ou un alkyle en C₁-C₄,
R² est l'hydrogène, un halogène, un alkyle en C₁-C₄, un alcoxy en C₁-C₄, un hydroxy ou, dans le cas où Q et R⁴ ne contiennent pas de groupe OH et où R³ n'est pas un hydroxy, aussi un (alcanoyle en C₁-C₄)oxy, et
R³ est l'hydrogène, un halogène, un alkyle en C₁-C₄, un alcoxy en C₁-C₄, un hydroxy ou, dans le cas où Q et R⁴ ne contiennent pas de groupe OH et où R² n'est pas un hydroxy, aussi un (alcanoyle en C₁-C₄)oxy ou, dans le cas où R² est l'hydrogène, aussi un trifluorométhyle, un nitro, un amino, un (alkyle en C₁-C₄)amino, un (alkyle en C₁-C₄)sulfonylamino ou un (alcanoyle en C₁-C₄)amino, où cependant R³ ne représente pas un nitro dans le cas où R⁶ est un amino, un (alkyle en C₁-C₄)amino ou un (alcanoyle en C₁-C₄)amino, et ne représente pas un (alcanoyle en C₁-C₄)amino dans le cas où R⁶ est un amino ou un alkylamino, ou
R² et R³ sont liés sur deux atomes de carbone adjacents et représentent conjointement un groupe alkylènedioxy comportant 1 à 2 atomes de carbone,
R⁴ est un thiényle ou un groupe phényle éventuellement substitué de formule générale a 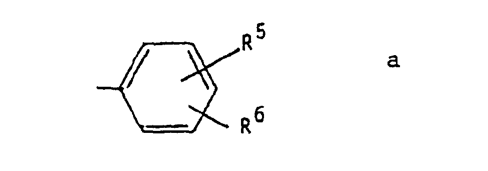
dans laquelle
R⁵ est l'hydrogène, un halogène, un alkyle en C₁-C₄, un alcoxy en C₁-C₄, un hydroxy ou, dans le cas où Q ne contient pas de groupe OH, et R², R³ et R⁶ ne sont pas un hydroxy, aussi un (alcanoyle en C₁-C₄)oxy,
R⁶ est l'hydrogène, un halogène, un alkyle en C₁-C₄, un alcoxy en C₁-C₄ ou, dans le cas où Q ne contient pas de groupe OH et R², R³ et R⁵ ne sont pas un hydroxy, aussi un (alcanoyle en C₁-C₄)oxy ou, dans le cas où R⁵ est l'hydrogène, aussi un trifluorométhyle, un nitro, un amino, un (alkyle en C₁-C₄)amino ou un (alcanoyle en C₁-C₄)amino,
A représente l'azote ou un groupe R⁷-C, où R⁷ est l'hydrogène ou un alkyle en C₁-C₄,
B est l'oxygène ou, si A est l'azote, aussi le soufre,
n est un nombre entier compris de 2 à 4, et
Q est un groupe (CH₂)ₘ, où m est un nombre entier compris de 2 à 8 et qui peut éventuellement être substitué par alkyle en C₁-C₄, ou est la chaîne 2-hydroxypropylène,
ainsi que leurs sels d'addition d'acide physiologiquement compatibles, **caractérisé en ce que**
a) pour préparer des composés de formule générale Ia 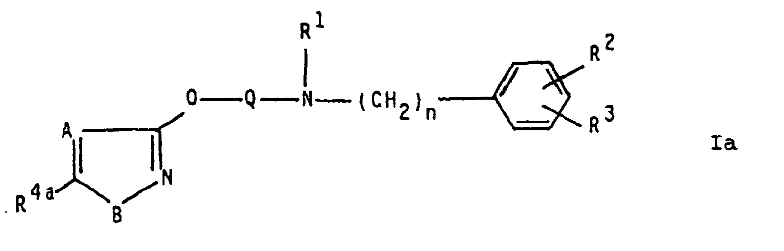 dans laquelle R¹, R², R³, A, B, Q et n ont les significations précédentes, et R^{4a} a la signification indiquée pour R⁴ à l'exception des radicaux contenant NH,
on convertit des composés de formule générale II 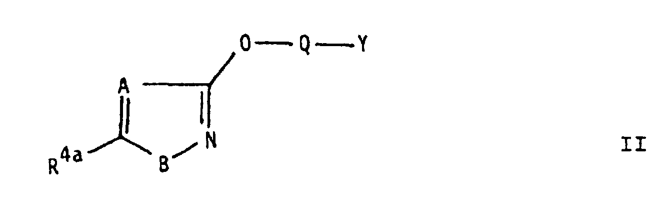 dans laquelle R^{4a}, A, B et Q ont les significations précédentes et Y est un groupe partant séparable par aminolyse, avec des composés de formule générale IIIa 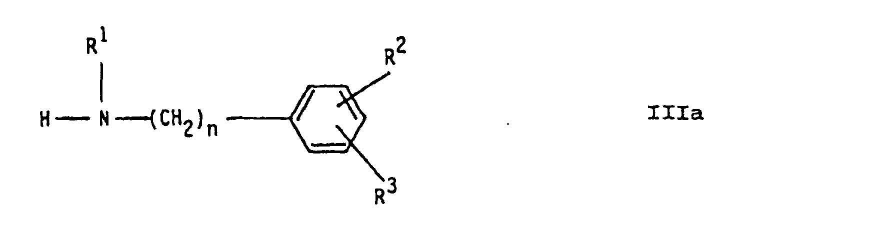 dans laquelle R¹, R², R³ et n ont les significations précédentes, où cependant les éventuels groupes hydroxy et/ou amino libres R² et/ou R³ sont pourvus d'un groupe protecteur, puis on sépare à nouveau les éventuels groupes protecteurs d'hydroxy et/ou d'amino, ou
b) pour préparer des composés de formule générale Ib 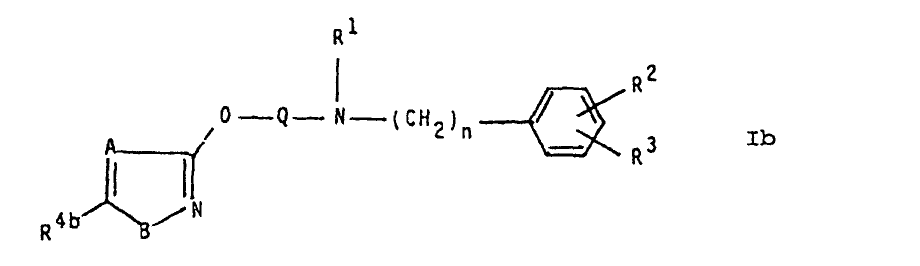 dans laquelle R¹, R ², R³, A, B, Q et n ont les significations précédentes, et R^{4b} a la signification indiquée pour R⁴ à l'exception des radicaux contenant un hydroxy et/ou NH,
on convertit des composés de formule générale IVa 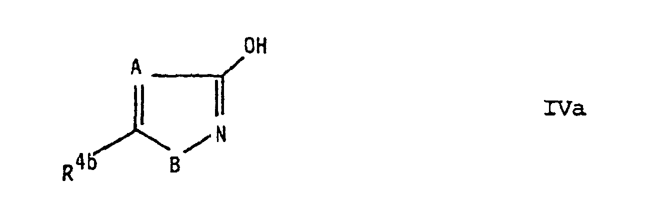 dans laquelle R^{4b}, A et B ont la signification précédente, avec des composés de formule générale V 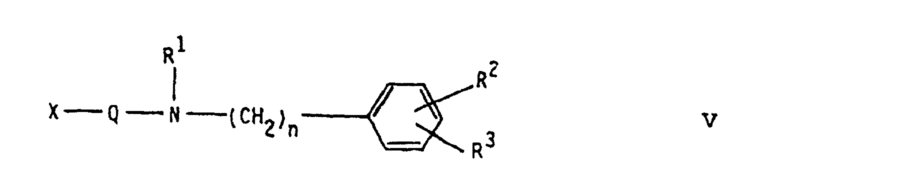 dans laquelle R¹, R², R³, n et Q ont les significations précédentes, où cependant les éventuels groupes hydroxy et/ou amino libres sont pourvus d'un groupe protecteur, et dans laquelle X est un groupe partant séparable, puis on sépare à nouveau les éventuels groupes protecteurs d'hydroxy et/ou d'amino, ou
c) pour préparer des composés de formule générale Ic 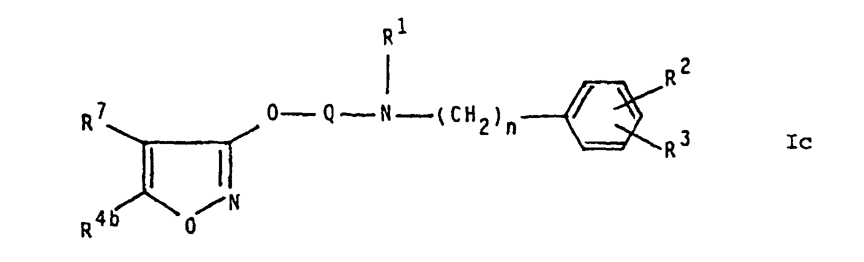 dans laquelle R', R², R³, R^{4b}, R⁷, Q et n ont les significations précédentes, on convertit des composés de formule générale VI 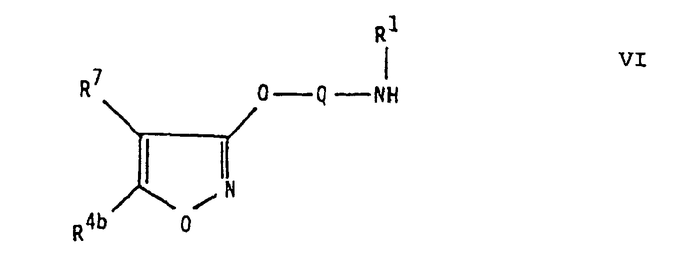 dans laquelle R¹, R⁷ et R^{4b} ont les significations précédentes, avec des composés de formule générale VII 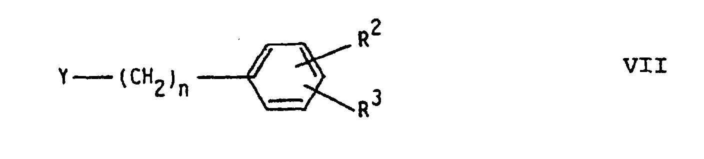 dans laquelle Y, n, R² et R³ ont les significations précédentes, où cependant les groupes hydroxy et/ou amino libres R² et/ou R³ sont pourvus d'un groupe protecteur, puis on sépare à nouveau les éventuels groupes protecteurs d'hydroxy et/ou d'amino, ou
d) pour préparer des composés de formule générale Id 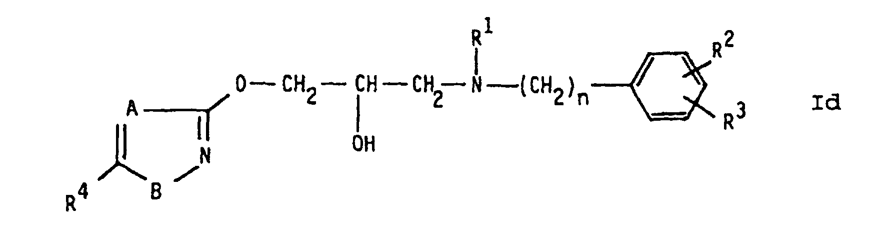 dans laquelle R¹, R², R³, R^{4b}, A, B et n ont les significations précédentes, on convertit des composés de formule générale IV 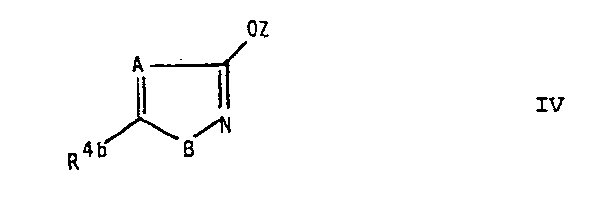 dans laquelle R^{4b}, A et B ont les significations précédentes et Z est un groupe 2,3-époxypropyle ou, dans le cas où A est un groupe R⁷-C-, aussi l'hydrogène, avec des composés de formule générale III 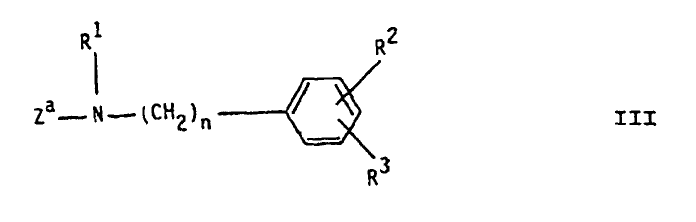 dans laquelle R¹, R², R³ et n ont les significations précédentes, où cependant les éventuels groupes hydroxy et/ou amino libres sont pourvus d'un groupe protecteur, et Z^{a} est l'hydrogène ou, dans le cas où Z dans les composés de formule IV est l'hydrogène, aussi le groupe 2,3-époxypropyle, puis on sépare à nouveau les éventuels groupes protecteurs d'hydroxy et/ou d'amino, ou
e) pour préparer des composés de formule générale Ie 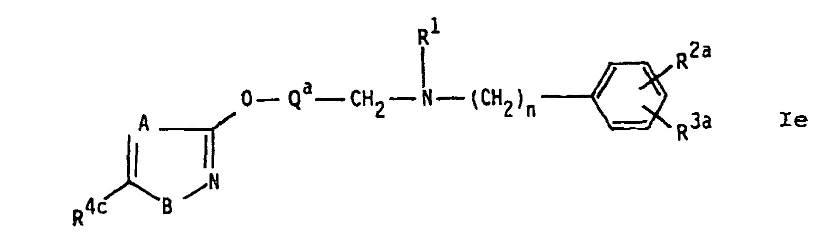 dans laquelle R¹, n, A et B ont les significations précédentes, et R^{4c} a la signification indiquée pour R^{4b} à l'exception des radicaux contenant un nitro et/ou un (alcanoyle en C₁-C₄)oxy, R^{2a} et R^{3a} ont les significations indiquées pour R² et R³ à l'exception d'un nitro, d'un (alcanoyle en C₁-C₄)oxy et d'un (alcanoyle en C₁-C₄)amino, et Q^{a} est un groupe (CH₂)ₘ^{a}, où m^{a} vaut 3 ou 4 et qui peut éventuellement être substitué par un alkyle en C₁-C₄,
on réduit des composés de formule générale VIII 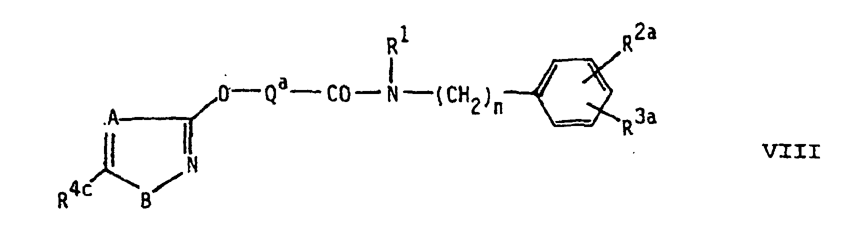 dans laquelle R¹, R^{2a}, R^{3a}, R^{4c}, A, B, Q^{a} et n ont les significations précédentes, où cependant les éventuels groupes hydroxy et/ou amino libres sont pourvus d'un groupe protecteur, puis on sépare à nouveau les éventuels groupes protecteurs d'hydroxy et/ou d'amino, ou
f) pour préparer des composés de formule générale If 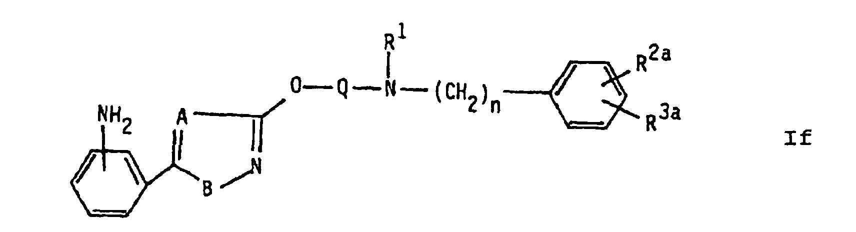 dans laquelle R¹, R^{2a}, R^{3a}, A, B, Q et n ont les significations précédentes, on réduit des composés de formule générale Ig 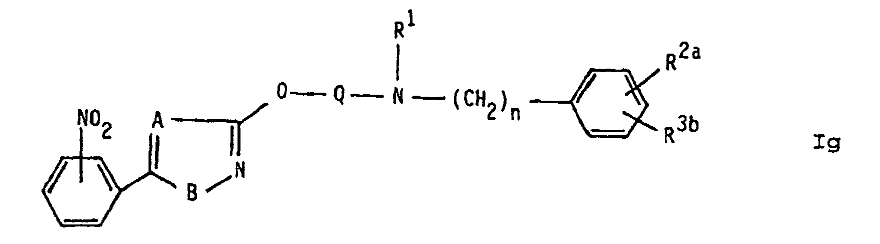 dans laquelle R¹, R^{2a}, A, B, Q et n ont les significations précédentes, et R^{3b} a la signification indiquée pour R³ à l'exception des radicaux contenant un alcanoyle, ou
g) pour préparer des composés de formule générale Ih 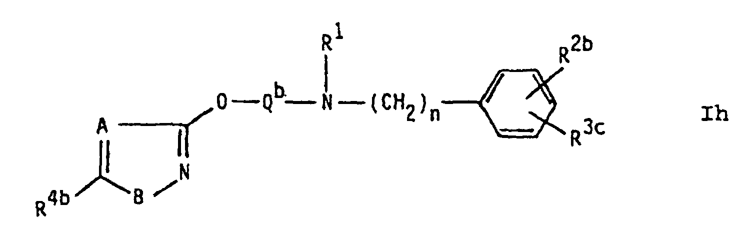 dans laquelle R¹, R^{4b}, A, B et n ont les significations précédentes, Q^{b} a la signification indiquée pour Q à l'exception de la chaîne 2-hydroxypropylène, et R^{2b} et R^{3c} ont les significations indiquées pour R² et R³ à l'exception des groupes OH, où cependant, parmi les substituants R^{2b} et R^{3c} et/ou parmi les substituants R⁵ et R⁶ contenus dans R^{4b}, au moins un représente un (alcanoyle en C₁-C₄)oxy ou un (alcanoyle en C₁-C₄)amino,
on acyle des composés de formule générale IX 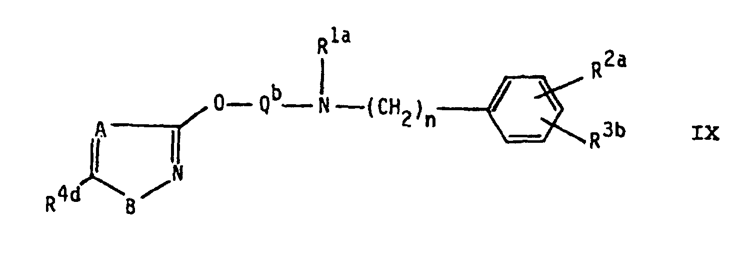 dans laquelle R^{2a}, R^{3b}, A, B, Q^{b} et n ont les significations précédentes, R^{1a} est un alkyle en C₁-C₄ ou un groupe protecteur d'amino, et R^{4d} a la signification indiquée pour R⁴ à l'exception des radicaux contenant un groupe alcanoyle en C₁-C₄ et dans laquelle peuvent éventuellement être contenus des groupes protecteurs d'hydroxy et/ou d'amino, où cependant, parmi les substituants R^{2a} et R^{3b} et/ou parmi les substituants R⁵ et R⁶ contenus dans R^{4d}, au moins un représente un groupe OH- ou NH₂- libre, puis on sépare à nouveau les éventuels groupes protecteurs, ou
h) pour préparer des composés de formule générale Ii 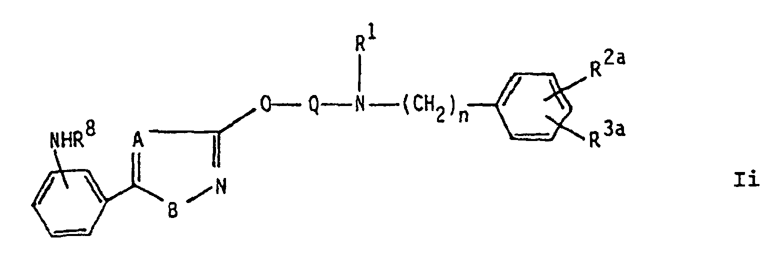
dans laquelle A, B, Q, R¹, R^{2a}, R^{3a} et n ont les significations précédentes, et R⁸ est un alkyle en C₁-C₄,
on réduit des composés de formule générale Ij 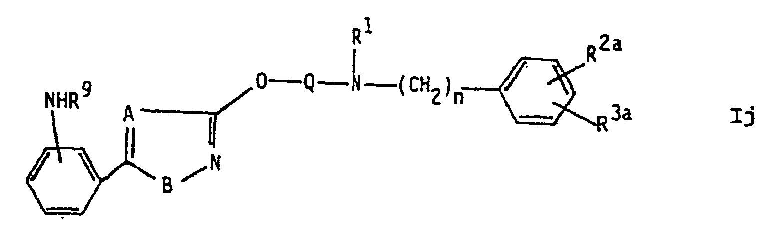 dans laquelle R¹, R^{2a}, R^{3a}, A, B et n ont les significations précédentes et R⁹ représente un alcanoyle en C₁-C₄, et
si cela est souhaité, dans les composés obtenus de formule I dans laquelle R², R³, R⁵ et/ou R⁶ représentent un méthoxy, on libère de ces groupes un groupe hydroxy et/ou, si cela est souhaité, on alkyle les composés obtenus de formule I dans laquelle R¹ représente l'hydrogène, en composés de formule I, dans laquelle R¹ représente un alkyle en C₁-C₄, et si cela est souhaité, on transforme les composés libres de formule I en leurs sels d'addition d'acide ou les sels d'addition d'acide en composés libres de formule I.

12. Composés de formule générale VIII 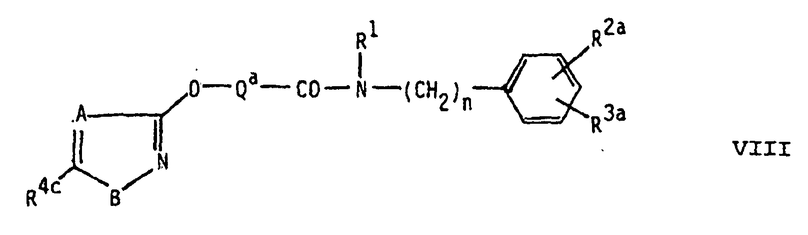 dans laquelle :
R¹ est l'hydrogène ou un alkyle en C₁-C₄,
R^{2a} est l'hydrogène, un halogène, un alkyle en C₁-C₄, un alcoxy en C₁-C₄, ou un hydroxy, et
R^{3a} est l'hydrogène, un halogène, un alkyle en C₁-C₄, un alcoxy en C₁-C₄, un hydroxy ou, dans le cas où R² est l'hydrogène, aussi un trifluorométhyle, un amino, un (alkyle en C₁-C₄)amino ou un (alkyle en C₁-C₄)sulfonylamino, ou
R² et R³ sont liés sur deux atomes de carbone adjacents et représentent conjointement un groupe alkylènedioxy comportant 1 à 2 atomes de carbone,
R^{4c} est un thiényle ou un groupe phényle éventuellement substitué de formule générale a' 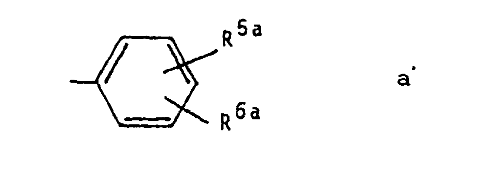
dans laquelle
R^{5a} est l'hydrogène, un halogène, un alkyle en C₁-C₄, un alcoxy en C₁-C₄, ou, dans le cas où R^{2a} et R^{3a} ne sont pas un hydroxy, aussi un (alcanoyle en C₁-C₄)oxy, et
R^{6a} est l'hydrogène, un halogène, un alkyle en C₁-C₄, un alcoxy en C₁-C₄ ou, dans le cas où R^{2a} et R^{3a} ne sont pas un hydroxy, aussi un (alcanoyle en C₁-C₄)oxy ou, dans le cas où R⁵ est l'hydrogène, aussi un trifluorométhyle, ou un (alcanoyle en C₁-C₄)amino,
A représente l'azote ou un groupe R⁷-C, où R⁷ est l'hydrogène ou un alkyle en C₁-C₄,
B est l'oxygène ou, si A est l'azote, aussi le soufre,
n est un nombre entier compris de 2 à 4, et
Q^{a} est un groupe (CH₂)ₘ^{a}, où m^{a} vaut 3 ou 4 et qui peut éventuellement être substitué par alkyle en C₁-C₄.
